(19) Europäisches Patentamt / European Patent Office / Office européen des brevets

(11) EP 1 428 886 B1

(12) **EUROPÄISCHE PATENTSCHRIFT**

(45) Veröffentlichungstag und Bekanntmachung des Hinweises auf die Patenterteilung:
**05.08.2009 Patentblatt 2009/32**

(51) Int Cl.:
*C12N 15/86* (2006.01) *C12N 7/00* (2006.01)
*C12N 5/10* (2006.01) *A61K 48/00* (2006.01)

(21) Anmeldenummer: **02027555.8**

(22) Anmeldetag: **09.12.2002**

(54) **Verbesserte retrovirale Vektoren für die Gentherapie**

Improved retroviral vectors for gene therapy

Vecteurs rétroviraux améliorés pour la thérapie génique

(84) Benannte Vertragsstaaten:
**DE FR GB IT SE**

(43) Veröffentlichungstag der Anmeldung:
**16.06.2004 Patentblatt 2004/25**

(73) Patentinhaber: **Celltech GmbH Biotechnologie**
**22459 Hamburg (DE)**

(72) Erfinder:
- **Heberlein, Christoph**
  **22869 Schenefeld (DE)**
- **Gindullis, Frank**
  **25337 Elmshorn (DE)**
- **Hannemann, Jürgen**
  **25469 Halstenbek (DE)**
- **Strathmann, Götz**
  **20144 Hamburg (DE)**

(74) Vertreter: **Maiwald Patentanwalts GmbH**
**Elisenhof**
**Elisenstrasse 3**
**80335 München (DE)**

(56) Entgegenhaltungen:
- **FEHSE B ET AL: "A novel 'sort-suicide' fusion gene vector for T cell manipulation." GENE THERAPY, [Online] Bd. 9, Nr. 23, 8. November 2002 (2002-11-08), Seiten 1633-1638, XP009010172 December, 2002 ISSN: 0969-7128**
- **OHNISHI N ET AL: "High expression of transgenes mediated by hybrid retroviral vectors in hepatocytes: Comparison of promoters from murine retroviruses in vitro and in vivo." GENE THERAPY, Bd. 9, Nr. 4, Februar 2002 (2002-02), Seiten 303-306, XP002240459 February, 2002 ISSN: 0969-7128**
- **RAPPA GERMANA ET AL: "Novel bicistronic retroviral vector expressing gamma-glutamylcysteine synthetase and the multidrug resistance protein 1 (MRP1) protects cells from MRP1-effluxed drugs and alkylating agents." HUMAN GENE THERAPY, Bd. 12, Nr. 14, 20. September 2001 (2001-09-20), Seiten 1785-1796, XP002240460 ISSN: 1043-0342**
- **HILDINGER MARKUS ET AL: "Design of 5' untranslated sequences in retroviral vectors developed for medical use." JOURNAL OF VIROLOGY, Bd. 73, Nr. 5, Mai 1999 (1999-05), Seiten 4083-4089, XP002240461 ISSN: 0022-538X**
- **HILDINGER M ET AL: "BICISTRONIC RETROVIRAL VECTORS FOR COMBINING MYELOPROTECTION WITH CELL-SURFACE MARKING" GENE THERAPY, MACMILLAN PRESS LTD., BASINGSTOKE, GB, Bd. 6, Nr. 7, 1999, Seiten 1222-1230, XP000990645 ISSN: 0969-7128**
- **CHALMERS D ET AL: "Elimination of the truncated message from the herpes simplex virus thymidine kinase suicide gene." MOLECULAR THERAPY: THE JOURNAL OF THE AMERICAN SOCIETY OF GENE THERAPY. UNITED STATES AUG 2001, Bd. 4, Nr. 2, August 2001 (2001-08), Seiten 146-148, XP002240462 ISSN: 1525-0016**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 428 886 B1

## Beschreibung

Die vorliegende Erfindung betrifft neue verbesserte retrovirale Vektoren für die Gentherapie, insbesondere für die Behandlung der Graft versus Host Disease (GvHD). Die erfindungsgemäßen neuen retroviralen Vektoren zeichnen sich dadurch aus, dass sie die Leadersequenz des Murine Embryonic Stem cell Virus (MESV) oder modifizierte Formen dieser Leadersequenz, die 3'-LTR des Myeloproliferative Sarcoma Virus (MPSV) sowie zwei transgene Sequenzen enthalten, wobei die Expression des zweiten Transgens durch eine interne ribosomale Eintrittsstelle (Internal Ribosomal Entry Site, IRES) gesteuert wird. Ferner betrifft die Erfindung Verfahren zur Herstellung von infektiösen Viruspartikeln, die die erfindungsgemäßen retroviralen Vektoren enthalten. Die Erfindung betrifft ebenfalls die Verwendung von erfindungsgemäßen retroviralen Vektoren für die Anwendung in der Gentherapie, insbesondere für die Kontrolle der GvHD.

Retrovirale Vektoren werden üblicherweise für den stabilen Gentransfer in Säugetierzellen verwendet und ermöglichen die Expression sowohl von endogenen als auch von heterologen Proteinen in den jeweiligen Wirtszellen. Sie finden insbesondere Anwendung, um zum Beispiel Wirts-Proteine, die aufgrund von Mutationen nur in einer aberranten Form exprimiert werden, zu ersetzen. Daneben können retrovirale Vektoren auch ganz allgemein verwendet werden, um die Expression von heterologen Proteinen in Säugetierzellen für Produktionszwecke zu ermöglichen.

Wenn die retroviralen Vektoren Sequenzen, die für Wirts-Faktoren kodieren, in antisense-Orientierung enthalten, können sie dagegen verwendet werden, um die Expression von spezifischen Proteinen oder RNAs zu unterdrücken. Unter Expression von RNAs wird in diesem Sinne die Transkription von RNAs verstanden, die nicht weiter translatiert werden. Dies gilt auch dann, wenn die für die RNA kodierenden DNA-Sequenzen sense-Orientierung aufweisen.

Aufgrund der genannten Eigenschaften haben sich retrovirale Vektoren insbesondere für die Anwendung in der Gentherapie bewährt, bei der es häufig darauf ankommt, ein defektes Genprodukt durch ein funktionelles Genprodukt zu ersetzen. Aber auch die Expression eines heterologen Proteins, d.h. eines natürlicherweise nicht im Wirt vorkommenden Proteins, kann für die Gentherapie wünschenswert sein (siehe unten).

Für den stabilen Gentransfer in Säugetierzellen sind retrovirale Vektoren auf der Grundlage von Maus-Leukämieviren (MLV) das gegenwärtig am häufigsten verwendete und am besten charakterisierte System (Miller, A.D., 1993, Methods Enzymol., 217, 581-599).

Anwendungsgebiete dieser retroviralen Vektoren sind beispielsweise die bereits erwähnte Überexpression von Proteinen mit dem Ziel der Gewinnung reiner Proteine, die Expressionsklonierung mit dem Ziel neue Proteine zu identifizieren, sowie die stabile Expression eines Proteins in einer Körperzelle mit dem Ziel einer therapeutischen Wirkung des exprimierten Proteins.

Retrovirale Vektoren können wie Retroviren in zwei Formen vorliegen, als provirale DNA oder als Vektor-RNA. Stabil im Genom der Zielzelle integriert ist die provirale doppelsträngige DNA. Von dieser wird das so genannte genomische Vektortranskript abgelesen, das wie eine zelluläre mRNA aufgebaut ist und in virale Partikel verpackt wird, um die Erbinformation des Retrovirus zu übertragen. Das genomische Vektortranskript wird nach retroviraler Infektion einer Zielzelle durch reverse Transkription in ein neues Provirus umgeschrieben und in das Genom der Zelle stabil integriert (Miller, A.D. vide supra).

Charakteristischerweise wird das Provirus am 5'- und 3'-Ende von "Long Terminal Repeats" (LTR) flankiert, welche die Regionen U3, R und U5 umfassen (Fig. 1). Die U3-Region enthält Enhancer- und Promotorsequenzen, die die Transkription des Vektorgenoms steuern. Die R-Region trägt das Polyadenylierungssignal. Die U5-Region enthält Sequenzen, die für die Integration des Retrovirus notwendig sind. Die kodierenden Sequenzen der transgenen Sequenz eines retroviralen Vektors liegen gewöhnlich zwischen der 5'- und 3'-LTR im Vektor und werden von regulatorisch wichtigen Kontrollsequenzen flankiert, die für den Ablauf des retroviralen Lebenszyklus essentiell sind und zugleich die Halbwertszeit der RNA sowie die Effizienz der Translation des transgenen Proteins beeinflussen. Der Transkriptionsstart der RNA liegt an der Grenze zur R-Region des 5'-LTR. Das Transkriptionsende wird durch das Polyadenylierungs- und Terminationssignal in der R-Region des 3'-LTR bestimmt. Am Ende der R-Region des 3'-LTR wird ein Polyadenosin-Schwanz angeheftet. Spleißsignale des retroviralen Vektors bzw. des Retrovirus können zur Transkription mit internen Deletionen führen.

Der 5'-untranslatierte Bereich eines retroviralen Vektors setzt sich typischerweise zusammen aus (Miller, A.D. vide supra):

1. Der R-Region und US-Region des 5'-LTR, die notwendig für die reverse Transkription und die Integration des viralen Genoms in das Genom des Wirts sind.
2. Primerbindungsstelle, die notwendig für die reverse Transkription des viralen Genoms ist.
3. Sowie die Leaderregion, die bei gängigen retroviralen Vektoren mindestens 800 Nukleotide lang ist. Sie schließt sich an die Primerbindungsstelle an und reicht bis zum Start der kodierenden Sequenz.

Die Leaderregion enthält das Verpackungs- und Dimerisierungssignal, welches für die Inkorporation der retroviralen RNA in retrovirale Partikel notwendig ist. Am Beginn des Leaders liegt ein retroviraler Spleißdonor, am Ende des Leaders kann ein kryptischer (d.h. nur teilweise erkennbarer) Spleißakzeptor liegen. Spleißdonor und Spleißakzeptor bestimmen damit die Größe des RNA-Transkripts, sowie die Effizienz der Spleißreaktion (Zhuang, Y.A. et al., 1989, Proc. Natl. Acad. Sci. USA, 86, 2752-2756). Die Effizienz der Translation wird durch die Größe des 5'-untranslatierten Bereichs (5'-UTR) beeinflusst. Zum Beispiel können die Spleissignale des Leaders Anlass zu verkürzten Transkripten mit deutlich kleineren 5'-UTRs geben, die eine erhöhte Translationseffizienz aufweisen (Krall, W.J. et al., 1996, Gene Ther., 3, 37-48).

Der 3'-untranslatierte Bereich der RNA enthält den Polypurintrakt, welcher für die reverse Transkription notwendig ist, sowie die U3- und R-Region des 3'-LTR. Das Ende der RNA umfasst den Polyadenosinschwanz von ca. 200 Nukleotiden, der für die Stabilität im Zytoplasma mitbestimmend ist.

Im Laufe des Lebenszyklus eines retroviralen Vektors wird nach Tranduktion einer Zielzelle mit einem infektiösen Viruspartikel die tranduzierte virale RNA zunächst revers transkribiert und in Form des Provirus in das Genom der Wirtszelle integriert. Im Laufe dieses Vorgangs wird die U3-Region des 3'-LTR in die entsprechende U3-Region des 5'-LTR kopiert. Die Transkription der kodierenden Sequenzen der proviralen, genomisch integrierten DNA wird durch diese in die 5'-LTR kopierte U3-Region bestimmt. Insofern kann bei der Konstruktion von retroviralen Vektoren zunächst immer eine beliebige 5'-LTR verwendet werden, da nach der Integration des Virus in das Genom der Zielzelle, die Transkription und damit die Expression der durch den retroviralen Vektor übertragenen Gene durch die 3'-LTR, bzw. die U3 Region der 3'-LTR gesteuert wird (Baum, C. et al., 1995, J. Virol., 69, 7541-7547). Man kann daher auch an Stelle der Bezeichnung "U3 Region der 3'-LTR des MPSV" von der "U3 Region der LTR des MPSV" sprechen.

Retroviren enthalten neben den genannten Kontrollelementen (5'-LTR, Leaderregion, 3'-LTR, etc.) häufig Nukleotidsequenzen, die für virale Proteine oder zumindest Teile davon kodieren. Zu diesen zählen das gag-Gen, das für Strukturproteine des Virus kodiert, das pro-Gen, das für die virale Protease kodiert, das pol-Gen, das für die reverse Transkriptase kodiert, sowie das env-Gen, das für Virushüllglykoproteine kodiert. In retroviralen Vektoren ist typischerweise mindestens eines dieser Gene deletiert bzw. mutiert, so dass retrovirale Vektoren per se replikationsdefizient sind. Entsprechend braucht man zur Herstellung von infektiösen Viruspartikeln, die retrovirale Vektoren enthalten, so genannte Helferviren bzw. verpackungskompetente Helferzelllinien.

Solche verpackungskompetente Zelllinien tragen typischerweise deletierte retrovirale Genome, die noch in der Lage sind für die Strukturproteine, die Hüllproteine, die reverse Transkriptase und die virale Protease zu kodieren, die aber keine viralen RNAs herstellen, die zusammen mit diesen Strukturproteinen und Hüllproteinen in infektiöse Partikel verpackt werden können. Werden solche verpackungskompetenten Helferzelllinien mit retroviralen Vektoren transfiziert, die zwar weder für die Hüllproteine, die Strukturproteine, die Protease oder die reverse Transkriptase kodierende Sequenzen tragen, sehr wohl aber für andere Proteine kodierende Sequenzen und die entsprechenden Primerbindungsstellen, die Verpackungssequenzen, etc. aufweisen, so können die durch die Helferzelllinien zur Verfügung gestellten Hüll-, Struktur- und Funktionsproteine mit der vom als Provirus vorliegenden Vektor produzierten RNA assoziieren und infektiöse Viruspartikel bilden.

Durch Integration der proviralen DNA von retroviralen Vektoren in verpackungskompetente Helferzelllinien können auf diese Weise so genannte stabile Producer-Zelllinien hergestellt werden, die das provirale Vektorgenom in das Genom der Verpackungszelllinie integriert haben und in der Lage sind, infektiöse Viruspartikel zu produzieren, die replikationsdefekt sind.

Retrovirale Sequenzen sind evolutiv darauf hin optimiert worden, effizient Zielzellen zu infizieren, in das Zielgenom zu integrieren und eine gute Expression der integrierten DNA-Sequenzen zu gewährleisten. Diese Eigenschaften von retroviralen Sequenzen haben hinsichtlich der Expression von DNA-Sequenzen, die über retrovirale Vektoren in Zielzellen eingeführt werden, Vorteile gegenüber anderen Methoden des Gentransfers, bringen aber auch inhärente Gefahren mit sich, die insbesondere bei der Gentherapie zum Tragen kommen. Zum Beispiel können die in den retroviralen Vektoren enthaltenen viralen Sequenzen durch Rekombination mit komplementären retroviralen Sequenzen in der Verpackungszelllinie bewirken, dass replikationskompetente infektiöse Retroviren entstehen. Solche replikationskompetenten infektiösen Retroviren können natürlich für die Gentherapie nicht verwendet werden, da sie die Gefahr der Auslösung schwerer Krankheiten und Infektionen mit sich bringen. In Tierversuchen ist zum Beispiel gezeigt worden, dass auf diese Weise replikationskompetente Retroviren entstehen, die Leukämien und Enzephalopathien auslösen können (Anderson, W.F., 1993, Hum. Gene Ther., 4, 311-321, Münck, C., 1997, Proc. Natl.Acad. Sci., USA, 94, 5837-5842).

Die in retroviralen Vektoren enthaltenen retroviralen Sequenzen weisen darüber hinaus häufig kryptische Start-Kodons und/oder Spleiß-Sites auf. Bei der Transkription und/oder Translation von retroviralen Sequenzen können daher häufig verkürzte, fehlerhafte und toxische Produkte entstehen, was die Anwendung solcher retroviralen Vektoren für die Gentherapie in Frage stellt. Aufgrund der obigen Ausführungen müssen retrovirale Vektoren, die für die Gentherapie verwendet werden sollen, mehrere Eigenschaften aufweisen. Zum einen müssen sie hohen Sicherheitsmaßstäben genügen, d.h. es darf nicht die Gefahr bestehen, dass bei der Herstellung von infektiösen Virenpartikeln in Verpackungszelllinien durch Rekombination infektiöse replikationsfähige Retroviren entstehen, die sich in die Zielzellen der Patienten integrieren könnten. Weiterhin muss sicher gestellt werden, dass bei der Transkription und der Translation der retroviralen Sequenzen keine Nebenprodukte entstehen, die einen schädlichen Einfluss auf die zellulären Funktionen des Patienten haben. Typischerweise wird versucht, die Sicherheit von retroviralen Vektoren dadurch zu erhöhen, dass sämtliche retroviralen Sequenzen, die nicht unbedingt für die Funktion des retroviralen Vektors notwendig sind, aus diesem Vektor entfernt werden.

Gleichzeitig müssen retrovirale Vektoren in der Lage sein, eine hohe Expression, d.h. eine effiziente Transkription und/oder Translation der durch den retroviralen Vektor in die Zielzelle eingeführten DNA-Sequenzen in der Zielzelle zu gewährleisten.

Im Stand der Technik sind daher mehrfache Versuche unternommen worden, um retrovirale Vektoren dahin gehend zu optimieren, dass sie auf der einen Seite aufgrund des oben genannten Sicherheitsaspektes möglichst wenig retrovirale Sequenzen aufweisen, auf der anderen Seite hinsichtlich der Expression der zu übertragenden Sequenz sowie des Virustiters optimiert sind.

Ein zentrales Anwendungsgebiet von retroviralen Vektoren, die von Maus-Leukämieviren (MLV) abgeleitet wurden, betrifft den Gentransfer in hämatopoetische Stammzellen (Dunbar, C.G., 1996, Ann. Rev. Med., 47, 11-20). Dabei sollen durch Transfer und Expression des vom Vektor kodierten Proteins therapeutische Wirkungen erzielt werden. Das Protein wird in den Zellen permanent produziert, wodurch eine lang anhaltende Therapie gewährleistet wird, die direkt auf die der Krankheit zugrunde liegenden genetischen Defekte wirkt. Beispielsweise können retrovirale Vektoren Gene wie das menschliche MDR1 (Multi Drug Resistance 1)-Gen tragen. Derartige modifizierte Zellen sind gegen die toxischen Nebenwirkungen bei einer zytostatischen Chemotherapie geschützt (Baum, C. et al., 1996, Gene Ther., 3, 1-3, Baum, C. et al., 1995, vide supra).

Darüber hinaus können MLV-basierte retrovirale Vektoren für die adoptive Immuntherapie verwendet werden. Bei der Transplantation von Knochenmark zur Behandlung von Leukämie kommt es häufig zu einer Entzündungs- und Abstoßungsreaktion, die als Graft versus Host Disease (GvHD) bezeichnet wird und im schlimmsten Fall zum Tode des Patienten führen kann. Verantwortlich für die GvHD sind vor allem die T-Zellen. T-Zellen-depletiertes Knochenmark kann für entsprechende Transplantationen zwar verwendet werden, solches Knochenmark zeigt aber nicht den gewünschten Graft versus Leukemia (GvL)-Effekt (Bonini, C. et al., 1997, Science, 276, 1719-1724, Bordignon, C. et al., 1995, Science, 270, 470-475, Tiberghien, P. et al., 1997, Hum. Gene Ther., 8, 615-624). Eine Möglichkeit, die GvHD gezielt zu unterdrücken, besteht darin, durch retrovirale Vektoren ein so genanntes "Suicide"- bzw. Selbstmord-Gen in T-Zellen zu exprimieren. Bei einem solchen Selbstmord-Gen kann es sich zum Beispiel um die Thymidinkinase von Herpes simplex-Virus (HSV-tk) handeln. Sich replizierende Zellen, die die Thymidinkinase von Herpes simplex-Virus exprimieren und mit Ganciclovir behandelt werden, sterben.

Werden T-Zellen, die mit retroviralen Vektoren transduziert worden sind, die die kodierenden Sequenzen für HSV-tk enthalten, zusammen mit Knochenmark transplantiert, kann zunächst beobachtet werden, ob durch das Transplantat ein GvL-Effekt ausgelöst wird. Sollte es infolge der Transplantation zusätzlich zu einem GvHD-Effekt kommen, kann durch Verabreichung von Ganciclovir eine selektive Abtötung der für die GvHD verantwortlichen T-Zellen induziert werden. Auf diese Weise können die Vorteile eines Transplantats, das T-Zellen umfasst, genutzt werden, ohne dass das Auftreten einer GvHD billigend in Kauf genommen werden muss (Bonini et al., 1997, vide supra, Tiberghien et al., 1997, vide supra). Insbesondere für diese adoptive Immuntherapie ist eine hohe Expression des Transgens sowie ein hoher Virustiter der nicht replikationsfähigen, infektiösen Virus-Partikel, die den retroviralen Vektor enthalten, notwendig, da nur auf diese Weise gewährleistet ist, dass zum einen die T-Zellen in ausreichendem Umfang mit dem retroviralen Vektor transduziert werden und sie wegen der hohen Expression auch eine entsprechend hohe Sensitivität gegenüber Ganciclovir aufweisen. Gleichzeitig werden auch die Gefahren von infektiösen, replikationsfähigen Viruspartikeln, die durch Rekombination mit viralen Sequenzen in der Verpackungszelllinie entstanden sind, deutlich. Solche replikationsfähigen, infektiösen Viruspartikel breiten sich über alle Zelltypen des behandelten Patienten aus, so dass eine selektive Abtötung der T-Zellen und somit eine selektive Bekämpfung der GvHD nicht mehr möglich ist.

Im Stand der Technik sind verschiedene Versuche unternommen worden, retrovirale Vektoren herzustellen, die aus Sicherheitsaspekten auf der einen Seite möglichst wenig retrovirale Sequenzen aufweisen, auf der anderen Seite eine ausreichende Expression der durch den retroviralen Vektor zu übertragenen Transgene gewährleisten.

Für so genannte monocistronische retrovirale Vektoren, die nur ein Transgen exprimieren, haben Hildinger et al. (Gene Ther. (1998), 5, 1575-1579) gezeigt, dass ein hoher Virustiter und eine besonders effiziente Expression des Transgens, bei dem es sich in diesem Falle um das Gen für die Neomycinresistenz ($neo^R$), für EGFP oder für LNGFR handelte, immer dann erreicht werden kann, wenn als Leadersequenz MESV-Leadersequenzen und als 3'-LTR Sequenzen des Spleen Focus Forming Virus (SFFV) verwendet werden. Insbesondere zeigte diese Studie, dass die Leadersequenz-vermittelte Repression der retroviralen Aktivität, wie sie häufiger in humanen Zellen beobachtet wird, durch die Verwendung von MESV-Leadersequenzen verhindert werden kann.

Die Leadersequenzen dieses Standes der Technik wiesen ebenso wie die Leadersequenzen von anderen retroviralen Vektoren des Standes der Technik Teile der für das gag-Gen kodierenden Nukleinsäure auf. Auch wenn in der Publikation von Hildinger et al. (Gene Ther. (1998), 5, 1575-1579) das AUG-Startkodon des gag-Gens mutiert war, besteht aufgrund der vorhandenen retroviralen Sequenzen die Gefahr einer Rekombination in der Verpackungszelllinie.

Auf dieser Studie aufbauend zeigten Hildinger et al., 1999, (J. Virol., 73, 4083-4089) dann, dass retrovirale Vektoren, in denen diese viralen gag-Sequenzen deletiert sind und die als 3'-LTR die Sequenzen des SFFV aufweisen, sich ebenfalls durch eine gute Expression des Transgens in hämatopoetischen Stammzellen und einen hohen Virustiter auszeichnen. Insbesondere zeigte diese Studie, dass solche Vektoren, die in der MESV-Leaderregion eine Spleißdonorsite und eine Spleißakzeptorsite sowie ein mutiertes kryptisches AUG-Kodon aufwiesen, eine besonders hohe Expression des Transgens erlaubten. Als Transgene wurden das MDR1-Gen, das Gen für β-Galaktosidase sowie das Gen für EGFP in verschiedenen Zelltypen exprimiert. Dieser Stand der Technik beschäftigt sich nicht mit Vektoren, die die Expression von zwei Transgenen erlauben.

Solche bicistronischen (d.h. es werden zwei Transgene exprimiert) Vektoren sind für eine Reihe von Anwendungen und insbesondere bei retroviralen Vektoren, die für die adoptive Immuntherapie verwendet werden sollen, von großer Wichtigkeit. Das erste Transgen kodiert nämlich typischerweise für ein Protein, dessen Funktion durch die Anwendung bestimmt wird. Zum Beispiel kann es sich bei der Chemotherapie um das MDR1-Gen, bei der adoptiven Immuntherapie um HSV-tk handeln. Das zweite Transgen kodiert in der Regel für einen selektionierbaren Marker, wie zum Beispiel das Gen für $neo^R$ oder das ΔLNGFR-Gen (Mutante des Low affinitiy Nerve Growth Factor Receptor mit deletierter cytoplasmatischer Domäne). Die Verwendung des zweiten Transgens ist notwendig, um sicherzustellen, dass nur solche T-Zellen für die adoptive Immuntherapie verwendet werden, die tatsächlich auch mit dem retroviralen Vektor transduziert wurden. Im Falle von $neo^R$ können Zellen, die das Transgen integriert haben, durch Anzucht unter selektiven Bedingungen, d.h. unter Anwesenheit von Geneticin selektioniert werden. Im Falle des ΔLNGFR-Gens exprimieren transduzierte T-Zellen (oder entsprechend andere transduzierte Zellen) auf ihrer Oberfläche das durch das Transgen kodierte Epitop. Unter Verwendung von monoklonalen Antikörpern können dann im Rahmen einer FACS-Analyse die T-Zellen isoliert werden, die durch den retroviralen Vektor transduziert worden sind.

Die Verwendung von bicistronischen retroviralen Vektoren, die zwei Transgene exprimieren sollen, bringt zusätzliche Anforderungen mit sich, da die Expression des zweiten Transgens durch eine zusätzliche regulatorische Sequenz gesteuert bzw. mit der Expression des ersten Gens gekoppelt werden muss. Bei solchen regulatorischen Sequenzen kann es sich zum Beispiel um Spleißakzeptorsites (SA) handeln, die 5' vom zweiten Transgen liegen, um Internal Ribosomal Entry Sites (IRES) oder um interne Promotorsequenzen, die jeweils ebenfalls 5' des zweiten Transgens liegen.

Hildinger et al. (Gene Ther. (1999), 6, 1222-1230) haben untersucht, welche der genannten regulatorischen Sequenzen eine optimale Expression sowohl des ersten als auch des zweiten Transgens ermöglichen. Diese retroviralen Vektoren zeichneten sich ansonsten durch die 3'-LTR des SFFV sowie die Leaderregion des MESV aus, wobei die Leaderregion noch Teile der für gag kodierenden Nukleinsäuresequenz enthielt. Die genannte Publikation zeigte, dass für die Expression des zweiten Transgens eine Spleißakzeptorsite, die 5' Zum zweiten Transgen liegt, optimal ist. Die Verwendung einer IRES oder eines internen Promotors führte dagegen zu retroviralen Vektoren, deren Expression nicht optimal ist. Außerdem haben die genannten Vektoren den Nachteil, dass sie aufgrund des Vorhandenseins von retroviralen Sequenzen des gag-Gens den beschriebenen Sicherheitserfordernissen nicht genügen.

Fehse B. et al in Gene Therapy 9:1633-1638 (2002) offenbaren im Kontext der adoptiven Immunotherapie einen "R1" benannten retroviralen Hybridvektor, der auf MPSV und MESV beruht (3'-LTR des MPSV) und eine bicistronische Transkriptionseinheit trägt, die sowohl für CD34 wie für eine "Spleiß-korrigierte" Variante der Thymidinkinase des Herpes Simplex Virus (HSV-TK, scTK) kodiert. Die Leader-Sequenz ("Leader 71 ") dieses Vektors stammt aus MESV, ist frei von Überresten viraler Leseraster und enthält eine künstliche Intronsequenz. Die Autoren von D1 kommen jedoch zu dem Schluss, dass der Vektor R1 für die adoptive Gentherapie nicht geeignet ist.

Aus dem Stand der Technik sind damit keine retroviralen Vektoren bekannt, die zum einen nicht über wesentliche Anteile an retroviralen Sequenzen verfügen, und zum anderen die Expression von zwei Transgenen erlauben, wie sie für die adoptive Immuntherapie und andere gentherapeutische Anwendungen notwendig sind.

Aufgabe der vorliegenden Erfindung ist es, retrovirale Vektoren zur Verfügung zu stellen, die zum einen möglichst wenig retrovirale Sequenzen aufweisen und deswegen den gängigen Sicherheitsmaßstäben genügen, zum anderen aber gewährleisten, dass mindestens zwei Transgene effizient exprimiert werden können. Es ist eine weitere Aufgabe der vorliegenden Erfindung, retrovirale Vektoren zur Verfügung zu stellen, die möglichst wenig retrovirale Sequenzen aufweisen und die Expression von mindestens zwei Transgenen ermöglichen, so dass diese Vektoren für die adoptive Immuntherapie und andere gentherapeutische Anwendungen verwendet werden können.

Diese und weitere Aufgaben der vorliegenden Anmeldung, wie sie sich aus der Beschreibung ergeben, werden durch die kennzeichnenden Merkmale des unabhängigen Patentanspruchs gelöst. Bevorzugte Ausführungsformen werden durch die Unteransprüche definiert.

Gemäß der vorliegenden Anmeldung wird ein retroviraler Vektor der allgemeinen Formel:
5'- Ende - [5'- LTR] - [Leader] - [Ex1] - [IRES] - [Ex2] - [3'- LTR] - 3'- Ende zur Verfügung gestellt, der dadurch gekennzeichnet ist, dass er eine beliebige 5'-LTR, die Leaderregion des MESV ohne für gag oder andere virale Proteine kodierende Sequenzen, operativ daran gebunden eine Nukleinsäuresequenz [Ex1], die für ein erstes Transgen kodiert, eine IRES und operativ an die IRES gebunden eine Nukleinsäuresequenz [Ex2], die für ein zweites Transgen kodiert, sowie eine 3'-LTR mit der U3-Region der 3'-LTR des MPSV enthält.

Überraschenderweise ist im Rahmen der vorliegenden Erfindung erstmals gefunden worden, dass ein retroviraler Vektor, der eine MESV-Leaderregion aufweist, in der alle für Teile des gag-Gens kodierenden Sequenzen deletiert sind, der die 3'-LTR des MPSV enthält und in dem die Expression des zweiten Transgens durch eine IRES gesteuert wird, sich durch eine hohe Expression auszeichnet, die über dem Expressionslevel aller bisher bekannten retroviralen Vektoren, wie sie typischerweise für die Gentherapie eingesetzt werden, liegt. Angesichts des Standes der Technik konnte nicht erwartet werden, dass diese spezifische Kombination von regulatorischen retroviralen und nicht-retroviralen Sequenzen die Konstruktion eines retroviralen Vektors erlaubt, der die effiziente Expression von zwei Transgenen in Zielzellen ermöglicht. Solche erfindungsgemäßen retroviralen Vektoren sind damit idealerweise geeignet, um in verschiedenen gentherapeutischen Anwendungen wie zum Beispiel der adoptiven Immuntherapie eingesetzt zu werden.

Gegenstand der Erfindung sind ebenfalls infektiöse Viruspartikel, die einen erfindungsgemäßen retroviralen Vektor enthalten. Ein weiterer Gegenstand der vorliegenden Anmeldung sind so genannte stabile Producer-Zelllinien, die infektiöse, replikationsdefekte Viruspartikel herstellen können, die den erfindungsgemäßen retroviralen Vektor enthalten.

Ebenfalls Gegenstand der Erfindung sind Verfahren, die die Herstellung von infektiösen Viruspartikeln, die den erfindungsgemäßen retroviralen Vektor enthalten, ermöglichen. Ein weiterer Gegenstand der vorliegenden Erfindung sind Verfahren, die die Herstellung von stabilen Producer-Zelllinien ermöglichen, die infektiöse Viruspartikel produzieren, die den erfindungsgemäßen retroviralen Vektor enthalten.

Ein weiterer Gegenstand der vorliegenden Erfindung ist die Verwendung der erfindungsgemäßen retroviralen Vektoren für die Expression von Proteinen in verschiedenen Zielzellen. Ebenfalls ein Gegenstand der vorliegenden Erfindung ist die Verwendung der erfindungsgemäßen retroviralen Vektoren für verschiedene gentherapeutische Anwendungen, wie zum Beispiel die adoptive Immuntherapie.

Wirtszellen, wie zum Beispiel T-Zellen, die mit einem erfindungsgemäßen retroviralen Vektor transduziert worden sind, sind ebenfalls ein Gegenstand der vorliegenden Erfindung.

Genauso sind Verfahren ein Gegenstand der vorliegenden Erfindung, gemäß denen Wirtszellen, wie zum Beispiel T-Zellen, unter Verwendung von erfindungsgemäßen retroviralen Vektoren hergestellt werden.

Die erfindungsgemäßen Vektoren der vorliegenden Erfindung weisen eine Nukleotidsequenz der allgemeinen Formel

5'- Ende - [5'- LTR] - [Leader] - [Ex1] - [IRES] - [Ex2] - [3'- LTR] - 3'- Ende

auf, wobei

- die 5'-LTR die Nukleinsäuresequenz für eine beliebige -LTR, eines Retrovirus enthält,
- die Leader region die Nukleinsäuresequenz für die MESV-Leaderregion ohne für virale Proteine oder Teile davon kodierende Sequenzen enthält,
- Ex1 eine Nukleinsäuresequenz enthält, die für ein erstes Transgen kodiert,
- IRES die Nukleinsäuresequenz für eine Internal Ribosomal Entry Site enthält,
- Ex2 eine Nukleinsäuresequenz enthält, die für ein zweites Transgen kodiert,
- die 3'-LTR die Nukleinsäuresequenz für eine 3'-LTR mit der U3-Region der LTR des MPSV-Virus enthält,
- die Leaderregion ausgewählt ist aus den in SEQ ID No.5 und SEQ ID No.6 angegebenen Leadersequenzen, und
- Ex1 die Nukleinsäuresequenz für Herpes Simplex Virus I Thymidin-Kinase und Ex2 die Nukleinsäuresequenz für das $Neo^R$-Resistenzgen enthält.

Überraschenderweise bewirken retrovirale Vektoren, die diese spezifische Kombination von retroviralen und transgenen Sequenzmotiven aufweisen, eine Expression der beiden Transgene, die deutlich über der Expression von Transgenen liegt, wie sie mit retroviralen Vektoren aus dem Stand der Technik erreicht werden kann. Da die erfindungsgemäßen retroviralen Vektoren keine Teile der für retrovirale Proteine wie z.B. gag, pol, env etc. kodierenden Nukleinsäuresequenzen aufweisen, ist die Gefahr der Rekombination der retroviralen Vektoren mit retroviralen Sequenzen in Verpackungszelllinien minimiert, so dass die erfindungsgemäßen retroviralen Vektoren den Sicherheitsansprüchen, die typischerweise an retrovirale Vektoren gestellt werden, die für gentherapeutische Anwendungen eingesetzt werden, genügen.

Als 5'-LTR kann eine beliebige LTR-Region verwendet werden, da nach der Infektion der Wirtszelle mit dem Vektor und nach Ablauf des retroviralen Lebenszyklus die 3'-LTR an die 5'-LTR-Position kopiert wird und die Genexpression des ins Genom integrierten Vektors treibt. 5'-LTR-Regionen stammen beispielsweise aus den Moloney Murine Leukemia Virus (MoMuLV)- oder Moloney Murine Sarcoma Virus (MoMuSV)-Derivaten, wie beispielsweise MPSV, PCMV und MESV. Ebenso geeignet als 5'-LTRs sind 5'-LTRs von Friend Murine Leukemia Virus oder Friend Mink Cell Focus Forming Viruses wie zum Beispiel dem SFFV. Dem Fachmann sind weitere 5'-LTRs aus murinen Leukämieviren bekannt.

Wenn erfindungsgemäß von der MESV-Leaderregion gesprochen wird, handelt es sich dabei um die MESV-Leaderregion, wie sie in der Publikation von Hildinger et al. bezeichnet ist (Hildinger et al., 1999, J. Virol., 73, 4083-4089). Unter Leaderregion von MESV ist damit die Summe von Sequenzen aus dem kurzen direkten terminalen Repeat (R) der U5-Region und der unmittelbar an das 5'-LTR von MESV anschließenden Sequenz zu verstehen, welche sowohl die tRNA-Primer binding site als auch die Packaging-Region (ψ) enthalten. Die Leadersequenz kann maximal aus der vollständigen Sequenz bestehen, die im Virus von R bis zum Beginn der gag-Region (Translationsstart) enthalten ist. Erfindungsgemäß enthält die Leaderregion von MESV jedoch keine Nukleotide, die zur gag kodierenden Nukleotidsequenz gehören.

Die erfindungsgemäßen MESV-Leadersequenzen umfassen eine Spleißdonorsequenz am 5'-Ende der Leadersequenz. Überraschenderweise müssen im Gegensatz zur Lehre von Hildinger et al., 1999 (J. Virol., 73, 4083-4089) erfindungsgemäße retrovirale Vektoren in der MESV-Leadersequenz keine Spleißakzeptorsite am 3'-Ende oder ein mutiertes AUG-Kodon an der Position 270 (gezählt vom Transkriptionsstart, dem Initiationspunkt der retroviralen Transkription an der Grenze zur R-Region in der 5'-LTR) aufweisen, um eine effiziente Expression der beiden Transgene zu gewährleisten. Erfindungsgemäße MESV-Leadersequenzen können damit eine Spleißakzeptorsite am 3'-Ende und ein mutiertes AUG-Kodon an der Position 270 aufweisen, müssen es aber nicht. Erfindungsgemäß weisen MESV-Leadersequenzen keine Nukleinsäuresequenzen auf, die für Teile von viralen Proteinen, d.h. gag, pol, pro oder env kodieren. Erfindungsgemäß umfassen MESV-Leadersequenzen um die 600 bis 800 Basenpaare, bevorzugt um die 600 bis 700 Basenpaare, besonders bevorzugt um die 650 Basenpaare, insbesondere bevorzugt um die 640 Basenpaare und um die 670 Basenpaare, wenn sie eine Spleißakzeptorsite und/oder ein mutiertes AUG-Kodon an der Position 270 aufweisen.

Erfindungsgemäß kann die Internal Ribosomal Entry Site (IRES), über die die Expression des zweiten Transgens gesteuert wird, jede beliebige IRES sein, wie sie dem Fachmann bekannt ist. Typischerweise handelt es sich bei der IRES-Sequenz um die IRES-Sequenz aus dem Polio-Virus. Beispiele für eine verwendbare IRES-Sequenz können den erfindungsgemäßen retroviralen Vektoren entnommen werden. Erfindungsgemäß ist die IRES-Sequenz operativ mit der Nukleinsäuresequenz von Ex2 verknüpft. Dies bedeutet, dass durch die IRES die Expression (d.h. die Translation) der in Ex2 enthaltenen Nukleinsäuresequenzen gesteuert wird. Dem Fachmann ist aber bewusst, dass an der Kontrolle der Expression (Tarnskription und Translation) zusätzlich zur IRES Sequenzen der 3'-LTR und der Leaderregion beteiligt sind.

In bevorzugten Aufführungsformen der vorliegenden Erfindung handelt es sich bei den Nukleinsäuresequenzen des zweiten Transgens um Nukleinsäuresequenzen, die für so genannte selektionierbare Marker kodieren. Dabei handelt es sich um die Nukleinsäuresequenz für $neo^R$. Dem Fachmann sind weitere Sequenzen für selektionierbare Markergene bekannt, die eine Selektion der transfizierten bzw. infizierten Zellen dahin gehend erlauben, dass die im retroviralen Vektor enthaltenen kodierenden Sequenzen stabil in das Genom der Wirtszelle integriert worden sind bzw. dass die Zielzellen erfolgreich mit dem retroviralen Vektor transduziert worden sind.

Die 3'-LTR von erfindungsgemäßen retroviralen Vektoren umfasst die LTR des MPSV-Virus. Dies bedeutet insbesondere, dass die 3'-LTR von erfindungsgemäßen retroviralen Vektoren in der Form des Plasmids die Promoter/Enhancer-Sequenzen der U3-Region der LTR des MPSV-Virus enthalten. Die LTR-Sequenz von MPSV kann den in den Beispielen angegebenen Vektoren pMPSV11TIN, pMPSV11T*IN, pMPSV71TIN, pMPSV71T*IN, pMPSV91TIN und pMPSV91T*IN entnommen werden (3'-LTR). Erfindungsgemäß sind mit der LTR von MPSV auch solche Nukleinsäuresequenzen gemeint, die eine hochgradige Homologie zu der LTR-Sequenz des MPSV aufweisen, d.h. die zumindest zu 90% identisch, bevorzugt zu mindestens 95% identisch, und insbesondere bevorzugt zu mindestens 98% identisch zu den LTR-Sequenzen des MPSV sind.

Überraschenderweise hat sich gezeigt (siehe Beispiele), dass die genannte Kombination von spezifischen regulatorischen retroviralen Sequenzen (MESV-Leaderregion ohne gagkodierende Sequenzen), 3'-LTR-Sequenzen des MPSV sowie die Verwendung einer IRES zur Steuerung der Expression des zweiten Transgens die Herstellung von retroviralen Vektoren ermöglicht, die beide Transgene mit hoher Effizienz exprimieren. Der Stand der Technik (siehe oben) hat im Gegensatz zur vorliegenden Erfindung nahe gelegt, dass bei der Herstellung von bicistronischen retroviralen Vektoren eine effiziente Expression des zweiten Transgens insbesondere durch Verwendung einer Spleißakzeptorsite erzielt werden könne, während für die effiziente Expression des ersten Transgens die Kombination eines MESV-Leaders, der keine gag-kodierenden Sequenzen, kein AUG und eine Spleißakzeptorsite sowie die 3'-LTR eines Friend-Mink Cell Focus Forming Virus, bevorzugt des SFFV, enthält, zu verwenden sei.

Überraschenderweise haben die im Rahmen der vorliegenden Erfindung durchgeführte Experimente gezeigt, dass mit den erfindungsgemäßen Vektoren eine Expression sowohl des ersten Transgens (im vorliegenden Fall HSV-tk) als auch des zweiten Transgens ($neo^R$) erreicht werden kann, die deutlich über dem Expressionslevel liegt, wie er für bekannte retrovirale Vektoren des Standes der Technik beobachtet wird.

Die erfindungsgemäßen retroviralen Vektoren können benutzt werden, um eine Vielzahl von hämatopoetischen Zellen zu transfizieren, die bis dato häufig unter einer mangelnden Expression der transgenen Sequenzen gelitten haben.

Gemäß einer weiteren Ausführungsform der Erfindung weisen die Nukleinsäuresequenzen der Transgene, die für Proteine kodieren, bevorzugt solche Mutationen auf, die, ohne Veränderung der durch die Nukleinsäuresequenz kodierten Proteinsequenz, zur Entfernung kryptischer Spleißstellen und/oder kryptischer Poly-A-Stellen führen. Dadurch wird vermieden, dass die durch Transkription der für die Proteine kodierenden Nukleotidsequenzen entstehenden mRNAs falsch gespleißt werden oder zu kurz sind, wenn z.B. der Poly-A-Schwanz zu früh angehängt wird (Mclfor, R.S., 1990, Virology, 176, 652-655; Johnson, J.J. et al., 1995, Hum. Gene Ther., 6, 611-623).

Die bevorzugte Methode zur Einführung von Mutationen ist die "Site Directed Mutagenesis" durch die Polymerase-Kettenreaktion. Dem Fachmann sind mehrere Literaturstellen bekannt, denen die genaue Durchführung dieser Methode zu entnehmen ist (siehe zum Beispiel Ausubel, I. et al., 1994, Current Protocols in Molecular Biology, John Wiley und Sons, New York, und Sambrook et al., 2001, Molecular Cloning: A Laboratory Manual, Coldspring Harbour Laboratory Press). Der Fachmann ist sich bewusst, dass auch andere Techniken verwendet werden können, um gezielt Nukleotidmutationen, d.h. Insertionen, Deletionen oder Substitutionen in Nukleinsäuresequenzen einzufügen.

In einer anderen bevorzugten Ausführungsform der Erfindung sind die Nukleinsäuren der Transgene, die für Proteine kodieren, dahin gehend modifiziert, dass sie keine AUG-Triplets enthalten, die zu einem fehlerhaften Start der Translation der transgenen Sequenz führen könnten. In einer weiteren bevorzugten Ausführungsform der Erfindung sind die eigentlichen AUG-Startkodons hinsichtlich ihrer Erkennung durch die Transkriptionsmaschinerie durch Optimierung der so genannten Kozak-Konsensussequenz verbessert (Krall, W.J. et al., 1996, Gene Ther., 3, 37-48). Je nach Funktion der durch die Nukleinsäuren der transgenen Sequenzen kodierten Proteine können die Nukleinsäuresequenzen der Transgene zusätzliche dem Fachmann geläufige Signalsequenzen, wie zum Beispiel Kernlokalisationssignal-, Sekretions- oder allgemein Translokalisationssequenzen enthalten.

Bei der vorliegenden Erfindung handelt es sich um die erfindungsgemäßen retroviralen Vektoren pMPSV11TIN, pMPSV11T*IN, pMPSV91TIN und pMPSV91T*IN.

Der als pMPSV11TIN bezeichnete Vektor zeichnet sich dadurch aus, dass sämtliche Nukleotidsequenzen entfernt wurden, die für retrovirale Proteine kodieren, wie sie in den Retroviren, von den sich der Vektor ableitet (z.B. gag, pol etc.) vorkommen. Am 5'-Ende des DNA-Konstrukts befinden sich die U3-, R- und US-Regionen des 5'-LTR. Im 5'-LTR liegt die CAP-Stelle, an der die Transkription der viralen mRNA beginnt. 3' von 5'-LTR liegt die MESV-Leaderregion, die einen Spleißdonor und eine Verpackungsregion (ψ), aber keinen Spleißakzeptor enthält. Das Nukleotid 270 (gezählt ab CAP-Stelle) wurde nicht mutiert. Insofern befindet sich dort nach wie vor ein kryptisches AUG. 3' von der MESV-Leadersequenz befinden sich die kodierenden Sequenzen für das HSV-tk-Gen. Darauf folgt eine IRES aus dem Polio-Virus, die dann operativ mit der kodierenden Sequenz für $neo^R$ verbunden ist. 3' von der kodierenden Sequenz für das Neomycinresistengen befindet sich die 3'-LTR mit den U3-, R- und U5-Regionen, wobei insbesondere die U3-Region die Sequenzen des MPSV enthält.

Der als pMPSV11T*IN bezeichnete Vektor weist zwei mutierte Sequenzbereiche auf, durch die zwei kryptische Spleißmotive entfernt wurden (siehe Beispiele). Ansonsten ist er identisch zu pMPSV11TIN.

Der als pMPSV71TIN bezeichnete retrovirale Vektor (nicht Gegenstand der Erfindung) zeichnet sich dadurch aus, dass ebenfalls sämtliche Nukleotidsequenzen entfernt wurden, die für retrovirale Proteine kodieren wie sie in den Retroviren, von den sich der Vektor ableitet (z.B. gag, pol etc.), vorkommen. Zudem weist er die gleiche 5'-LTR auf wie der erwähnte pMPSV11TIN. Im MESV-Leaderbereich weist der pMPSV71TIN ebenfalls einen Spleißdonor sowie eine Verpackungsregion (ψ) auf. Zusätzlich enthält er einen Spleißakzeptor am 3'-Ende der MESV-Leaderregion. Die Bereiche 3' vom Spleißakzeptor sind zu den Bereichen des pMPSV11TIN identisch.

Der als pMPSV71T*IN bezeichnete Vektor (nicht Gegenstand der Erfindung) unterscheidet sich von pMPSV71TIN lediglich darin, dass im HSV-tk-Gen zwei kryptische Spleißmotive entfernt wurden (siehe Beispiele).

Der als pMPSV91TIN bezeichnete Vektor unterscheidet sich vom pMPSV71TIN-Vektor lediglich darin, dass in der MESV-Leaderregion das Nukleotid 270 (gezählt ab CAP-Stelle) mutiert wurde, um ein kryptisches AUG zu entfernen. Ansonsten ist der Vektor mit pMPSV71TIN identisch.

Der als pMPSV91T*IN bezeichnete Vektor unterscheidet sich von pMPSV91TIN lediglich darin, dass im HSV-tk-Gen zwei kryptische Spleißmotive entfernt wurden (siehe Beispiele).

Die Sequenzen für die genannten und andere erfindungsgemäßen Vektoren, d.h. die MPSV-LTR-Sequenzen, die jeweiligen MESV-Leadersequenzen, die HSV-tk-Sequenzen, die IRES-Sequenzen und die $neo^R$-Sequenzen und die Herstellung der erfindungsgemäßen Vektoren sind explizit weiter unten bei den Beispielen dargestellt.

Die erfindungsgemäßen retroviralen Vektoren und insbesondere die als pMPSV11TIN, pMPSV11T*IN, pMPSV91TIN und pMPSV91T*IN bezeichneten retroviralen Vektoren eignen sich in hervorragender Weise zur Transduktion hämatopoetischer Stammzellen. Daneben eignen sie sich ebenfalls in hervorragender Weise zur Transduktion von Zelltypen, die aus hämatopoetischen Stammzellen gebildet werden, insbesondere zur Transduktion von Leukozyten und insbesondere zur Transduktion von T-Lymphozyten und B-Lymphozyten..

Damit eignen sich die erfindungsgemäßen Vektoren im Besonderen für gentherapeutische Anwendungen.

Bisher ist nur in wenigen Fällen von Gentherapien, die auf retroviralen Vektoren beruhen; durch klinische Studien eine positive Funktion der genetisch modifizierten Zellen nach-gewiesen worden (Blaese et al., 1995, Science 270, 475-480 und Losordo et al.,1998, Circulation 98, 2800-2804). Die geringe Expression von Transgenen wurde in diesem Zusammenhang als der Hauptgrund dafür angesehen, dass die genmodifizierten Zellen in der Regel nicht in der Lage waren, die Krankheit komplett zu heilen. Allerdings ist durch zwei unabhängige klinische Studien die generelle Machbarkeit von spezifischen gentherapeutischen Ansätzen gezeigt worden (Bonini et al., 1997, vide supra sowie Tiberghien et al., 1997, vide supra). Bei diesem Ansatz wurden so genannte retrovirale Selbstmord-Genvektoren benutzt, um die GvHD zu kontrollieren, die als eine der Hauptkomplikationen bei der adoptiven Immuntherapie auftritt.

Trotz der mit ihr verbundenen Risiken ist die adoptive Immuntherapie dabei, die reguläre Therapie für die Behandlung von Leukämien und anderen Tumorarten zu werden. Dies beruht auf den vielfältigen Vorteilen, die die transplantierten Donor-T-Zellen mit sich bringen. Zum Beispiel ist für allogenische T-Zellen gezeigt worden, dass sie die Annahme des Knochenmarktransplantats durch den Empfänger erhöhen (Martin et al., 1985, Blood 66, 664-672). Zusätzlich fungieren sie als ein frühzeitig angepasstes Immunsystem, da sie nach der Transplantation Tumorzellen und Virus-infizierte Zellen identifizieren und töten können. Diese vorteilhaften Reaktionen der allogenischen T-Zellen sind als der Graft versus Leukemia (GvL)-Effekt und Graft versus Infection (GvI)-Effekt bezeichnet worden (Horowitz et al., 1990, Blood 75, 555-562).

Allerdings können die allogenischen T-Zellen auch mit normalen Geweben des Patienten reagieren, insbesondere dann, wenn ein teilweise unverträgliches Transplantat benutzt wird, wenn eine große Anzahl an T-Zellen transplantiert wird oder wenn Stammzelltransplantate des peripheren Blutsystems benutzt werden (Brown et al., 1999, J. Clin. Oncol. 17, 806-812). Diese Abstoßungsreaktion ist als GvHD bezeichnet worden, die in unterschiedlichen Stufen auftritt und je nach Schwere für den Patienten lebensbedrohlich werden kann (Goker et al., 2001, Exp. Hematol. 29, 259-277).

Im Stand der Technik sind verschiedene Ansätze beschrieben worden, um den GvL- und den GvI-Effekt von der GvHD zu trennen (Slavin et al., 1998, Blood 91, 756-763; Kottaridis et al., 2000, Blood 96, 2419-2425; Schmaltz et al., 2001, Transplant 97, 2886-2895). Bis heute gibt es kein experimentelles Protokoll, das einen effizienten GvL-Effekt garantieren kann, ohne dass das Risiko einer gefährlichen GvHD auftritt. Hinzu kommt, dass das Auftreten und das Voranschreiten der GvHD bei schwereren Formen noch immer nicht vorhersagbar ist. Entsprechend ist versucht worden, Methoden zu entwickeln, die es erlauben, den GvHD-Effekt spezifisch zu kontrollieren. Einer der Ansätze, der in diesem Zusammenhang diskutiert worden ist, beinhaltet die retrovirale Expression von Selbstmord-Genen wie der HSV-tk (Bonini et al., 1997, Science, 276, 1719-1724 und Tiberghien et al., 1997, Hum. Gene Ther., 8, 615-624). Das Problem, das in diesen Studien auftrat, war, dass alloreaktive T-Zellen bei manchen Patienten nicht ausreichend selektioniert werden konnten, um die Patienten von schweren Formen der GvHD zu heilen. Dies führte zu der Annahme, dass die durch retrovirale Vektoren vermittelte Expression der HSV-tk nicht ausreichend war. Manche Forscher haben vorgeschlagen, größere Anzahlen an T-Zellen zu transplantieren, um den GvL Effekt zu erhöhen. Mit der Anzahl der transplantierten allogenen T-Zellen steigt jedoch auch die Gefahr einer GvHD. Eine Alternative besteht natürlich darin, T-Zellen einzusetzen, die das Transgen des retroviralen Vektors effizient exprimieren und somit eine sensitive Selektion der transduzierten T-Zellen ermöglichen.

Aus dem vorher Gesagten wird deutlich, dass für eine effektive und sichere Gentherapie, die auf retroviralen Selbstmord-Vektoren beruht, verbesserte retrovirale Expressionsvektoren benötigt werden.

Wie die im Folgenden ausgeführten Beispiele zeigen, haben die erfindungsgemäßen retroviralen Vektoren die Eigenschaft, dass sie zu hohen Virustitern in VerpackungsZelllinien und den Producer-Zelllinien führen. Sie erlauben darüber hinaus eine effiziente Transduktion der Zielzellen und integrieren in das Genom der Zielzellen, ohne dass es zu Umlagerungen der retroviralen Sequenzen kommt. Vor allem aber bewirken die erfindungsgemäßen retroviralen Vektoren eine wesentlich höhere Expression des HSV-Thymidinkinasegens als alle anderen aus dem Stand der Technik bekannten retroviralen Vektoren. Ohne an eine wissenschaftliche Theorie gebunden sein zu wollen, wird vermutet, dass diese verbesserte Expressionsfähigkeit der erfindungsgemäßen retroviralen Vektoren auf der spezifischen Kombination von retroviralen Regulationssequenzen wie der MESV-Leader-Sequenz, der 3'-LTR von MPSV und Regulationssequenzen wie der IRES, die die Expression des zweiten Transgens steuert, beruht.

Verbesserte Expressionsfähigkeit der erfindungsgemäßen Vektoren bedeutet im Rahmen der vorliegenden Erfindung, dass ein vergleichsweise höherer Anteil an funktionellen Transgenen bei gleichen Absolutmengen oder gleicher Kopienanzahl der Transgene exprimiert wird. Dies äußert sich im Falle von z. B. HSV-tk in einer erhöhten Sensitivität der transduzierten Wirtszellen gegenüber dem Selektionsmittel GCV (siehe unten), wenn gleiche Virustiter von Producer-Zelllinien, die jeweils die gleiche Kopienanzahl des Transgens in ihr Genom integriert haben, aber unter Verwendung unterschiedlicher Vektoren hergestellt wurden, zur Transduktion der Wirtszellen eingesetzt werden. Verbesserte Expressionsfähigkeit bedeutet im Rahmen der vorliegenden Erfindung auch, dass eine absolut höhere Menge an Transgen, also z.B. HSV-tk exprimiert wird. Entsprechend ist die Angabe einer höheren Expression eines Transgens dahingehend zu verstehen, dass hinsichtlich der Funktion oder der Absolutmenge des Transgens eine verbesserte Expression vorliegt. Die Angabe einer niedrigen oder schlechteren Expression sind entsprechend zu verstehen.

Die verbesserte Expressionsfähigkeit der erfindungsgemäßen retroviralen Vektoren bringt wesentliche Vorteile für die adoptive Immuntherapie mit sich. Für den Fall, dass eine GvHD-Reaktion auftritt, ist aufgrund der hohen Expression der HSV-tk gewährleistet, dass mit bereits geringen Mengen an Ganciclovir selektiv und hochspezifisch die allogenen T-Zellen, die für den GvHD-Effekt verantwortlich sind, abgetötet werden können. Da es sich bei Ganciclovir um ein Nukleotid-Analogon handelt, ist es per se wünschenswert, dass nur geringe Konzentrationen dieses Nukleotid-Analogons verwendet werden müssen, da es auch in anderen replizierenden Zellen des menschlichen Körpers möglicherweise in die DNA eingebaut wird und zum Absterben der Zellen führt. Darüber hinaus erlaubt eine effiziente und selektive Abtötung der T-Zellen, dass die GvHD effizient kontrolliert werden kann und damit potentiell höhere Mengen allogener T-Zellen mit dem Ziel der Verbesserung der GvL-Reaktion appliziert werden können.

Gegenstand der vorliegenden Erfindung ist daher die Verwendung der erfindungsgemäßen retroviralen Vektoren für verschiedene gentherapeutische Anwendungen, insbesondere für die adoptive Immuntherapie. In einer bevorzugten Ausführung der Erfindung werden die erfindungsgemäßen Vektoren verwendet, um eine effiziente Depletion von allo-reaktiven Zellen, wie sie nach einer Knochenmarktransplantation oder einer Lymphozyten-Infusion auftreten, zu gewährleisten.

In einer weiteren Ausführungsform der Erfindung werden die erfindungsgemäßen Vektoren benutzt, um infektiöse, nicht-replikationskompetente Viruspartikel herzustellen. Die dem Verfahren zugrunde liegenden Techniken sind dem Fachmann bekannt (Ausubel, I. et al., 1994, vide supra). Mit Hilfe einer verpackungskompetenten Zelllinie, die das Genom eines Helfervirus enthält, werden infektiöse Viruspartikel hergestellt, die den erfindungsgemäßen retroviralen Vektor enthalten. Das Helfervirus wird als replikations-inkompetentes Partikel von den Verpackungszellen produziert. Dafür ist das Genom des Helfervirus gentechnisch so verändert worden, dass die Virusprotein-kodierende RNA des Helfervirus nicht in die Viruspartikel verpackt werden kann, da sie nicht über das Verpackungssignal verfügt (Miller, A.D. et al., vide supra). Bei Verpackungszelllinien kann es sich erfindungsgemäß um PG13 oder PG53 (Miller et al., 1991, J. Virol., 65, 2220-2224), GP+envAM12 (Markowitz et al., 1988, 167, 400-406), FLY (Cosset et al., 1995, J. Virol., 69, 7430-7436), PA12 (Miller et al. 1985, Mol. Cell Biol., 5, 431-437), PA317 (Miller and Buttimore, 1986, Mol. and Cell. Biol., 6, 2895-2902) , Psi2 (Mann et al., 1983, Cell, 33, 153-159), und 293 GPG (Ory et al., 1996, Proc. Natl. Acad. Sci. USA, 93, 11400-11406) handeln.

Wenn ein retroviraler Vektor stabil in das Genom einer Verpackungszelllinie integriert wird, entsteht eine so genannte "Producer-Zelllinie". Producer-Zelllinien produzieren permanent infektiöse, replikationsdefekte Viruspartikel und können, da der retrovirale Vektor sich in das Genom der Verpackungszelllinie integriert hat, hinsichtlich ihrer genomischen Struktur analysiert werden. Dies hat den Vorteil, dass solche Producer-Zelllinien entwickelt werden können, die nur eine Kopie des integrierten retroviralen Vektor aufweisen. Solche Producer-Zelllinien, die ebenfalls ein Gegenstand der vorliegenden Erfindung sind, erlauben durch Bestimmung des Virustiters die Aussage, welche retroviralen Sequenzen bzw. nicht-retroviralen Sequenzen der erfindungsgemäßen retroviralen Vektoren für einen hohen Virustiter verantwortlich sind. In gleicher Weise können Rückschlüsse hinsichtlich des Einflusses der verschiedenen Sequenzen auf die Expressionsfähigkeit des Vektors gezogen werden.

Gegenstand der vorliegenden Erfindung ist ferner eine Wirtszelle, die mit dem erfindungsgemäßen retroviralen Vektor transduziert worden ist. Bevorzugt wird die Wirtszelle mit einem infektiösen Viruspartikel infiziert, das den erfindungsgemäßen retroviralen Vektor enthält. Diese Wirtszelle kann ausgewählt werden aus verfügbaren immortalisierten hämatopoetischen Zelllinien (Baum, C. et al., 1995, J. Virol., 69, 7541-7547) oder primären menschlichen Blutzellen (Eckert, H.G. et al., 1996, Blood, 88, 3407-3415), ist aber nicht auf diese beschränkt. Vorzugsweise ist die Wirtszelle eine T-Zelle (T-Lymphozyten), die mit den erfindungsgemäßen Vektoren transduziert worden ist. Weitere Zelllinien und Zelltypen, die mit den erfindungsgemäßen Vektoren bevorzugt transduziert werden können, sind insbesondere hämatopoetische Stammzellen, B-Lymphozyten, sowie die hämatopoetischen Vorläuferzellen der lymphoiden, der myeloiden und der erythroiden Entwicklungsreihen, terminale Entwicklungsstufen (reifen Zellen) dieser Entwicklungsreihen.

Erfindungsgemäß können die erfindungsgemäßen retroviralen Vektoren nicht nur für verschiedene gentherapeutische Anwendungen eingesetzt werden. Sie können ganz allgemein auch dafür verwendet werden, um Proteine in Säugerzellen zu exprimieren. Dabei erlaubt die Expression des zweiten Transgens zunächst die Selektion von Zellen, die den retroviralen Vektor erfolgreich in ihr Genom integriert haben und die deswegen stabil selektioniert werden können. Durch das erste Transgen können dann die gewünschten Proteine exprimiert werden.

Die Selektions- und Anzuchtsbedingungen von solchen transfizierten oder transduzierten Zellen sind dem Fachmann bekannt (Ausubel et al., 1994, vide supra). Danach wird das erzeugte Protein mit Hilfe von Techniken gereinigt, die dem Fachmann ebenfalls bekannt sind (Ausubel et al., 1994, vide supra). Die Reinigung kann dabei z.B. durch klassische biochemische Fraktionierung erfolgen. Je nachdem ob die transgenen Sequenzen kodierende Sequenzen für so genannte Affinitäts-Marker tragen (wie His-, myc-, GST-Tags), kann die Reinigung der exprimierten Proteine auch über entsprechende Affinitätschromatographie erfolgen.

Die erfindungsgemäßen retroviralen Vektoren können auch für gentherapeutische Anwendungen eingesetzt werden, wo anders als bei der adoptiven Immuntherapie, nicht die Eliminierung eines spezifischen Zelltyps das Ziel der Therapie ist, sondern vielmehr die Expression eines Proteins, das in den Wirtszellen nicht oder nur in einer mutierten und nicht funktionellen Form exprimiert wird. In einer besonders bevorzugten Ausführungsform werden die erfindungsgemäßen retroviralen Vektoren benutzt, um zum Beispiel für die Gentherapie bei dem Severe Combined Immuno Deficiency-Syndrome (SCID) eingesetzt zu werden. Die Nukleinsäuresequenz des ersten Transgens kann in diesem Fall dann die Nukleinsäuresequenz für das Adenosindeaminase Gen umfassen. Andere entsprechende therapeutische Anwendungen der erfindungsgemäßen retroviralen Vektoren, die darauf abzielen, in den Wirtszellen der Patienten ein nicht funktionelles Proteinprodukt durch ein funktionelles Proteinprodukt zu ersetzen, sind dem Fachmann aus dem Stand der Technik bekannt.

Gemäß einer besonderen Ausführungsform der Erfindung findet der retrovirale Gentransfer- bzw. Expressionsvektor Verwendung in der Gentherapie. Dies schließt ein, dass der Vektor in einem pharmazeutischen Präparat derart vorliegt, dass er in die Zielzelle eingeführt werden kann. Bevorzugt liegt der erfindungsgemäße retrovirale Vektor in einem infektiösen Viruspartikel verpackt vor. Bevorzugt wird der retrovirale Vektor zur Transfektion hämatopoetischer Stammzellen verwendet (Baum, C. et al., 1997 in: Concept in Gene Therapy, eds. M. Straus, W. Barrangier, De Gruyter-Verlag, Berlin, S. 233-266).

Im Folgenden werden die verschiedenen Ausführungsformen der Erfindung an einer Reihe von Beispielen dargestellt. Zum Beispiel wird gezeigt, dass die Nukleinsäuresequenz des ersten Transgens das HSV-tk-Gen enthält, während die Nukleinsäure des zweiten Transgens das Neomycin-Resistenzgen ($neo^R$) enthält. Darüber hinaus wird gezeigt, dass die erfindungsgemäßen retroviralen Vektoren jeweils eine effizientere Expression der Transgene bewirken, als dies für bekannte retrovirale Vektoren beobachtet worden ist. Die Erfindung ist dabei keineswegs auf die in den Beispielen genannten Ausführungsformen zu beschränken, vielmehr sind dem Fachmann anhand der Beschreibung weitere Ausführungsformen vorstellbar und durchführbar.

Wie oben ausgeführt, ist durch verschiedene Studien gezeigt worden, dass die Kontrolle der GvHD durch einen Selbstmord-Mechanismus erfolgreich sein kann. Allerdings besteht nach wie vor großer Bedarf an retroviralen Vektoren, die eine effiziente Expression des Selbstmord-Gens und eine effiziente Selektion der transduzierten Wirtszellen erlauben. Um retrovirale Vektoren herzustellen, die eine effiziente Depletion von allo-reaktiven Zellen ermöglichen, wurden im Rahmen der Erfindung 21 neue retrovirale Vektoren hergestellt, die sich in ihren LTRs, in den Leaderregionen und in der Koexpressions-Strategie für die zwei selektionierbaren Marker Thymidinkinase von Herpes simplex-Virus 1 und Neomycinphosphotransferase (Neomycin-Resistenzgen, $neo^R$) unterscheiden. Zusätzlich wurde im Rahmen der vorliegenden Erfindung ein Zwei-Stufen-Selektionssystem entwickelt (1. G418 = Geneticin, 2. Ganciclovir), das die Analyse der biologischen Funktion der retroviralen Vektoren in vitro erlaubte.

Die Funktion der neuen Vektoren wurde direkt mit der Funktion von retroviralen Selbstmord-Vektoren verglichen, die aus dem Stand der Technik bekannt waren und speziell für die Behandlung der GvHD hergestellt worden waren (siehe Hildinger et al., 1999, J. Virol., 73, 4083-4089; Hildinger et al., 1999, Gene Ther., 6, 1222-1230).

Die neuen Selbstmord-Genvektoren wurden hergestellt, indem die selektionierbaren Marker HSV-Thymidinkinase (tk) und Neomycinphosphotransferase ($neo^R$) mit verschiedenen retroviralen Sequenzen kombiniert wurden. Es wurden verschiedene LTRs von verschiedenen murinen Retroviren hinsichtlich ihres Einflusses auf die Expression der Transgene analysiert. Dazu gehörten die LTRs des MoMuLV, des MPSV und des SFFV. Für die Koexpression der zwei erwähnten Transgene wurde in den bicistronischen retroviralen Vektoren der Elongationsfaktor 1$\alpha$-Promotor (Uetsuki et al., 1989, J. Biol. Chem. 264, 5791-5798; Wakabayashi-Ito und Nagata, 1994, J. Biol. Chem. 269, 29831-29837) als ein interner Promotor für die Expression von tk analysiert. Zusätzlich wurde der Einfluss einer zweiten Spleißakzeptorsite oder einer IRES vom Poliovirus untersucht, um die effizienteste Expressionsstrategie für das zweite Transgen zu bestimmen.

Wie im Folgenden gezeigt, wurde überraschend gefunden, dass die Kombination einer MPSV-LTR, einer optimierten MESV-Leadersequenz und einer tk-IRES-$Neo^R$-Expressionskassette die beste Expression der beiden Transgene und damit die effizienteste Selektion der transduzierten Wirtszellen erlaubte. Dies war möglich, weil durch die vorliegende Erfindung erstmals gezeigt wurde, dass nur eine gründliche genomische Analyse der Producer-Zelllinien einen aussagekräftigen Vergleich der verschiedenen Vektoren zulässt.

<u>Materialien und Methoden</u>

Im Folgenden werden die Materialien und Methoden angegeben, die für die Herstellung der verschiedenen Zelllinien und Vektorkonstrukte benutzt wurden.

<u>Zelllinien</u>

PG13 galv-Zellen (ECACC-Nr. 95110215) (Miller et al., 1991, J. Virol., 65, 2220-2224) wurden von der Europäischen Zellkultursammlung bezogen. GP+envAM12-Zellen (ATCC-Nr. CRL-9641) (Markowitz et al., 1988, Virol., 167, 400-406) und Jurkat-Zellen (ECACC-Nr. 88042803) wurden freundlicherweise von Wolfram Ostertag vom Heinrich-Pette-Institut (Hamburg, Deutschland) zur Verfugung gestellt. PG13 galv-Zellen und GP+envAM12-Zellen wurden in DMEM-Medium (Biowhittaker Europe, Verviers, Belgien) und Jurkat-Zellen in RPMI 1640-Medium (Gibco BRL Life Technologies Ltd., Paisley, Großbritannien) kultiviert. Beide Medien waren mit 10% FCS (Sigma Chemical Co., St. Louis, USA), 1 mM NatriumPyruvat-Medium (Gibco BRL Life Technologies Ltd., Paisley, Großbritannien), 2 mM L-Glutamin (Gibco BRL Life Technologies Ltd., Paisley, Großbritannien) und 50 IU/ml Penicillin - 50 µg/ml Streptomycin (Gibco BRL Life Technologies Ltd., Paisley, Großbritannien) versetzt.

Vektorherstellung

a) Klonierung des Vektors pMO3TIN Die 3'-LTR des Vektors pMO3TIN enthält die MoMLV-Enhancer/Promoter-Sequenzen zwischen NheI und KpnI von MO-CAT (Baum et al., 1995, J. Virol., 69, 7541-7547). In dem Ausgangsvektor pMO1 wurde der MESV-Leader zwischen KpnI und PstI gegen den MoMSV-Leader (V-MDR Fragment) ausgetauscht. Es resultierte der Vektor pM03 (Hildinger et al., 1998, Gene Therapy, 5, 1575-1579). Durch Klonierung der neo$^R$ cDNA aus p50-M-neo (Laker et al., 1998, J. Virol., 72, 339-348) als Not-Hind-Fragment (SEQ ID NO: 10) in den Vektor pMO3 wurde der Vektor pM03N (Hildinger et al., 1998, Gene Therapy, 5, 1575-1579) erhalten. In den mit Not I geöffneten Vektor pMO3N wurden das Thymidinkinasegen aus Herpes Simplex Virus (HSV-tk) als Not I-Hind III-Fragment (SEQ ID NO: 7) und die Interne Ribosomale Eintrittsstelle aus Polio Virus (IRES) als Hind III-Not I-Fragment (SEQ ID NO: 9) ligiert. Hieraus resultierte der Vektor pM03TIN (SEQ ID NO: 4).
Die Klonierung des HSV-tk (GenBank acc. number AF057310) erfolgte über PCR mit:

Forward Primer [5'-AAGCGGCCGCCATGGCTTCGTACCCCTGCCATCAACACGC-3'] und

Reverse Primer [5'-AAAGCTTATCAGTTAGCCTCCCCCATCTCCCGGGCAAACG-3'].

b) Klonierung des Vektors pMPSV11TIN
Dieser Vektor wurde auf der Basis des in der Arbeitsgruppe Dr. C. Baum, Heinrich-Pette-Institut, Hamburg, klonierten Vektors pMPSV11d2EGFP (SHX) Vektors konstruiert. Dieser Vektor enthält die Enhancer/Promotor-Sequenzen des MPSV (Myeloproliferatives Sarkom Virus) in den U3-Regionen sowohl der 3'-LTR als auch der 5'-LTR. Die 5'-UTR (5' untranslated region) enthält den Leader11 (SEQ ID NO: 5). Die Leaderregion kann durch Kpn I+Not I-Spaltung ausgetauscht werden. Die d2EGFP-Sequenz kann durch Spaltung mit den Restriktionsenzymen Not I und Hind III entfernt bzw. ausgetauscht werden.
Für die Herstellung des Vektors pMPSV 11 TIN wurde zunächst die Vorstufe pMPSV11Neo kloniert. Hierfür wurde der Vektor pMPSV11d2EGFP (SHX) mit Not I und Hind III gespalten, um die d2EGFP-Sequenz zu entfernen. Das Rückgrat pMPSV11 wurde als Not I-Hind III-Fragment nach Aufreinigung über Agarosegelelektrophorese isoliert. Die NeoR kodierende Sequenz wurde durch Spaltung des Vektors pMO3TIN mit Not I und Hind III erhalten, ebenfalls über Agarosegelelektrophorese aufgereinigt und in das pMPSV11 Rückgrat kloniert. Hieraus resultierte der Vektor pMPSV11Neo.
Der Vektor pMPSV11Neo wurde mit Not I linearisiert, gelelektrophoretisch gereinigt und anschließend mit alkalischer Phosphatase dephosphoryliert. Das Thymidinkinase-IRES-Fragment wurde durch Spaltung von MO3TIN mit Not I aus dem Vektor MO3TIN erhalten, gelelektrophoretisch gereinigt und in das mit Not I linearisierte pMPSV11Neo Rückgrat kloniert. Hieraus resultierte der Vektor MPSV 11 TIN (SEQ ID NO: 1)
c) Klonierung des Vektors pMPSV91 TIN
Für die Klonierung dieses Vektors wurde das Rückgrat aus dem Plasmid pSFβ91 (SHX) (Arbeitsgruppe Dr. C. Baum, Heinrich-Pette-Institut, Hamburg) benutzt. Die SFβ-Sequenzen des Vektors pSFβ91 (SHX) wurden durch gleichzeitige Spaltung mit Not I und Xho I entfernt. Nach Auftrennung der Spaltprodukte durch Agarose-Gelelektrophorese wurde das Plasmidrückgrat mit der 5'-LTR und dem Leader 91 (SEQ ID NO: 6) isoliert. Aus dem Vektor PMPSV11Neo wurde das Neo-3'MPSV-LTR-Fragment durch Spaltung mit Not I und Xho I erhalten. Nach Auftrennung der Spaltprodukte durch Agarose-Gelelektrophorese wurde das Neo-3'MPSV-LTR-Fragment isoliert und in das Plasmidrückgrat mit der 5'-LTR und dem Leader 91 ligiert. Das resultierende Plasmid pMPSV91Neo wurde mit Not I geöffnet. Durch Spaltung von pMO3TIN mit NotI wurde das TK-IRES-Fragment erhalten und anschließend in den geöffneten pMPSV91Neo ligiert. Hieraus resultierte der Vektor pMPSV91TIN (SEQ ID NO: 2).
d) Klonierung des Vektors pMPSV11T*IN
Der Vektor pMPSV11 T*IN (SEQ ID NO: 3) ist bis auf zwei zerstörte Spleiß-Motive in der HSV-tk-Sequenz mit dem Vektor pMPSV11TIN (SEQ ID NO:1) identisch. Die Veränderungen wurden in den von Garin et al. beschriebenen Sequenzmotiven (Garin et al., 2001, Blood 97: 122-129) durchgeführt. Zur Einführung der Mutationen wurde das tk-Gen aus dem Plasmid pMPSV11TIN als NotI-HindIII-Fragment in das Plasmid pBlueScript II SK (Stratagene) subkloniert (siehe unten). Durch Site-Directed Mutagenesis mit Hilfe des QuikChange™ Site-Directed Mutagenesis Kits von Stratagene wurden die Spleiß-Motive sequentiell zerstört. Für die Mutation der putativen Spleißdonor-Stelle wurden als Forward Primer TKdSD5 (SEQ ID NO: 11) und als Backward Primer TKdSD3 (SEQ ID NO: 12) verwendet.

Für die Mutation der putativen Spleißakzeptor-Stelle wurden als Forward Primer TKdSA5 (SEQ ID NO: 13) und als Backward Primer TKdSA3 (SEQ ID NO: 14) verwendet.

Die Herstellung von Plasmid pMPSV11T*IN erfolgte in den folgenden Schritten:

Zunächst wurden durch Spaltung von pMPSV11TIN mit NotI und HindIII die inserierten Fremdsequenzen (TK, IRES, Neo) entfernt. Nach Auftrennung der Spaltprodukte durch Agarosegelelektrophorese wurde das Plasmid-Rückgrat (pMPSV11) isoliert. Das Spleißbereinigte tk Gen (SEQ ID NO: 8) wurde als NotI-HindIII-Fragment aus dem Bluescript Plasmid isoliert (siehe oben) und anschließend in das pMPSV11-Rückgrat inseriert. Es resultierte das Plasmid pMPSV11T*. Aus dem Plasmid pMPSV91 TIN wurde durch Spaltung mit HindIII das IRES-Neo-Fragment freigesetzt und in das mit HindIII linearisierte Plasmid pMPSV11T* kloniert. Hieraus resultierte der Vektor MPSV11T*IN. Die korrekte Orientierung der IRES-Neo-Sequenzen wurde durch Sequenzanalyse überprüft.

e) Klonierung des Vektors pMPSV71TIN

Für die Klonierung dieses Vektors wurde das Rückgrat aus dem Plasmid pSFα71 (SHX) (Arbeitsgruppe Dr. C. Baum, Heinrich-Pette-Institut, Hamburg) benutzt. Der Vektor pSFα71 (SHX) enhält die Enhancer/Promoter-Sequenzen des SFFV (α-Form) in der 3'LTR. Diese SFα Sequenzen wurden durch gleichzeitige Spaltung mit Not I in der Multiplen Klonierungsstelle intern im Vektor und der einzelnen 3' von der 3'LTR gelegenen Xho I Stelle entfernt. Nach Auftrennung der Spaltprodukte durch Agarose-Gelelektrophorese wurde das Plasmidrückgrad mit der 5'LTR und dem leader 71 (SEQ ID NO: 18) isoliert. Aus dem Vektor pMPSV11Neo (siehe b.) wurde das Neo-3'MPSV-LTR-Fragment durch Spaltung mit Not I und Xho I erhalten. Nach Auftrennung der Spaltprodukte durch Agarose-Gelelektrophorese wurde das Neo-3'MPSV-LTR-Fragment isoliert und in das Plasmidrückgrat mit der 5'MPSV-LTR und dem leader 71 ligiert. Der resultierende Vektor ist pMPSV71Neo. pMPSV71Neo wurde mit Not I geöffnet. Durch Spaltung von pM03TIN mit NotI wurde das TK-IRES-Fragment erhalten und anschließend in den geöffneten pMPSV71Neo ligiert. Hieraus resultierte der Vektor pMPSV71TIN (SEQ ID NO: 15).

f) Klonierung des Vektors pMPSV91T*IN

Zunächst wurden durch Spaltung von pMPSV91TIN mit NotI und HindIII die inserierten Fremdsequenzen (TK, IRES, Neo) entfernt. Nach Auftrennung der Spaltprodukte durch Agarosegelelektrophorese wurde das Plasmid-Rückgrat (pMPSV91) isoliert. Das Spleißbereinigte tk Gen wurde als NotI-HindIII-Fragment aus dem Bluescript Plasmid isoliert (siehe oben) und anschließend in das pMPSV91-Rückgrat inseriert. Es resultierte das Plasmid pMPSV91T*. Aus dem Plasmid pMPSV91TIN wurde durch Spaltung mit HindIII das IRES-Neo-Fragment freigesetzt und in das mit HindIII linearisierte Plasmid pMPSV91T* kloniert. Hieraus resultierte der Vektor MPSV91T*IN (SEQ ID NO: 16). Die korrekte Orientierung der IRES-Neo-Sequenzen wurde durch Sequenzanalyse überprüft.

g) Klonierung des Vektors MPSV71T*IN

Zunächst werden durch Spaltung von pMPSV71TIN mit NotI und HindIII die inserierten Fremdsequenzen (TK, IRES, Neo) entfernt. Nach Auftrennung der Spaltprodukte durch Agarosegelelektrophorese wird das Plasmid-Rückgrat (pMPSV71) isoliert. Das Spleißbereinigte tk Gen wird als NotI-HindIII-Fragment aus dem Bluescript Plasmid isoliert (siehe oben) und anschließend in das pMPSV71-Rückgrat inseriert. Es resultiert das Plasmid pMPSV71T*. Aus dem Plasmid pMPSV71TIN wird durch Spaltung mit HindIII das IRES-Neo-Fragment freigesetzt und in das mit HindIII linearisierte Plasmid pMPSV71T* kloniert. Hieraus resultiert der Vektor MPSV71T*IN (SEQ ID NO: 17). Die korrekte Orientierung der IRES-Neo-Sequenzen wird durch Sequenzanalyse überprüft.

Funktionelle Analyse der retroviralen Selbstmord-Genvektoren in primären humanen T-Lymphozyten

Mononukleäre Zellen aus peripherem Blut wurden aus buffy coats (Thrombozytenabgereicherte Blutpräparate) durch Ficollgradienten-Zentrifugation isoliert. Die Zellen wurden dann in cryo-Medium (80% FCS, 10% DMEM, 10% DMSO [Sigma-Aldrich + Co. Ltd., Irvine, Großbritannien]) in einer Konzentration von 3 x $10^7$ Zellen/ml verdünnt und bis zur weiteren Benutzung in Stickstoff (Gasphase) eingefroren. Nach dem Auftauen wurden die Zellen in X-VIVO 10-Medium (Biowhittaker, Walkersville, Maryland, USA) gewaschen. Die T-Lymphozyten wurden in X-VIVO 10-Medium, 10% FCS, 200 U/ml Proleukin-2 (Chiron, Ratingen, Deutschland) und 10 ng/ml OKT3 (Jannsen-Cilag, Neuss, Deutschland) resuspendiert. Die Zellen wurden dann für drei Tage wachstumsstimuliert und die Stimulation durch FACS-Analyse unter Benutzung von CD3-FITC/CD4-PE und CD3-FITC/CD8-PE monoklonalen Antikörpern (Coulter-Immunotech Diagnostics, Krefeld, Deutschland) nachgewiesen. Der Nachweis der Fluoreszenz erfolgte mit einem Coulter EPICS XL-MCL-Zytometer, das mit einem 488 nm Argonlaser, einem 488 nm Sidescatterfilter und einem 525 nm Fluoreszenzbypassfilter ausgerüstet war. Die "Gate"-Kriterien wurden entsprechend den isotypischen Kontrollen angepasst.

Für die Transduktion wurden 4 x $10^5$ Zellen pro Well in 6-Well-Platten ausgesät und in dreifacher Ausführung mit zellfreien Kulturüberständen des jeweiligen PG13-Virusproduzentenklons (Multiplizität der Infektion [MOI] ≤0,15) in Anwesenheit von 16 μg/ml Protaminsulfat (Kraeber, Ellerbek, Deutschland) inkubiert und für 1 Std. bei 1000 g bei Raumtemperatur zentrifugiert. Die Prozedur wurde am nächsten Tag wiederholt. Stabil transduzierte T-Lymphozyten wurden dann für 7 Tage mit 800 μg/ml Geneticin (Gibco BRL Life Technologies Ltd., Paisley, Großbritannien) selektioniert.

Vor dem Start der funktionellen Analyse der Vektorkonstrukte durch negative Selektion wurden die Kulturen für zwei Tage ohne Geneticin angezogen, um das Geneticin-vermittelte Abtöten von der Ganciclovir (GCV)-Sensitivität der transduzierten Zellen unterscheiden zu können. Für die Bestimmung der GCV-Sensitivität wurden die Kulturen halbiert und parallel bis zu 7 Tagen entweder mit oder ohne GCV bei einer Konzentration von 0,1 bis 10 μg/ml angezogen. Lebende Zellen wurden mit Calcein AM (Molecular Probes, Leiden, Niederlande) angefärbt und anschließend durch FACS analysiert. Die Tötungseffizienz wurde folgendermaßen kalkuliert:

$$100 - 100 \text{ x } \frac{lebendeZellen(GCV - selektiert)}{lebendeZellen(nichtselektioniert)} = \text{Tötungseffizienz in Prozent}$$

In vitro-Mutagenese des Thymidinkinase-Gens

Kryptische Spleißdonor- und Spleißakzeptorstellen im Herpes simplex-Virus-Thymidin-kinase-Gen (tk) (Garin et al., 2001) wurden mit Hilfe des QuikChange™ site-directed mutagenesis kit (Stratagene, Cedar Creek, Texas, USA) entsprechend der Herstellerangaben modifiziert. Für die Spleißdonorsite wurden die Primer 5'-CAA CAC CGC CTC GAC CAA GGG GAG ATA TCG GC-3' und 5'-GCC GAT ATC TCC CCT TGG TCG AGG CGG TGT TG-3' und für die Spleißakzeptorstelle die Primer 5'-GCA TGA CCC CCC AAG CCG TGC TGG CGT TC-3' und 5'-GAA CGC CAG CAC GGC TTG GGG GGT CAT GC-3' benutzt.

Die Sequenzen des veränderten tk-Gens und die Zerstörung der kryptischen Spleißmotive wurde durch Sequenzierung bestätigt.

Herstellung der PG13 Producer-Klone

GP+envAM12-Zellen (Markowitz et al., 1988, Virol., 167, 400-406) wurden mit dem jeweiligen Plasmidvektor unter Verwendung des Lipofection-Reagenz FuGENE™ Kit (Roche Diagnostics GmbH, Mannheim, Deutschland) transfiziert. Kulturüberstände, die amphotrope Viruspartikel enthielten, wurden 2 Tage nach der Lipofektion geerntet, durch 0,45 μm-Filter gefiltert und für die Transduktion der PG13 Verpackungszellen verwendet (Miller et al., 1991, J. Virol, 65, 2220-2224). Klone wurden dann für 11 bis 13 Tage mit 500 μg/ml Geneticin (G418) (Gibco BRL Life Technologies Ltd., Paisley, Großbritannien) selektioniert, bis die Koloniegröße auf 3 bis 5 mm Durchmesser angewachsen war. Für jedes Konstrukt wurden dann 5 bis 24 Klone auf 96 Wellplatten transferiert und in Medium ohne G418 angezogen. Nach der Expansion wurden Aliquots der Zellen in 24 Wellplatten transferiert und der Virustiter durch TaqMan-PCR getestet. Klone mit den höchsten Virustitern wurden weiter expandiert und analysiert.

Southern Blot Analyse

Genomische DNA der PG13 Producer-Klone wurde mit Hilfe des QIAamp DNA Blood Maxi Kit (Qiagen GmbH, Hilden, Deutschland) entsprechend den Herstellerangaben isoliert. 12 μg genomische DNA wurde mit den jeweiligen Restriktionsenzymen verdaut und auf 0,8%-igem Agarosegel in Tris-Acetat-Puffer aufgetrennt. Die Restriktionsfragmente wurden dann auf positiv geladene Nylonmembranen übertragen (Boehringer Mannheim GmbH, Mannheim, Deutschland) und anschließend mit Digoxigenin-markierten Sonden hybridisiert. Sonden für tk und $Neo^R$ wurden mit dem PCR DIG Probe Synthesis Kit (Roche Diagnostics GmbH, Mannheim, Deutschland) entsprechend den Herstellerangaben markiert.

TaqMan-PCR

PG13 Producer-Klone wurden in 1 ml DMEM, das mit 10% FCS, 1 mM Natriumpyruvat, 2 mM L-Glutamin und 50 IU/ml Penicillin - 50 μg/ml Streptomycin (Gibco BRL Life Technologies Ltd., Paisley, Großbritannien) versetzt war, bis zur Konfluenz in 24 Wellplatten angezogen. Die Platten wurden für 5 Min. bei 140 g zentrifugiert und die Überstände in 1,5 ml-Gefäße überführt. Virale RNA wurde dann mit Hilfe des QIAamp viral RNA mini kit isoliert und in 60 μl AVE-Puffer eluiert (Qiagen GmbH, Hilden, Deutschland). Virale Titer der Kulturüberstände wurden entsprechend Sanburn und Cornetta (1999, Gene Ther., 6, 1340-1345) durch PCR bestimmt. Dazu wurde der TaqMan One-step RT-PCR mastermix, 5 μl virale RNA als Template in einer 25 μl-Reaktion und das ABI PRISM 7700 Sequence Detection System (Applied Biosystems, Foster City, Kalifornien, USA) benutzt. Virale Titer wurden durch Vergleich mit einer Standardkurve quantifiziert. Der Titer des Standards wurde durch einen GTU-Assay bestimmt (siehe unten).

Bestimmung des Virustiter durch Geneticintransfer-Unit Assay (GTU)

Die Titer von retroviralen Überständen wurden durch einen Geneticin transfer unit assay (GTU) bestimmt. Dazu wurde eine Titration von retroviralen Überständen auf HT1080-Zielzellen in dreifacher Ausführung durch limitierende Verdünnung der zellfreien Überstände in Anwesenheit von 8 μg/ml Polybrene (Sigma-Aldrich Chemie GmbH, Deisenhofen, Deutschland) im Kulturmedium bestimmt. 24 Stunden nach der Transduktion wurden die Zellen 0,5 mg/ml G418 ausgesetzt und die überlebenden Klone mikroskopisch nach 10 bis 12 Tagen unter Selektionsbedingungen ausgezählt.

Ergebnisse

Im Folgenden sind die Ergebnisse für die Experimente angegeben, durch die die Expressionsfähigkeit der erfindungsgemäßen retroviralen Vektoren bestimmt wurde. Die in Fig. 2 dargestellten 23 retroviralen Vektoren wurden entweder wie oben dargestellt (pMPSV11TIN, pMPSV11T*IN, pMPSV71TIN, pMPSV71T*IN, pMPSV91TIN, pMPSV91T*IN) oder ausgehend von diesen und aus dem Stand der Technik bekannten Vektoren (Hildinger et al., 1998, vide supra; Hildinger et al., 1999, vide supra) durch Standardklonierungsverfahren und ihre Selbstmordfunktion in vitro analysiert. Alle Vektoren wiesen zwei Selektionsmarker auf, die sowohl eine positive Selektion ($Neo^R$) und eine negative Selektion (HSV-tk) der transduzierten Zellen erlaubten. Für die Koexpression der zwei Markergene wurden verschiedene Strategien benutzt, die die Verwendung von internen Promotoren, die Einführung einer Spleißakzeptorstelle oder einer IRES des Poliovirus umfassten. Zusätzlich wurden verschiedene LTRs und verschiedene Leaderregionen analysiert. In Fig. 2a sind die Komponenten der verschiedenen Vektoren dargestellt.

Insgesamt 23 Vektoren (Fig. 2b) wurden hergestellt und zur Transfektion der amphotropen Verpackungszelllinie GP+envAM12 verwendet. Zellfreie Kulturüberstände dieser transfizierten Massenkulturen wurden anschließend benutzt, um die Verpackungszelllinie PG13 mit einer MOI <1 zu transfizieren. Dies sollte sicher stellen, dass nur jeweils eine Kopie des retroviralen Vektor in das Genom der PG13 Producer-Zelllinie integrierte. Für jeden Vektor wurden zwischen 5 bis 24 Klone gepickt, was zu einer Gesamtzahl von 312 Klonen führte. Anschließend wurde der Virustiter der Kulturüberstände durch quantitative TaqMan-PCR für alle Klone bestimmt.

Die viralen Titer lagen zwischen $1 \times 10^2$ und $7 \times 10^5$ Partikeln pro ml. Der Durchschnittstiter für alle 312 Klone war ca. $5 \times 10^4$ Partikel pro ml mit keinen entscheidenden Unterschieden zwischen den verschiedenen Vektorkonstrukten. Bis zu 5 Producerklone, die den höchsten Virustiter aufwiesen, wurden pro Vektor (insgesamt 87 Klone) expandiert und im Detail auf der molekularen Ebene untersucht.

Molekulare Charakterisierung der PG13 Producerklone

Um die Vektoren mit der höchsten GCV-Sensitivität zu bestimmen, musste als Voraussetzung sicher gestellt werden, dass nur solche PG13 Producerklone benutzt wurden, die eine einzelne und intakte Kopie des Provirus enthielten. Dadurch kann nämlich sicher gestellt werden, dass die T-Lymphozyten mit einer homogenen, funktionellen Viruspopulation transduziert werden, die es erlaubt, die beobachtete GCV-Sensitivität einem einzelnen viralen Konstrukt zuzuweisen.

Dazu wurde die genomische DNA der 87 PG13 Producerklone, die den höchsten Virustiter aufwiesen, isoliert und mit Hilfe von Southern Blots und PCR analysiert. Um die Anzahl der Kopien des Provirus für jeden Klon zu bestimmen, wurden abhängig vom Vektor intrinsische BamHI- oder XbaI-Restriktionsschnittstellen benutzt und eine Hybridisierung mit einer tk-spezifischen Sonde durchgeführt. Zusätzlich zu den proviralen Kopien detektiert diese Sonde ebenfalls eine Kopie des HSV-tk-Gens, die bereits im Genom der parentalen PG13-Zellen als eine einzelne 11 kb-Bande anwesend ist (Fig. 3, linke Spalte, zeigt einige Beispiele der molekularen Analyse) (Miller et al., 1991, J. Virol., 65, 2220-2224). Die Anzahl der zusätzlichen Banden zeigt die Anzahl der Kopien des Provirus für jeden einzelnen Klon an.

Die Integrität der proviralen Insertion wurde mit Hilfe der Southern-Hybridisierung unter Verwendung von $neo^R$- oder tk-spezifischen Sonden analysiert. Dazu wurde genomische DNA mit XbaI, SacI oder BamHI und XbaI (Integrität des tk-Gens) oder mit XbaI, SacI oder EcoRI und EcoRV (Integrität des $neo^R$-Gens) verdaut, um provirale Restriktionsfragmente mit einer definierten Größe freizusetzen (Fig. 3, rechte Spalte). Zusätzlich wurden Fragmente, die spezifisch für tk, $neo^R$ und die Sequenzen zwischen beiden Genen sind, durch PCR mittels tk- und $neo^R$-spezifischer Primerpaare amplifiziert, um die korrekte Größe der zentralen Module des Vektors zu verifizieren (Daten nicht gezeigt).

Unter den 87 PG13-Producerklonen konnten 62 Klone (71%) identifiziert werden, die unzweifelhaft eine einzelne Kopie des Provirus integriert haben, wohingegen nur ein Klon identifiziert wurde, der zwei Kopien aufweist. Für die anderen 24 Klone konnte entweder keine Kopie detektiert werden (n = 7) oder die Kopienanzahl konnte nicht unzweifelhaft bestimmt werden (n = 17). 36 der 62 Klone (58%) mit einer Einzelkopie-Insertion zeigten keine Änderung im Southern Blot und der PCR-Analyse. Diese Klone wurden hinsichtlich ihrer Vektoren weiter analysiert. Überraschenderweise zeigten 10 Klone (16%) eine Umlagerung der viralen Sequenzen. Für 16 Klone (26%) konnten keine schlüssigen Ergebnisse hinsichtlich der Integrität des Provirus erhalten werden. Dies war zum Teil in der limitierenden Gelauflösung der Southern Blot-Analyse begründet, die einen Nachweis von Änderungen, die auf geringen Größenunterschieden der entsprechenden Restriktionsfragmente beruhen, schwierig machte.

Die Häufigkeit der Änderungen des Vektors ist überraschend hoch, wenn man alle Klone betrachtet. 29 der 87 Klone (33%) haben veränderte Vektorkopien. Diese Häufigkeit der Änderungen hängt zum Teil mit dem Vektortyp zusammen, wie insbesondere die Analyse der Vektoren zeigte, die das erste Intron des EF1$\alpha$-Gens enthalten.

Für das erste Intron des EF1$\alpha$-Gens ist ein verstärkender Effekt für die Promotoraktivität in Transfektionsexperimenten nachgewiesen worden (Kim et al., 1990, Gene, 91, 217-223; Wakabayashi-Ito und Nagata, 1994, J. Biol. Chem., 269, 29831-29837; Kim et al., 2002, J. Biotechnol., 93, 183-187). Alle Klone (n = 12) der retroviralen Vektoren, die die lange Version des Elongationsfaktor 1$\alpha$ (EF1$\alpha$)-Promotors mit diesem Intron für die Expression von tk benutzten, zeigten eine Umlagerung der viralen Sequenzen in der Southern Blot-Analyse. Die Hauptänderung ist eine ca. 1 kb-Deletion, die höchstwahrscheinlich auf ein unerwünschtes Spleißen des viralen Genom während der Virusreplikation in der Verpackungszelllinie zurückzuführen ist. Diese Annahme basiert auf Southern Blot-Ergebnissen, die zeigen, dass das tk-Gen selbst die erwartete Größe hat, wohingegen Restriktionsfragmente, die die neo$^R$-EF1$\alpha$-Promotor/erstes Intron-tk-Kassette enthalten, ca. 1 kb kleiner als erwartet sind (Daten nicht gezeigt). Diese Deletion korreliert gut mit der Größe der Intronsequenz.

Damit zeigen die Ergebnisse der vorliegenden Erfindung die herausragende Bedeutung einer detaillierten molekularen Charakterisierung von Producerklonen, da die Frequenz von Rekombinationsereignissen in hohem Maße von Vektorsequenzen abhängig sein kann. Entsprechend können Aussagen darüber, welche retroviralen Sequenzen und welche Vektorsequenzen im Allgemeinen für eine effiziente Expression der Transgene verantwortlich sind, nur getätigt werden, wenn vorher die Producerklone im Detail charakterisiert worden sind.

Im Rahmen der vorliegenden Erfindung wurden daher nur die PG13 Producerklone mit einer unveränderten Einzelkopieinsertion des Provirus zur Herstellung der Viren und der funktionellen Analysen verwendet.

Verbesserte Selbstmordfunktion von erfindungsgemäßen Vektoren in primären humanen T-Lymphozyten

Die Fähigkeit der verschiedenen Vektorkonstrukte, eine GCV-Sensitivität zu vermitteln, wurde in primären humanen T-Lymphozyten bestimmt, die aus buffy coats isoliert worden waren. Wachstumsstimulierte T-Lymphozyten wurden mit den zellfreien Kulturüberständen der PG 13 Producerklone transduziert und für 7 Tage mit 800 $\mu$g/ml G418 selektioniert. Diese Zeitspanne ist ausreichend, um eine Population an T-Lymphozyten zu erhalten, die zu fast 100% mit den Vektoren transduziert ist (Daten nicht gezeigt). Die 24 Vektoren wurden in Gruppen von 3 bis 7 Vektoren in unabhängigen Transduktionsexperimenten analysiert. Der bekannte Vektor pMO3TIN diente dabei als eine Kontrolle in allen Experimenten, um die Reproduzierbarkeit der Transduktionseffizienz, der G418-Selektion und der GCV-Sensitivität für jeden einzelnen Ansatz von wachstumsstimulierten T-Lymphozyten zu überwachen. Die analysierten viralen Überstände vermittelten Tötungseffizienzen von bis zu 99% nach 3 Tagen als Antwort auf eine einzelne Verabreichung von 10 $\mu$g/ml GCV. Diese Wirkstoffkonzentration hatte keinen nennenswerten Einfluss auf die Viabilität von nichttransduzierten Zellen (Daten nicht gezeigt).

Wegen der modularen Struktur der retroviralen Vektoren kann der Einfluss der einzelnen Module auf die tk-Expression, wie sie durch die GCV-Sensitivität nachgewiesen wird, analysiert werden. Die Experimente der vorliegenden Erfindung zeigten eindeutig, dass die Verwendung von MPSV-LTRs zusammen mit den MESV-Leaderregionen vorteilhaft für die Expression des tk-Gens war. Alle Vektoren, die eine MPSV-LTR und einen MESV-Leader aufwiesen, bewirkten eine effizientere Expression des tk-Gens als die Vektoren, die eine SFFV-LTR aufwiesen. Dagegen konnte keine weitere Verbesserung der Effizienz beobachtet werden, wenn in dem tk-Gen kryptische Spleißmotive zerstört waren.

Die Vektoren, die die höchste GCV-Sensitivität in verschiedenen Transduktionsexperimenten vermittelten, wurden ausgewählt und parallel analysiert, um die effizientesten Konstrukte zu identifizieren. Um die Unterschiede in ihrer Fähigkeit, T-Lymphozyten zu töten, abschätzen zu können, wurde die GCV-Konzentration auf 1 $\mu$g/ml abgesenkt. Unter diesen Bedingungen wurden 75 bis 91% der transduzierten T-Lymphozyten 3 Tage nach der GCV-Selektion getötet (Fig. 4). Zellen, die das Selbstmord-Gen unter der Kontrolle des Internal Elongation Factor 1 $\alpha$ Promoter (NET-Expressionskassette) exprimieren, waren wesentlich weniger sensitiv für GCV als Zellen, die mit Vektorkonstrukten transduziert worden waren, die die TIN-Expressionskassette enthielten, bei der die Expression des tk-Gens durch die LTR gesteuert wird. Tötungseffizienzen von mehr als 90% nach einer einzelnen Verabreichung von GCV konnte für 3 Vektoren unter diesen Bedingungen beobachtet werden. Überraschenderweise benutzen all diese Vektoren die TIN-Expressionskassette in Kombination mit einer MPSV-LTR und einem MESV-Leader. Die Tatsache, dass sich die drei effizientesten Vektoren unter einer Vielzahl von getesteten Vektoren hinsichtlich der Sequenzmodule sehr ähneln, zeigt, dass die Kombination der MPSV-LTR zusammen mit der TIN-Expressionskassette und der MESV-Leadersequenz die beste Kombination für eine effiziente Koexpression von zwei Transgenen ist.

Um die Wirksamkeit dieser optimalen Vektoren im Detail zu analysieren, wurden T-Lymphozyten, die von verschiedenen Blutspendern isoliert worden waren, mit den Vektoren transduziert und nach G418-Selektion reduzierten GCV-Konzentrationen ausgesetzt. Die Konzentrationen, die in diesem Experiment eingesetzt wurden, decken den phys-

iologischen Bereich bis 1 μM (ca. 0,25 μg/ml GCV) ab, der in Patienten während einer GCV-Behandlung erreicht werden kann (Verzeletti et al., 1998, Hum. Gene Ther., 10, 2243-2251). Wie in Fig. 5 gezeigt ist, werden Unterschiede in der Tötungseffizienz der vier getesteten Vektoren unter den geringeren physiologischen GCV- Konzentrationen (0,1 bis 0,25 μg/ml) wesentlich verstärkt im Vergleich zu den höheren GCV-Konzentrationen (1 bis 10 μg/ml), die in den vorherigen Experimenten benutzt wurden. Dabei zeigte sich, dass der pMPSV91TIN der effizienteste Vektor ist, der eine bis zu 95%-ige GCV-Sensitivität nach einer einzelnen Wirkstoffverabreichung (0,25 μg/ml) und 3 Tagen der Selektion vermittelt. Bei gleichen Bedingungen vermittelten die Vektoren pMPSV11T*IN und pMPSV11TIN 75 bis 87%-ige GCV-Sensitivität, während nur 48 bis 54% der Zellen, die mit dem pMO3TIN transduziert worden waren, eliminiert wurden. Damit liegen die erfindungsgemäßen retroviralen Vektoren in ihrer Fähigkeit, Transgene zu exprimieren, deutlich über Vektoren, die aus dem Stand der Technik bekannt sind.

Bemerkenswerterweise war diese Wirkstoffkonzentration bereits ausreichend, um T-Lymphozyten, die mit pMPSV91TIN transduziert worden waren, quantitativ zu depletieren. Ansteigende Konzentration führt nicht zu einem zusätzlichen wesentlichen Anstieg der Tötung von MPSV91TIN-transduzierten Zellen. Im Gegensatz hierzu wurde für weniger effiziente Konstrukte ein Anstieg der Selbstmord-Wirksamkeit beobachtet, wenn GCV-Konzentrationen erhöht wurden. Aber selbst unter den höchsten GCV-Konzentrationen war der pMPSV91TIN-Vektor wesentlich effizienter als alle anderen bekannten Vektoren.

Wenn die Selektion über längere Zeiträume durchgeführt wurde und mehrfach GCV verabreicht wurde, war pMPSV91 TIN ebenfalls wesentlich effizienter als der M03TIN-Prototyp-Vektor (siehe Fig. 6). Mit MPSV91TIN transduzierte Zellen wurden bis zu 98% bei 0,25 μg/ml GCV depletiert, wohingegen M03TIN transduzierte Zellen nur bis zu 90% innerhalb 7 Tagen depletiert werden konnten.

Zusammenfassend zeigen die Experimente der vorliegenden Erfindung, dass erfindungsgemäße retrovirale Vektoren eine Expression des transduzierten Transgens bewirken, die deutlich höher und effizienter ist als für alle anderen bekannten retroviralen Vektoren. Dies war angesichts des Stands der Technik überraschend, da durch die Publikationen von Hildinger et al. (1999, J. Virol. 73, 4083-4089) gezeigt worden war, dass lediglich die Kombination von SFFV-LTRs mit MESV-Leadersequenzen, die einen Spleißakzeptor aufweisen und in denen ein kryptisches AUG mutiert ist, eine effiziente Expression des Transgens bewirken. Ebenso zeigte die Publikation von Hildinger et al. (1999, vide supra), dass im Falle bicistronischer retroviraler Vektoren für die Expression des zweiten Transgens die Verwendung eines Spleißakzeptors gegenüber einer IRES und einem internen Promotor vorteilhaft ist.

Ohne an eine wissenschaftliche Theorie gebunden sein zu wollen, wird vermutet, dass die spezifische Kombination von MPSV-LTR-Sequenzen, MESV-Leadersequenzen und die Verwendung einer IRES zur Steuerung der Expression des zweiten Transgens eine effiziente Expression beider Transgene in transduzierten Zellen erlaubt, weil die IRES eine im wesentlichen separate Steuerung des ersten und zweiten Transgens ermöglicht. Dieser Befund wurde durch die Durchführung einer neuen, detaillierten und gründlichen molekularen Analyse der Producerklone ermöglicht, die erstmals einen direkten Vergleich der Effekte der einzelnen regulatorischen Sequenzen von retroviralen Vektoren erlaubte.

Auf diese Weise konnten neue erfindungsgemäße retrovirale Vektoren identifiziert werden, die eine 97%-ige GCV-Selektierbarkeit innerhalb von 3 Tagen für primäre T-Zellen bei Konzentrationen von 1,0 μg/ml GCV gewährleisten. Die erfindungsgemäßen retroviralen Vektoren sind damit in funktionellen Tests effizienter als die bekannten Vektoren, die die SFFV-LTR verwenden oder der bekannte Vektor MO3TIN, die bereits in klinischen Phase 1/2-Studien getestet werden.

Durch die vorliegenden Experimente wird auch gezeigt, dass die Funktionalität der retroviralen Vektoren gegeben ist, wenn der Retrovirus als einzelne Kopie in das Genom der Zielzelle integriert ist, wie es durch Infektionen mit geringen Virusdosen, typischerweise mit einer MOI $\leq$0,25 erreicht werden kann. Solche Infektionen mit geringen MOIs haben mehrere Vorteile. Im Vergleich zu Virusinfektionen mit höheren MOIs, bei denen mehrere Kopien des Virus in das Genom der Zielzelle integrieren, wird das Risiko reduziert, dass durch die Integration des Virus in das Genom der Zielzelle Gene des Wirts zerstört werden bzw. die Expression der zellulären Gene beeinflußt wird (so genannte Insertional Mutagenesis). Da das Auftreten von retroviralen Sequenzen per se immer zu vermeiden ist, sind Infektionen mit geringen MOIs auch aus Sicherheitsaspekten zu bevorzugen. Durch Infektionen mit geringen MOIs und Vektoren, die nur geringe Anteile an retroviralen Sequenzen enthalten, aber eine effiziente Expression gewährleisten, wird das Risiko einer Immunabwehr, die für den Körper des Patienten eine erhebliche Belastung darstellt, erheblich abgeschwächt.

Die Tatsache, dass die erfindungsgemäßen Vektoren, die die höchste Expression im Vergleich zu bekannten retroviralen Vektoren zeigen, alle die MPSV-LTR enthalten, war zudem überraschend, da durch Publikationen gezeigt worden war (Plavec et al., 1997, Gene Ther., 4, 128-139), dass MPSV-Vektoren in humanen lympho-hämatopoetischen Zellen im Vergleich zu anderen LTRs nicht zu einer effizienten Expression des Transgens führen.

Außerdem zeigten die Experimente der vorliegenden Erfindung, dass nur solche erfindungsgemäßen retroviralen Vektoren eine verbesserte Expression gewährleisten, bei denen die Expression des zweiten Transgen durch eine IRES-Sequenz gesteuert wird. Die Verwendung von zusätzlichen Spleißsignalen, wie sie durch Hildinger et al. (1999) vorgeschlagen wurde, oder die Verwendung von internen Promotoren für die Expression des zweiten Transgens war dagegen überraschenderweise weniger effizient.

Im Falle von so genannten dualen Spleißvektoren wird die Translation des zweiten Transgens (neo$^R$) durch das Vorhandensein eines zusätzlichen Spleißakzeptors gewährleistet, der sich unmittelbar 5' vor der für neo$^R$-kodierenden Sequenz befindet. Obwohl in den Experimenten der vorliegenden Erfindung degenerierte Spleißakzeptorsignale verwendet wurden (Hildinger et al., 1999, vide supra), scheint das Spleißereignis die Expression des ersten Transgens (tk) zu reduzieren, was eine reduzierte Selektionierbarkeit der transduzierten Zellen bewirkt.

Zusätzlich können die zusätzlichen Spleißsignale das Risiko von unerwünschten Spleißereignissen erhöhen.

Darüber hinaus bestimmt die retrovirale MESV-Leadersequenz in erheblicher Weise die Effizienz der erfindungsgemäßen retroviralen Vektoren. Dabei spielt es keine Rolle, ob die erfindungsgemäßen retroviralen Vektoren am 3'-Ende eine Spleißakzeptorsite enthalten oder ob ein nicht-kodierendes AUG-Kodon mutiert worden ist. Auch diese Ergebnisse waren angesichts der Publikation von Hildinger et al. (1999, vide supra) überraschend.

Die Identifizierung der erfindungsgemäßen retroviralen Vektoren mit ihren vorteilhaften Eigenschaften war nur möglich, da im Rahmen der vorliegenden Erfindung die Producerklone, die für die Virusherstellung verwendet werden, intensiv charakterisiert wurden. Die Produktion von viralen Überständen von schlecht charakterisierten Producerklonen oder von Massenkulturen scheint zu einer Mischung von intakten und veränderten Viruspartikeln zu führen, was die Interpretation von Ergebnissen hinsichtlich des Einflusses von Modulen der retroviralen Vektoren auf die Expression schwierig macht.

Zum Beispiel würde die Transduktion von primären T-Lymphozyten mit solchen nichtcharakterisierten Viruspopulationen in den funktionellen Tests, die im Rahmen der vorliegenden Erfindung verwendet wurden, es unmöglich machen, die beobachtete GCV-Sensitivität auf ein einzelnes virales Konstrukt zurückzuführen. Dies wird daran deutlich, dass 33% der Producerklone, die im Rahmen der vorliegenden Erfindung hergestellt wurden, ein verändertes Vektorgenom enthielten. Dieses Ergebnis zeigt deutlich, dass eine sorgfältige Qualitätskontrolle durchgeführt werden muss, wenn die durch einen retroviralen Vektor hervorgerufenen Expressionsereignisse charakterisiert werden sollen und insbesondere wenn solche retroviralen Vektoren für klinische Zwecke eingesetzt werden sollen. Angesichts der Ergebnisse der vorliegenden Erfindung wird deutlich, dass Gentransfermethoden, die auf der Infektion mit Überständen von Massenkulturen (Rudoll et al., 1996, Gene Therapy, 3, 695-705) oder der Transduktion durch Kokultivierung mit Verpackungszelllinien (Mavilio et al., 1994, Blood, 83, 1988-1997; Bonini et al., 1997, Science, 276, 1719-1724) beruhen, für klinische Zwecke nicht angebracht sind.

Im Rahmen der vorliegenden Erfindung wurden Gene (HSV-Thymidinkinase und Neomycinphosphotransferase) verwendet, die in der Literatur bereits eingesetzt worden sind, um die GvHD zu kontrollieren und für die nachgewiesen worden ist, dass sie in der Gentherapie oder in Genmarkierungsexperimenten erfolgreich eingesetzt werden können. Zum Beispiel wurde der neo-Marker (neo$^R$) eingesetzt, um nicht-transduzierte Zellen vor der Infusion der Zellen in den Patienten zu eliminieren. Dem Fachmann ist klar, dass auch andere Gene, wie zum Beispiel das MDR1-Gen (Guild et al., 1988, Proc. Natl. Acad. Sci., USA, 85, 1595-1599; Sorrentino et al., 1992, Science, 257, 99-103, und Eckert et al., 1996, Blood, 88, 3407-3415), ein mutiertes Dihydrofolat-Reduktasegen (DHFR) (Miller et al., 1985, Mol. Cell. Biol., 5, 431-437) und Gene für Zelloberflächenmarker wie zum Beispiel CD24 (Pawliuk et al., 1994, Blood, 84, 2868-2877), die trunkierte Form des humanen "nerve growth factor receptor" (ΔLNGFR) (Mavilio et al., 1994, Blood, 83, 19881997; Rudoll et al., 1996, Gene Ther., 3, 695-705; Fehse et al., 1997, Hum. Gene Ther., 8, 1815-1824) und das murine hitzestabile Antigen (HSA) (Conneally et al., 1996, Blood, 87, 456-464) verwendet werden können.

<u>Figuren</u>

Fig. 1
Schematischer Aufbau der proviralen Sequenzen

Fig. 2

(A) Überblick über die Module, die für die Herstellung der erfindungsgemäßen retroviralen Vektoren benutzt wurden. Die verschiedenen LTRs, Leadersequenzen und Expressionskassetten sind aufgeführt.
(B) Zusammenfassung der analysierten Vektoren.

Folgende Abkürzungen wurden benutzt:

MoMuLV: Moloney Murine Leukemia Virus
MPSV: Myeloproliferative Sarkoma Virus
SFß: Spleen Focus Forming Virus
11, 71, 91: verschiedene Leadersequenzen (Hildinger et al., 1999)
T: Herpes Simplex Virus-Thymidinkinasegen
T*: Herpes Simplex Virus-Thymidinkinasegen mit zerstörten kryptischen Spleißmotiven

I: Internal Ribosomal Entry Site des Polio-Virus
N: Neomycinphosphotransferasegen
SA: Spleißakzeptor
E: Promotor des Elongationsfaktor 1α-Gens
EL: Promotor des Elongationsfaktor 1α-Gens mit dem ersten Intron

Fig. 3

Bestimmung der Kopienanzahl und der Integrität der proviralen Insertionen in den PG 13 Producer-Klonen durch Southern Blot-Analyse. Die Mehrzahl der Klone zeigen Einzelintegrationen der Vektoren (linker Teil der Fig.). Die Kopienanzahl jedes Producer-Klons wurde durch DNA-Verdau mit BamHI und Hybridisierung mit einer tk-spezifischen Sonde bestimmt.
Spur 1: Mo3TIN#b5, Spur 2: MPSV11TIN#a1, Spur 3: MPSV71TIN#a3, Spur 4:
MPSV91TIN#b6, Spur 5: MPSV91TSAN#a3, Spur 6: MPSV71TSAN#c5, Spur 7:
MPSV91NET#a1, c = Negativkontrolle: parentale PG13-Zellen.

Die korrekte Größe der integrierten proviralen Kopie wurde durch Vergleich mit dem entsprechenden Vektorplasmid getestet (rechter Teil der Fig.). Die Größe der Vektorfragmente wurde in diesem Fall durch DNA-Verdau mit Xbal und Hybridisierung mit einer tk-spezifischen Sonde bestimmt.
Spur 8: Mo3TIN#b5, Spur 9: Mo3TIN#a1, Spur 10: MPSV11TIN#a1, Spur 11:
MPSV11TIN#a6, Spur 12: MPSV71TIN#a3, Spur 13: MPSV71TIN#b5, Spur 14:
MPSV91TIN#b6, Spur 15: MPSV91TIN#c7, c = Negativkontrolle: parenterale PG13-Zellen, V1 = Vektorplasmid für Mo3TIN, V2 = Vektorplasmid für MPSV11TIN.

Fig. 4

Bewertung von prä-selektionierten Selbstmordgenvektoren, die primären humanen T-Zellen eine hohe Ganciclovir (GCV)-Selektivität verleihen. Die Selektion wurde durch Verabreichung einer einzelnen Dosis von 1 µg GCV/ml für 3 Tage durchgeführt. 6 der neu hergestellten Vektoren mit einer hohen GCV-Selektivität wurden mit dem Mo3TIN-Vektor verglichen, der momentan in klinischen Tests getestet wird.

Fig. 5

3 der neu hergestellten Vektoren erlauben eine effiziente T-Zelldepletion sogar bei geringen Ganciclovir-Konzentrationen (0,1 - 1,0 µg/ml). Alle Vektoren enthalten eine MPSV-LTR, eine MESV-Leaderregion und die Thymidinkinase-IRES-Neo$^R$ (TIN)-Expressionskassette.

Das Ausmaß der Depletion ist unabhängig von den Donorzellen, die für die Experimente verwendet wurden.

Fig. 6

Fast 100% der T-Zellen können durch die Verwendung des MPSV91TIN-Vektors depletiert werden.

## Patentansprüche

**1.** Retroviraler Vektor der allgemeinen Formel: 5'- Ende - [5'- LTR] - [Leader] - [Ex1] - [IRES] - [Ex2] - [3'- LTR] - 3'- Ende **dadurch gekennzeichnet, dass** die 5'-LTR die Nukleinsäuresequenz für eine beliebige LTR eines Retrovirus enthält,

- die Leaderregion die Nukleinsäuresequenz für die Leaderregion von MESV ohne für virale Proteine oder Teile davon kodierende Sequenzen enthält,
- Ex 1 eine Nukleinsäuresequenz, die für ein erstes Transgen kodiert, enthält,
- IRES die Nukleinsäuresequenz für eine Internal Ribosomal Entry Site enthält,
- Ex2 eine Nukleinsäuresequenz, die für ein zweites Transgen kodiert, enthält,
- die 3'-LTR die Nukleinsäuresequenz für eine 3'-LTR mit der U3-Region der LTR des MPSV enhält,

wobei die Leaderregion ausgewählt ist aus den in SEQ ID No. 5 und SEQ ID No. 6 angegebenen Leadersequenzen, und
wobei Ex1 die Nukleinsäuresequenz für Herpes Simplex Virus I Thymidin-Kinase und Ex2 die Nukleinsäuresequenz für das $Neo^R$-Resistenzgen enthält.

**2.** Vektor nach Anspruch 1,
**dadurch gekennzeichnet, dass** er keine für das gag-Protein, das pro-Protein, das env-Protein, das pol-Protein oder andere retrovirale Proteine, wie sie in den Retroviren, aus denen sich der Vektor ableitet, vorkommen, kodierenden Sequenzen oder Sequenzabschnitte enthält.

**3.** Vektor nach einem der Ansprüche 1 und 2,
**dadurch gekennzeichnet, dass** es sich bei der IRES bevorzugt um die IRES des Polio-Virus, des FMDV (Foot and Mouth Disease Virus) oder des EMCV (Encephalomyocarditis Virus) handelt.

**4.** Vektor nach Anspruch 1, wobei es sich um einen Vektor gemäß SEQ ID NO. 1, 2, 3 oder 16 handelt.

**5.** Infektiöses Viruspartikel,
**dadurch gekennzeichnet, dass** das Partikel den retroviralen Vektor nach einem der Ansprüche 1 bis 4 enthält.

**6.** Wirtszelle,
**dadurch gekennzeichnet, dass** sie mit dem retroviralen Vektor nach einem der Ansprüche 1 bis 4 transfiziert oder transduziert ist.

**7.** Wirtszelle nach Anspruch 6,
**dadurch gekennzeichnet, dass** es sich um hämatopoetische Stammzellen oder daraus resultierende Zelltypen, bevorzugt um T-Zellen, B-Zellen, myeloide Zellen, erythroide Zellen, oder Zelllinien, vorzugsweise Jurkat Zellen, CEM Zellen oder K562 Zellen, handelt.

**8.** Verfahren zur Gewinnung eines infektiösen Viruspartikels,
**dadurch gekennzeichnet, dass** man eine verpackungskompetente Helferzelllinie mit einem retroviralen Vektor nach einem der Ansprüche 1 bis 4 transfiziert oder transduziert und die Zelllinie in einem geeigneten Medium unter Bedingungen kultiviert, die für die Abgabe infektiöser Viruspartikel, die als Genom einen retroviralen Vektor nach den Ansprüchen 1 bis 4 enthalten, geeignet sind.

**9.** Verfahren nach Anspruch 8,
**dadurch gekennzeichnet, dass** es sich bei der Helferzelllinie um PG13, PG53, GP+envAM12, FLY, PA12, PA317, Psi2 oder 293 GPG handelt.

**10.** Producer-Zelllinie,
**dadurch gekennzeichnet, dass** sie nach einem Verfahren nach einem der Ansprüche 8 oder 9 hergestellt wird, wobei die Nukleinsäuresequenz eines retroviralen Vektors nach einem der Ansprüche 1 bis 4 in das Genom der Helferzelllinie integriert ist.

**11.** Verwendung des retroviralen Vektors nach einem der Ansprüche 1 bis 4 und/oder des Viruspartikels nach Anspruch 5 zur Herstellung eines pharmazeutischen Präparats zur Kontrolle der GvHD bei der adoptiven Gentherapie.

**12.** Verwendung nach Anspruch 11, wobei hämatopoetische Zellen mit dem retroviralen Vektor transduziert werden.

**13.** Verwendung nach den Ansprüchen 11 oder 12, wobei T-Zellen zur Kontrolle der Graft-versus-Host-Erkrankung bei der adoptiven Immuntherapie transduziert werden.

**14.** Pharmazeutisches Präparat,
**dadurch gekennzeichnet, dass** es einen Vektor nach den Ansprüchen 1 bis 4 zusammen mit pharmazeutisch verträglichen Hilfs- und Trägerstoffen enthält, die die Transfektion von Zielzellen erlauben.

## Claims

**1.** A retroviral vector having the general formula: 5' - end - [5'-LTR] - [Leader] - [Ex1] - [IRES] - [Ex2] - [3'-LTR] - 3' - end
**characterized in that**

- the 5'-LTR comprises the nucleic acid sequence of any LTR of a retrovirus,
- the leader region comprises the nucleic acid sequence of the leader region of MESV without the sequences encoding for viral proteins or parts thereof,
- Ex1 comprises a nucleic acid sequence encoding for a first transgene,
- IRES comprises the nucleic acid sequence for an internal ribosomal entry site,
- Ex2 comprises a nucleic acid sequence encoding for a second transgene,
- the 3'-LTR comprises the nucleic acid sequence of a 3'-LTR comprising the U3 region of the MPSV LTR,

wherein the leader region is selected from the leader sequences specified in SEQ ID No. 5 and SEQ ID No. 6, and wherein Ex1 comprises the nucleic acid sequence for Herpes Simplex Virus I thymidine-kinase and Ex2 comprises the nucleic acid sequence for the $Neo^R$-resistence gene.

**2.** The vector of claim 1,
**characterized in that** it does not comprise any sequences or sequence fragments encoding for the gag-protein, the pro-protein, the env-protein, the pol-protein or for other retroviral proteins being present in those retroviruses from which the vector is derived.

**3.** The vector according to claims 1 and 2,
**characterized in that** the IRES is preferably the IRES of the polio virus, FMDV (foot and mouth disease virus) or EMCV (encephalomyocarditis virus).

**4.** The vector according to claim 1, wherein the vector is a vector according to SEQ ID No. 1, 2, 3 or 16.

**5.** An infectious virus particle,
**characterized in that** the particle comprises the retroviral vector according to any of claims 1 to 4.

**6.** A host cell,
**characterized in that** it is transfected or transduced with the retroviral vector according to any of claims 1 to 4.

**7.** The host cell according to claim 6,
**characterized in that** it is a hematopoietic stem cell or cell type resulting therefrom preferably T-cells, B-cells, myeloid cells, erythroid cells, or cell lines, preferably Jurkat cells, CEM cells or K562 cells.

**8.** A method for producing an infectious virus particle,
**characterized in that** a packaging-competent helper cell line is transfected or transduced with a retroviral vector according to any of claims 1 to 4, and the cell line is cultivated in a suitable medium under conditions appropriate to release infectious virus particles having as genome a retroviral vector according to claims 1 to 4.

**9.** The method according to claim 8,
**characterized in that** the helper cell line is PG13, PG53, GP+envAM12, FLY, PA12, PA317, Psi2 or 293 GPG.

**10.** A producer cell line,
**characterized in that** it is produced according to any of claims 8 or 9, wherein the nucleic acid sequence of a retroviral vector according to any of claims 1 to 4 is integrated into the genome of the helper cell line.

**11.** Use of the retroviral vector according to any of claims 1 to 4 and/or the virus particles according to claim 5 for the manufacture of a pharmaceutical preparation to control GvHD in adoptive gene therapy.

**12.** The use of claim 11, wherein hematopoietic cells are transduced with the retroviral vector.

**13.** The use according to claims 11 or 12, wherein T-cells for controlling graft-versus-host disease are transduced in adoptive immunotherapy.

**14.** A pharmaceutical preparation,
**characterized in that** it comprises a vector according to claims 1 to 4 together with pharmaceutically acceptable adjuvants and excipients which permit the transfection of target cells.

## Revendications

**1.** Vecteur rétroviral de la formule générale suivante :

terminus 5'-[LTR 5']-[leader]-[Ex1]-[IRES]-[Ex2]-[LTR 3']-terminus 3'

**caractérisé par le fait que** :

- la LTR 5' contient la séquence d'acid nucléique pour une quelquonque LTR d'un rétrovirus ;
- la séquence de tête contient la séquence d'acide nucléique pour la séquence de tête de MESV exempte des séquences codant pour des protéines virales ou des parties de ceux-ci
- Ex 1 contient une séquence d'acide nucléique codant pour un premier transgène ;
- IRES contient la séquence d'acide nucléique pour un Site Entry Ribosomal Internal ;
- Ex 2 contient une séquence d'acide nucléique codant pour un deuxième transgène ;
- la LTR 3' contient la séquence d'acide nucléique pour une LTR 3' ayant la région U3 de la LTR du MPSV, la séquence de tête étant sélectionnée parmi les séquences de tête données dans la SEQ ID No : 5 et SEQ ID No : 6, et Ex 1 contenant la séquence d'acide nucléique pour la Thymidin Kinase de l'Herpes Simplex Virus I et Ex 2 contenant la séquence d'acide nucléique pour le gène de résistance Neo®.

**2.** Vecteur selon la revendication 1,
**caractérisé par le fait qu'**il ne contient pas de séquences ou sections de séquence codant pour la protéine gag, la protéine pro, la protéine env, la protéine pol ou d'autres protéines rétrovirales, telles qu'elles existent dans les rétrovirus dont le vecteur est dérivé.

**3.** Vecteur selon les revendications 1 et 2,
**caractérisé par le fait que** l'IRES est de préférence l'IRES du virus polio du FMDV (Foot and Mouth Disease Virus) ou de l'EMCV (Encephalomyocarditis Virus).

4. Vecteur selon la revendication 1, le vecteur étant un vecteur selon SEQ ID No : 1, 2, 3 ou 16.

5. Particule virale infectieuse,
**caractérisé par le fait que** la particule contient le virus rétroviral selon l'une des revendications 1 a 4.

6. Cellule hôte,
**caractérisé par le fait qu'**elle a subie une transfection ou transduction avec le vecteur rétroviral.

7. Cellule hôte selon la revendication 6,
**caractérisé par le fait qu'**il s'agit des cellules souches ou des types de cellule en résultant, de préférence les cellules T, les cellules B, les cellules myéloïdes, les cellules erythroïdes ou les lignes de cellule, préférentiellement les cellules Jurkat, les cellules CEM ou les cellules K562.

8. Procédé pour la production d'une particule infectieuse
**caractérisé par le fait que** l'on réalise une transfection ou transduction avec une ligne de cellule helper compétente en encapsidation avec un vecteur rétroviral selon l'une des revendications 1 à 4 et une cultivation de la ligne de cellule dans un milieu approprié sur des conditions aptes a la libération des particules infectieuses qui contiennent un vecteur rétroviral selon l'une des revendications 1 a 4 a titre de génome.

9. Procédé selon la revendication 8,
**caractérisé par le fait que** la ligne de cellule helper est PG13, PG53, GP+envAM12, FLY, PA12, PA317, Psi2 ou 293 GPG.

10. Ligne de cellule producer,
**caractérisé par le fait qu'**elle est obtenue par un procédé selon l'une des revendications 8 ou 9 dans lequel la séquence d'acide nucléique d'un vecteur rétroviral selon l'une des revendications 1 à 4 est insérée dans le génome du linge de cellule helper.

11. Utilisation du vecteur rétroviral selon l'une des revendications 1 à 4 et/or de la particule virale selon la revendication 5 pour la production d'une préparation pharmaceutique pour le contrôle de la maladie GvHD dans la thérapie génétique adoptive.

12. Utilisation selon la revendication 11, dans laquelle les cellules hématopoïétiques sont subies à une transduction avec le vecteur rétroviral.

13. Utilisation selon les revendications 11 ou 12, dans laquelle des cellules T sont subies d'une transduction pour le contrôle de la maladie graft-versus-host dans l'immunothérapie adoptive.

14. Préparation pharmaceutique
**caractérisé par le fait qu'**elle contient un vecteur selon les revendications 1 à 4 en association avec des additifs et des véhicules pharmaceutiquement compatibles permettant la transfection de cellules cible.

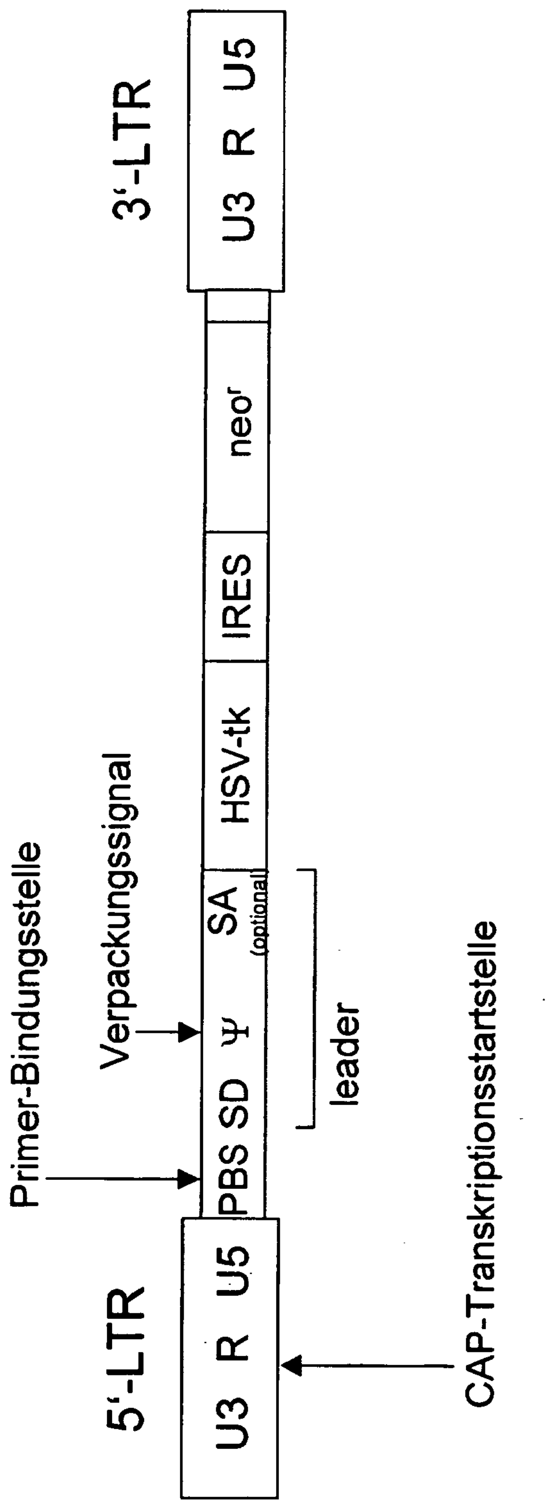
5'-LTR
U3 R U5
3'-LTR
U3 R U5
Primer-Bindungsstelle
Verpackungssignal
PBS SD Ψ SA
(optional)
HSV-tk
IRES
neo<sup>r</sup>
leader
CAP-Transkriptionsstartstelle

Fig. 1

A

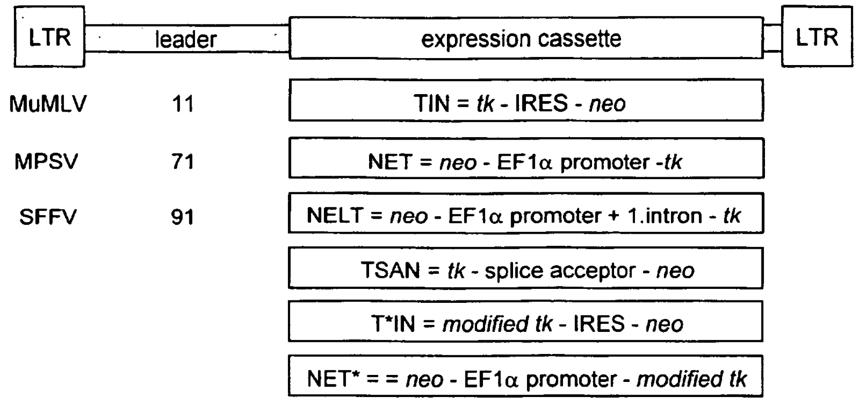


B

| co-expression strategy | name of vector | co-expression strategy | name of vector |
|---|---|---|---|
| **internal ribosomal entry site** | pMPSV11TIN | **internal promoter** | pMPSV11NET |
| | pMPSV71TIN | | pMPSV71NET |
| | pMPSV91TIN | | pMPSV91NET |
| | pSFβ11TIN | | pSFβ11NET |
| | pSFβ91TIN | | pSFβ91NET |
| | pMo11TIN | | pMo11NET |
| | pMPSV11T*IN | | pMo91NET |
| | pMPSV71T*IN | | pMPSV11NET* |
| | pMPSV91T*IN | | pMPSV91NET* |
| | | | pMPSV11NELT |
| **internal splice acceptor site** | pMPSV71TSAN | | pMPSV71NELT |
| | pMPSV91TSAN | | pMPSV91NELT |

Fig. 2

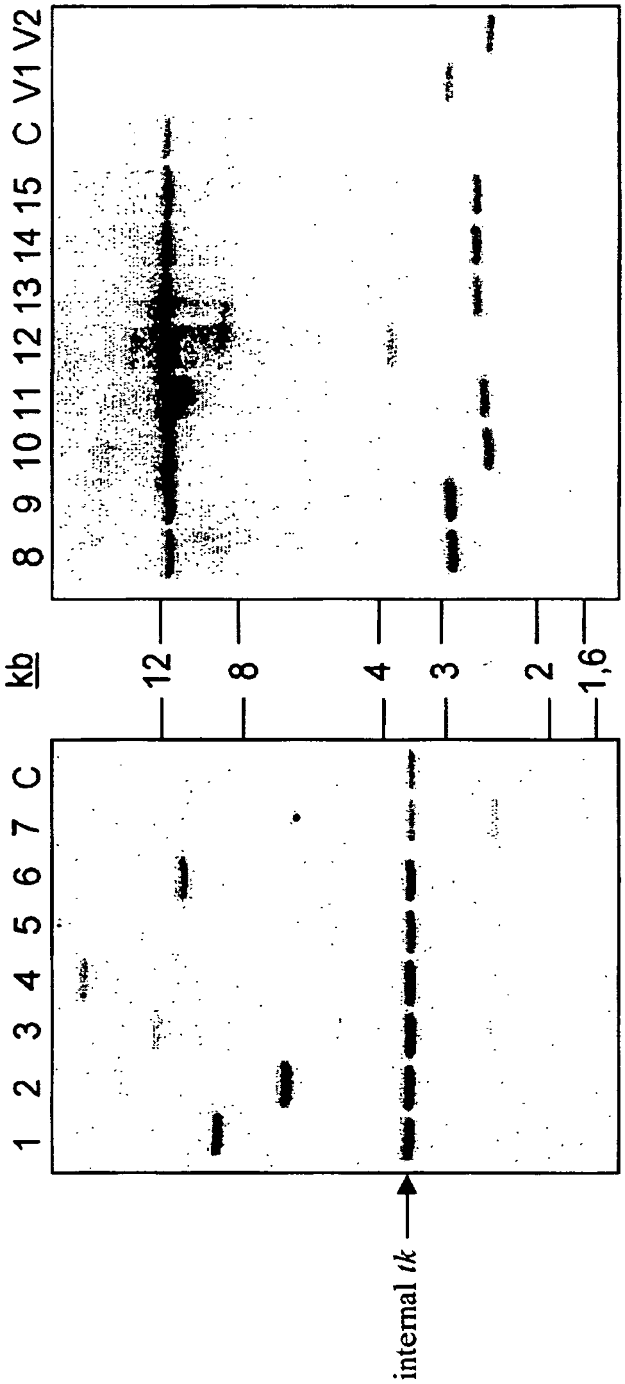


Fig. 3

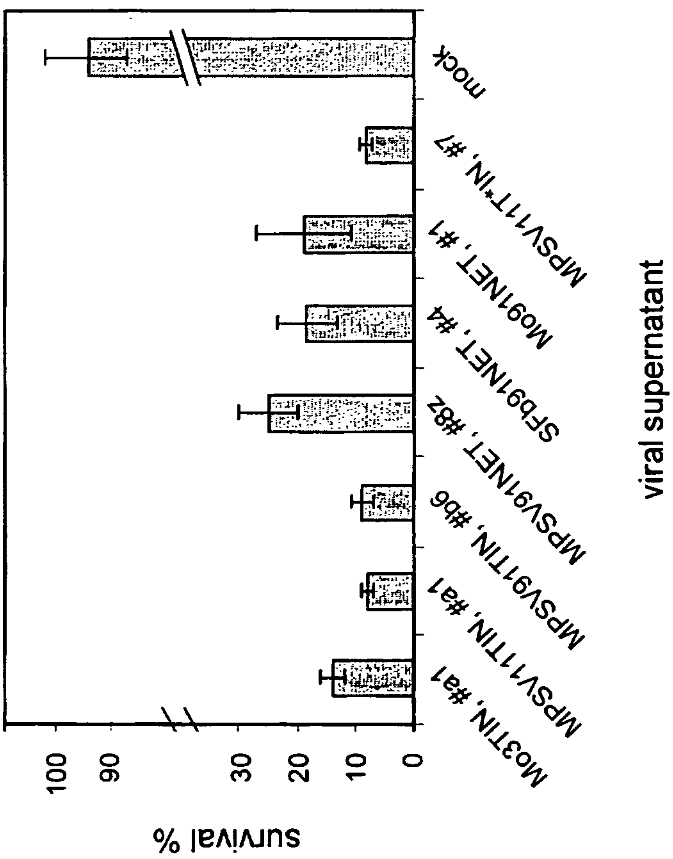
survival %
100
90
30
20
10
0
Mo3TIN, #a1
MPSV11TIN, #a1
MPSV91TIN, #b6
MPSV91NET, #82
SFb91NET, #4
Mo91NET, #1
MPSV11T*IN, #7
mock
viral supernatant

Fig. 4

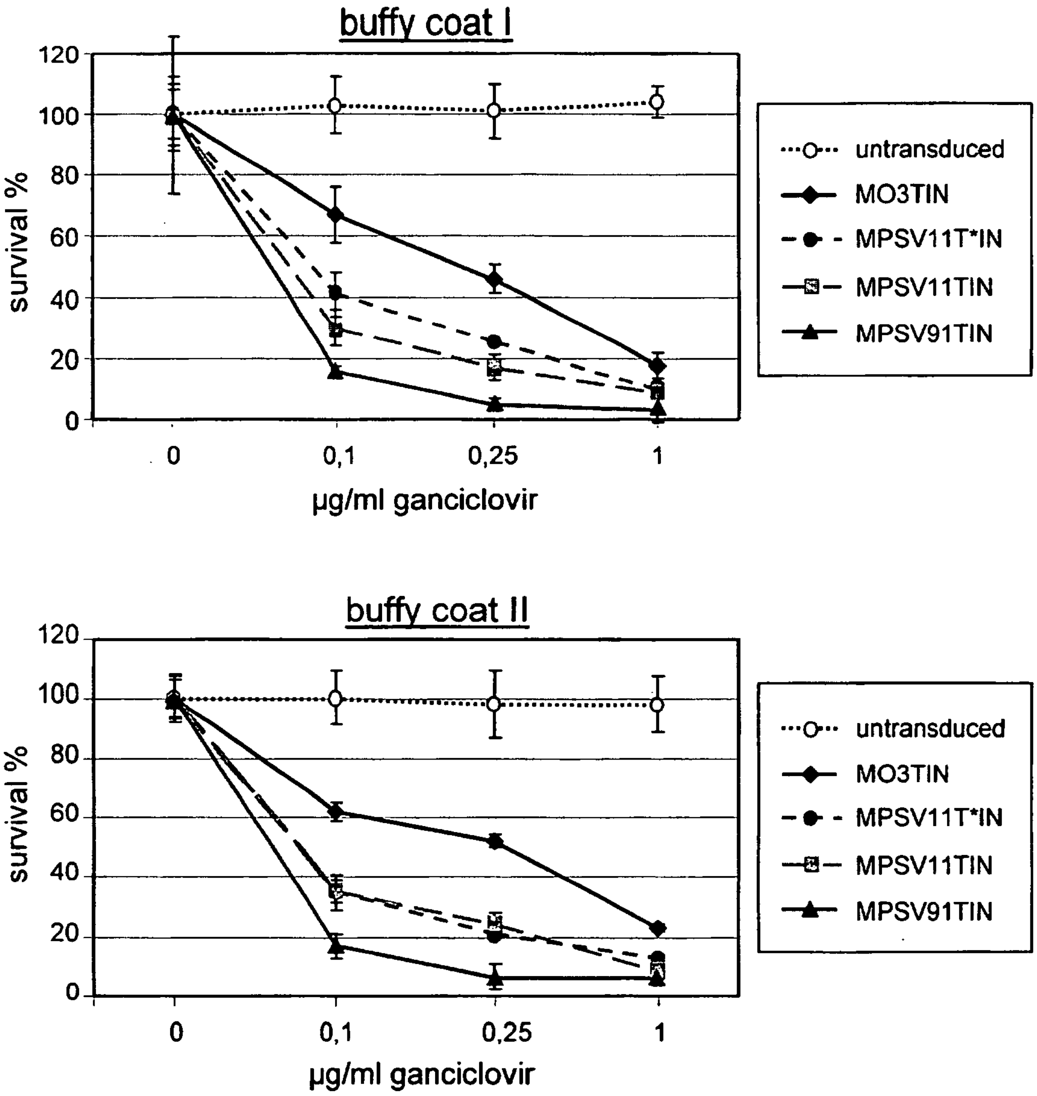
buffy coat I
120
100
80
60
40
20
0
survival %
0
0,1
0,25
1
µg/ml ganciclovir
untransduced
MO3TIN
MPSV11T*IN
MPSV11TIN
MPSV91TIN
buffy coat II
120
100
80
60
40
20
0
survival %
0
0,1
0,25
1
µg/ml ganciclovir
untransduced
MO3TIN
MPSV11T*IN
MPSV11TIN
MPSV91TIN

**Fig. 5**

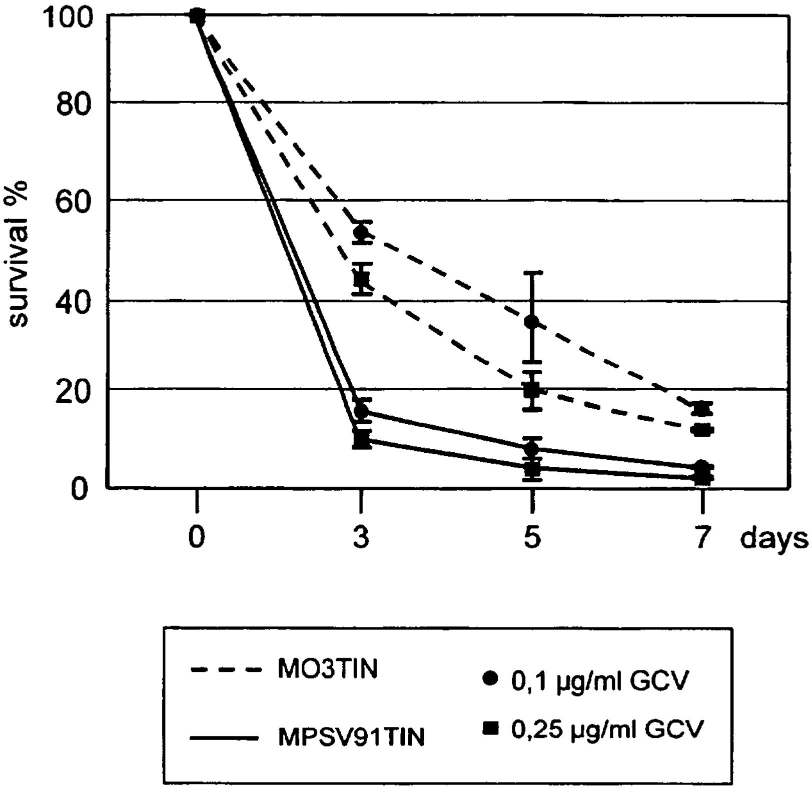

100
80
60
40
20
0
survival %
0
3
5
7
days
MO3TIN
MPSV91TIN
0,1 µg/ml GCV
0,25 µg/ml GCV


Fig. 6

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **Miller, A.D.** *Methods Enzymol.,* 1993, vol. 217, 581-599
- **Zhuang, Y.A. et al.** *Proc. Natl. Acad. Sci. USA,* vol. 86, 2752-2756
- **Krall, W.J. et al.** *Gene Ther.,* 1996, vol. 3, 37-48
- **Baum, C. et al.** *J. Virol.,* 1995, vol. 69, 7541-7547
- **Anderson, W.F.** *Hum. Gene Ther.,* 1993, vol. 4, 311-321
- **Münck, C.** *Proc. Natl. Acad. Sci., USA,* 1997, vol. 94, 5837-5842
- **Dunbar, C.G.** *Ann. Rev. Med.,* 1996, vol. 47, 11-20
- **Baum, C. et al.** *Gene Ther.,* 1996, vol. 3, 1-3
- **Bonini, C. et al.** *Science,* 1997, vol. 276, 1719-1724
- **Bordignon, C. et al.** *Science,* 1995, vol. 270, 470-475
- **Tiberghien, P. et al.** *Hum. Gene Ther.,* 1997, vol. 8, 615-624
- **Hildinger et al.** *Gene Ther.,* 1998, vol. 5, 1575-1579
- **von Hildinger et al.** *Gene Ther.,* 1998, vol. 5, 1575-1579
- **Hildinger et al.** *J. Virol.,* 1999, vol. 73, 4083-4089
- **Hildinger et al.** *Gene Ther.,* 1999, vol. 6, 1222-1230
- **Fehse B. et al.** *Gene Therapy,* 2002, vol. 9, 1633-1638
- **Mclfor, R.S.** *Virology,* 1990, vol. 176, 652-655
- **Johnson, J.J. et al.** *Hum. Gene Ther.,* 1995, vol. 6, 611-623
- **Ausubel, I. et al.** Current Protocols in Molecular Biology. John Wiley und Sons, 1994
- **Sambrook et al.** Molecular Cloning: A Laboratory Manual. Coldspring Harbour Laboratory Press, 2001
- **Blaese et al.** *Science,* 1995, vol. 270, 475-480
- **Losordo et al.** *Circulation,* 1998, vol. 98, 2800-2804
- **Martin et al.** *Blood,* 1985, vol. 66, 664-672
- **Horowitz et al.** *Blood,* 1990, vol. 75, 555-562
- **Brown et al.** *J. Clin. Oncol.,* 1999, vol. 17, 806-812
- **Goker et al.** *Exp. Hematol.,* 2001, vol. 29, 259-277
- **Slavin et al.** *Blood,* vol. 91, 756-763
- **Kottaridis et al.** *Blood,* 2000, vol. 96, 2419-2425
- **Schmaltz et al.** *Transplant,* 2001, vol. 97, 2886-2895
- **Bonini et al.** *Science,* 1997, vol. 276, 1719-1724
- **Tiberghien et al.** *Hum. Gene Ther.,* 1997, vol. 8, 615-624
- **Miller et al.** *J. Virol.,* 1991, vol. 65, 2220-2224
- **Markowitz et al.** *J.VIROL.,* 1988, vol. 167, 400-406
- **Cosset et al.** *J. Virol.,* 1995, vol. 69, 7430-7436
- **Miller et al.** *Mol. Cell Biol.,* 1985, vol. 5, 431-437
- **Miller ; Buttimore.** *Mol. and Cell. Biol.,* 1986, vol. 6, 2895-2902
- **Mann et al.** *Cell,* 1983, vol. 33, 153-159
- **Ory et al.** *Proc. Natl. Acad. Sci. USA,* 1996, vol. 93, 11400-11406
- **Eckert, H.G. et al.** *Blood,* 1996, vol. 88, 3407-3415
- **Baum, C. et al.** Concept in Gene Therapy. De Gruyter-Verlag, 1997, 233-266
- **Uetsuki et al.** *J. Biol. Chem.,* 1989, vol. 264, 5791-5798
- **Wakabayashi-Ito ; Nagata.** *J. Biol. Chem.,* 1994, vol. 269, 29831-29837
- **Markowitz et al.** *Virol.,* 1988, vol. 167, 400-406
- **Baum et al.** *J. Virol.,* 1995, vol. 69, 7541-7547
- **Hildinger et al.** *Gene Therapy,* 1998, vol. 5, 1575-1579
- **Laker et al.** *J. Virol.,* 1998, vol. 72, 339-348
- **Garin et al.** *Blood,* 2001, vol. 97, 122-129
- **Miller et al.** *J. Virol,* 1991, vol. 65, 2220-2224
- **Sanburn ; Cornetta.** *Gene Ther.,* 1999, vol. 6, 1340-1345
- **Kim et al.** *Gene,* 1990, vol. 91, 217-223
- **Kim et al.** *J. Biotechnol.,* 2002, vol. 93, 183-187
- **Verzeletti et al.** *Hum. Gene Ther.,* 1998, vol. 10, 2243-2251
- **von Hildinger et al.** *J. Virol.,* 1999, vol. 73, 4083-4089
- **Plavec et al.** *Gene Ther.,* 1997, vol. 4, 128-139

- **Rudoll et al.** *Gene Therapy,* 1996, vol. 3, 695-705
- **Mavilio et al.** *Blood,* vol. 83, 1988-1997
- **Guild et al.** *Proc. Natl. Acad. Sci., USA,* 1988, vol. 85, 1595-1599
- **Sorrentino et al.** *Science,* 1992, vol. 257, 99-103
- **Eckert et al.** *Blood,* 1996, vol. 88, 3407-3415
- **Miller et al.** *Mol. Cell. Biol.,* 1985, vol. 5, 431-437
- **Pawliuk et al.** *Blood,* 1994, vol. 84, 2868-2877
- **Mavilio et al.** *Blood,* 1994, vol. 83, 19881997
- **Rudoll et al.** *Gene Ther.,* 1996, vol. 3, 695-705
- **Fehse et al.** *Hum. Gene Ther.,* 1997, vol. 8, 1815-1824
- **Conneally et al.** *Blood,* 1996, vol. 87, 456-464

<u>Sequenzprotokoll</u>

(1) Allgemeine Angaben:

(i) Anmelder:

(A) NAME: CellTec GmbH Biotechnologie
(B) STRASSE: Frohmestrasse 110
(C) ORT: Hamburg
(E) LAND: Deutschland
(F) POSTLEITZAHL: D 22459
(G) TELEFON: 0049 40 55905 359
(H) TELEFAX: 0049 40 55905 364

(ii) BEZEICHNUNG DER ERFINDUNG: Retrovirale Suizidgenvektoren

(iii) ANZAHL DER SEQUENZEN: 18

(iv) COMPUTER-LESBARE FASSUNG:

(A) DATENTRÄGER: Floppy disk
(B) Computer: IBM PC compatible

(C) BETRIEBSSYSTEM: Windows NT 4.0
(D) SOFTWARE: Word, Version...

(2) Angaben zu SEQ ID NO: 1:

(i) SEQUENZKENNZEICHEN:

(A) LÄNGE: 7185 Basenpaare
(B) ART: Nukleotid
(C) STRANGFORM: Doppelstrang
(D) TOPOLOGIE: ringförmig

(ii) ART DES MOLEKÜLS: Sonstige Nucleinsäure

(A) BESCHREIBUNG: /desc = "Provirale Plasmid-DNA"

(ix) MERKMAL:

(A) NAME / SCHLÜSSEL: misc_RNA
(B) LAGE: 1-423
(D) SONSTIGE ANGABEN: /note= "Plasmid-Rueckgrat"

(ix) MERKMAL:

(A) NAME / SCHLÜSSEL: misc_RNA
(B) LAGE: 424-994
(D) SONSTIGE ANGABEN: /note= "5'-LTR"

(ix) MERKMAL:

(A) NAME / SCHLÜSSEL: 5' UTR
(B) LAGE: 995-1487

(ix) MERKMAL:

(A) NAME / SCHLÜSSEL: mat_peptide
(B) LAGE: 1488-2615
(D) SONSTIGE ANGABEN: /note= "HSV-TK cDNA"

(ix) MERKMAL:

(A) NAME / SCHLÜSSEL: misc_RNA
(B) LAGE: 2626-3250
(D) SONSTIGE ANGABEN: /note= "IRES"

(ix) MERKMAL:

(A) NAME / SCHLÜSSEL: mat_peptide
(B) LAGE: 3298-4098
(D) SONSTIGE ANGABEN: /note= "Neo cDNA"

(ix) MERKMAL:

(A) NAME / SCHLÜSSEL: misc_RNA
(B) LAGE: 4345-4931
(D) SONSTIGE ANGABEN: /note= "3'-LTR"

(ix) MERKMAL:

(A) NAME / SCHLÜSSEL: misc_RNA
(B) LAGE: 4932-7185
(D) SONSTIGE ANGABEN: /note= "Plasmid-Rueckgrat"

(ix) MERKMAL:

(A) NAME / SCHLÜSSEL: misc_RNA
(B) LAGE: 1..7185
(D) SONSTIGE ANGABEN: / product= "MPSV 11 TIN" /note= "Retroviraler Suizidgenvektor"

(xi) SEQUENZBESCHREIBUNG: SEQ ID NO: 1:

```
.....ctgcctcgcg cgtttcggtg atgacggtga aaacctctga cacatgcagc tcccggagac   60
ggtcacagct tgtctgtaag cggatgccgg gagcagacaa gcccgtcagg gcgcgtcagc   120
gggtgttggc gggtgtcggg gcgcagccat gacccagtca cgtagcgata gttactatgc   180
ggcatcagag cagattgtac tgagagtgca ccatatgcgg tgtgaaatac cgcacagatg   240
cgtaaggaga aaataccgca tcaggcgcca ttcgccattc aggctgcgca actgttggga   300
agggcgatcg gtgcgggcct cttcgctatt acgccagctg gcgaaagggg gatgtgctgc   360
aaggcgatta agttgggtaa cgccagggtt ttcccagtca cgacgttgta aaacgacggc   420
cagtgaatta gtactctagc ttaagtaacg ccattttgca aggcatggaa aatacataac   480
tgagaataga gaagttcaga tcaaggttag gaacagagag acagcagaat atgggccaaa   540
caggatatct gtggtaagca gttcctgccc cggctcaggg ccaagaacag ttggaacagc   600
agaatatggg ccaaacagga tatctgtggt aagcagttcc tgccccggct cagggccaag   660
aacagatggt ccccagatgc ggtcccgccc tcagcagttt ctagagaacc atcagatgtt   720
tccagggtgc cccaaggacc tgaaatgacc ctgtgcctta tttgaactaa ccaatcagtt   780
cgcttctcgc ttctgttcgc gcgcttctgc tccccgagct caataaaaga gcccacaacc   840
cctcactcgg cgcgccagtc ctccgataga ctgcgtcgcc cgggtacccg tattcccaat   900
aaagcctctt gctgtttgca tccgaatcgt ggactcgctg atccttggga gggtctcctc   960
agattgattg actgcccacc tcgggggtct ttcatttgga ggttccaccg agatttggag   1020
acccctgccc agggaccacc gacccccccg ccgggaggta agctggccag cggtcgtttc   1080
gtgtctgtct ctgtctttgt gcgtgtttgt gccggcatct aatgtttgcg cctgcgtctg   1140
tactagttgg ctaactagat ctgtatctgg cggtcccgcg gaagaactga cgagttcgta   1200
ttcccggccg cagcccctgg gagacgtccc agcggcctcg gggcccgtt ttgtggccca   1260
ttctgtatca gttaacctac ccgagtcgga ctttttggag ctccgccact gtccgagggg   1320
tacgtggctt tgttggggga cgagagacag agacacttcc cgcccccgtc tgaatttttg   1380
ctttcggttt tacgccgaaa ccgcgccgcg cgtcttgtct gctgcagcat cgttctgtgt   1440
tgtctctgtc tgacgtggtt ctgtattgtc tgaaaatagc ggccgccatg gcttcgtacc   1500
cctgccatca acacgcgtct gcgttcgacc aggctgcgcg ttctcgcggc catagcaacc   1560
gacgtacggc gttgcgccct cgccggcagc aagaagccac ggaagtccgc ccggagcaga   1620
aaatgcccac gctactgcgg gtttatatag acggtcccca cgggatgggg aaaaccacca   1680
ccacgcaact gctggtggcc ctgggttcgc gcgacgatat cgtctacgta cccgagccga   1740
tgacttactg gcgggtgctg gggcttccg agacaatcgc gaacatctac accacacaac   1800
accgcctcga ccagggtgag atatcggccg gggacgcggc ggtggtaatg acaagcgccc   1860
agataacaat gggcatgcct tatgccgtga ccgacgccgt tctggctcct catatcgggg   1920
gggaggctgg gagctcacat gccccgcccc cggccctcac cctcatcttc gaccgccatc   1980
ccatcgccgc cctcctgtgc tacccggccg cgcggtacct tatgggcagc atgacccccc   2040
aggccgtgct ggcgttcgtg gccctcatcc cgccgacctt gcccggcacc aacatcgtgc   2100
ttgggccct tccggaggac agacacatcg accgcctggc caaacgccag cgccccggcg   2160
agcggctgga cctggctatg ctggctgcga ttcgccgcgt ttacgggcta cttgccaata   2220
cggtgcggta tctgcagtgc ggcgggtcgt ggcgggagga ctggggacag ctttcggga   2280
```

cggccgtgcc gccccagggt gccgagcccc agagcaacgc gggcccacga ccccatatcg 2340
gggacacgtt atttaccctg tttcgggccc ccgagttgct ggcccccaac ggcgacctgt 2400
ataacgtgtt tgcctgggcc ttggacgtct tggccaaacg cctccgttcc atgcacgtct 2460
ttatcctgga ttacgaccaa tcgcccgccg gctgccggga cgccctgctg caacttacct 2520
ccgggatggt ccagacccac gtcaccaccc ccggctccat accgacgata tgcgacctgg 2580
cgcgcacgtt tgcccgggag atgggggagg ctaactgata agcttaaaac agctctgggg 2640
ttgtacccac cccagaggcc cacgtggcgg ctagtactcc ggtattgcgg taccettgta 2700
cgcctgtttt atactccctt cccgtaactt agacgcacaa aaccaagttc aatagaaggg 2760
ggtacaaacc agtaccacca cgaacaagca cttctgtttc cccggtgatg tcgtatagac 2820
tgcttgcgtg gttgaaagcg acggatccgt tatccgctta tgtacttcga gaagcccagt 2880
accacctcgg aatcttcaat gcgttgcgct cagcactcaa ccccagagtg tagcttaggc 2940
tgatgagtct ggacatccct caccggtgac ggtggtccag gctgcgttgg cggcctacct 3000
atggctaacg ccatgggacg ctagttgtga acaaggtgtg aagagcctat tgagctacat 3060
aagaatcctc cggcccctga atgcggctaa tcccaacctc ggagcaggtg gtcacaaacc 3120
agtgattggc ctgtcgtaac gcgcaagtcc gtggcggaac cgactacttt gggtgtccgt 3180
gtttcctttt attttattgt ggctgcttat ggtgacaatc acagattgtt atcataaagc 3240
gaattggatt gcggccgctc tagaactagt ggatctgaat taattcctgc agccaatatg 3300
ggatcggcca ttgaacaaga tggattgcac gcaggttctc cggccgcttg ggtggagagg 3360
ctattcggct atgactgggc acaacagaca atcggctgct ctgatgccgc cgtgttccgg 3420
ctgtcagcgc aggggcgccc ggttcttttt gtcaagaccg acctgtccgg tgccctgaat 3480
gaactgcagg acgaggcagc gcggctatcg tggctggcca cgacgggcgt tccttgcgca 3540
gctgtgctcg acgttgtcac tgaagcggga agggactggc tgctattggg cgaagtgccg 3600
gggcaggatc tcctgtcatc tcaccttgct cctgccgaga aagtatccat catggctgat 3660
gcaatgcggc ggctgcatac gcttgatccg gctacctgcc cattcgacca ccaagcgaaa 3720
catcgcatcg agcgagcacg tactcggatg gaagccggtc ttgtcgatca ggatgatctg 3780
gacgaagagc atcaggggct cgcgccagcc gaactgttcg ccaggctcaa ggcgcgcatg 3840
cccgacggcg aggatctcgt cgtgacccat ggcgatgcct gcttgccgaa tatcatggtg 3900
gaaaatggcc gcttttctgg attcatcgac tgtggccggc tgggtgtggc ggaccgctat 3960
caggacatag cgttggctac ccgtgatatt gctgaagagc ttggcggcga atgggctgac 4020
cgcttcctcg tgctttacgg tatcgccgct cccgattcgc agcgcatcgc cttctatcgc 4080
cttcttgacg agttcttctg agcgggactc tggggttcga aatgaccgac caagcgacgc 4140
ccaacctgcc atcacgagat ttcgattcca ccgccgcctt ctatgaaagg ttgggcttcg 4200
gaatcgtttt ccgggacgcc ggctggatga tcctccagcg cggggatctc atgctggagt 4260
tcttcgccca ccccggatc caagcttaac acgagccata gatagaataa aagattttat 4320
ttagtctcca gaaaaagggg ggaatgaaag accccacctg taggtttggc aagctagctt 4380
aagtaacgcc attttgcaag gcatggaaaa tacataactg agaatagaga agttcagatc 4440
aaggttagga acagagagac agcagaatat gggccaaaca ggatatctgt ggtaagcagt 4500
tcctgccccg gctcagggcc aagaacagtt ggaacagcag aatatgggcc aaacaggata 4560
tctgtggtaa gcagttcctg ccccggctca gggccaagaa cagatggtcc ccagatgcgg 4620
tcccgccctc agcagtttct agagaaccat cagatgtttc cagggtgccc caaggacctg 4680
aaatgaccct gtgccttatt tgaactaacc aatcagttcg cttctcgctt ctgttcgcgc 4740
gcttctgctc cccgagctca ataaaagagc ccacaacccc tcactcggcg cgccagtcct 4800
ccgatagact gcgtcgcccg ggtacccgtg ttctcaataa accctcttgc agttgcatcc 4860
gactcgtggt ctcgctgttc cttgggaggg tctcctctga gtgattgact gcccacctcg 4920
ggggtctttc attctcgagc agcttggcgt aatcatggtc atagctgttt cctgtgtgaa 4980
attgttatcc gctcacaatt ccacacaaca tacgagccgg aagcataaag tgtaaagcct 5040
ggggtgccta atgagtgagc taactcacat taattgcgtt gcgctcactg cccgctttcc 5100
agtcgggaaa cctgtcgtgc cagctgcatt aatgaatcgg ccaacgcgcg gggagaggcg 5160
gtttgcgtat tgggcgctct tccgcttcct cgctcactga ctcgctgcgc tcggtcgttc 5220
ggctgcggcg agcggtatca gctcactcaa aggcggtaat acggttatcc acagaatcag 5280
gggataacgc aggaaagaac atgtgagcaa aaggccagca aaaggccagg aaccgtaaaa 5340
aggccgcgtt gctggcgttt ttccataggc tccgcccccc tgacgagcat cacaaaaatc 5400
gacgctcaag tcagaggtgg cgaaacccga caggactata aagataccag gcgtttcccc 5460
ctggaagctc cctcgtgcgc tctcctgttc cgaccctgcc gcttaccgga tacctgtccg 5520
cctttctccc ttcgggaagc gtggcgcttt ctcaatgctc acgctgtagg tatctcagtt 5580
cggtgtaggt cgttcgctcc aagctgggct gtgtgcacga accccccgtt cagcccgacc 5640
gctgcgcctt atccggtaac tatcgtcttg agtccaaccc ggtaagacac gacttatcgc 5700
cactggcagc agccactggt aacaggatta gcagagcgag gtatgtaggc ggtgctacag 5760
agttcttgaa gtggtggcct aactacggct acactagaag gacagtattt ggtatctgcg 5820
ctctgctgaa gccagttacc ttcggaaaaa gagttggtag ctcttgatcc ggcaaacaaa 5880
ccaccgctgg tagcggtggt ttttttgttt gcaagcagca gattacgcgc agaaaaaaag 5940
gatctcaaga agatcctttg atcttttcta cggggtctga cgctcagtgg aacgaaaact 6000
cacgttaagg gattttggtc atgagattat caaaaaggat cttcacctag atccttttaa 6060

```
attaaaaatg aagttttaaa tcaatctaaa gtatatatga gtaaacttgg tctgacagtt      6120
accaatgctt aatcagtgag gcacctatct cagcgatctg tctatttcgt tcatccatag      6180
ttgcctgact ccccgtcgtg tagataacta cgatacggga gggcttacca tctggcccca      6240
gtgctgcaat gataccgcga gacccacgct caccggctcc agatttatca gcaataaacc      6300
agccagccgg aagggccgag cgcagaagtg gtcctgcaac tttatccgcc tccatccagt      6360
ctattaattg ttgccgggaa gctagagtaa gtagttcgcc agttaatagt ttgcgcaacg      6420
ttgttgccat tgctgctggc atcgtggtgt cacgctcgtc gtttggtatg gcttcattca      6480
gctccggttc ccaacgatca aggcgagtta catgatcccc catgttgtgc aaaaaagcgg      6540
ttagctcctt cggtcctccg atcgttgtca gaagtaagtt ggccgcagtg ttatcactca      6600
tggttatggc agcactgcat aattctctta ctgtcatgcc atccgtaaga tgcttttctg      6660
tgactggtga gtactcaacc aagtcattct gagaatagtg tatgcggcga ccgagttgct      6720
cttgcccggc gtcaatacgg gataataccg cgccacatag cagaacttta aaagtgctca      6780
tcattggaaa acgttcttcg gggcgaaaac tctcaaggat cttaccgctg ttgagatcca      6840
gttcgatgta acccactcgt gcacccaact gatcttcagc atcttttact ttcaccagcg      6900
tttctgggtg agcaaaaaca ggaaggcaaa atgccgcaaa aaagggaata agggcgacac      6960
ggaaatgttg aatactcata ctcttccttt ttcaatatta ttgaagcatt tatcagggtt      7020
attgtctcat gagcggatac atatttgaat gtatttagaa aaataaacaa ataggggttc      7080
cgcgcacatt tccccgaaaa gtgccacctg acgtctaaga aaccattatt atcatgacat      7140
taacctataa aaataggcgt atcacgaggc cctttcgtct tcaag                     7185
```

(2) Angaben zu SEQ ID NO: 2:

(i) SEQUENZKENNZEICHEN:

(A) LÄNGE: 7235 Basenpaare
(B) ART: Nukleotid
(C) STRANGFORM: Doppelstrang
(D) TOPOLOGIE: ringförmig

(ii) ART DES MOLEKÜLS: Sonstige Nucleinsäure

(A) BESCHREIBUNG: /desc = "Provirale Plasmid-DNA"

(ix) MERKMAL:

(A) NAME / SCHLÜSSEL: misc_RNA
(B) LAGE: 1-423
(D) SONSTIGE ANGABEN: /note= "Plasmid-Rueckgrat"

(ix) MERKMAL:

(A) NAME / SCHLÜSSEL: misc_RNA
(B) LAGE: 424-994
(D) SONSTIGE ANGABEN: /note= "5'-LTR"

(ix) MERKMAL:

(A) NAME / SCHLÜSSEL: 5' UTR
(B) LAGE: 995-1537

(ix) MERKMAL:

(A) NAME / SCHLÜSSEL: mat_peptide
(B) LAGE: 1538-2665
(D) SONSTIGE ANGABEN: /note= "HSV-TK cDNA"

(ix) MERKMAL:

(A) NAME / SCHLÜSSEL: misc_RNA
(B) LAGE: 2676-3300
(D) SONSTIGE ANGABEN: /note= "IRES"

(ix) MERKMAL:

(A) NAME / SCHLÜSSEL: mat_peptide
(B) LAGE: 3348-4148
(D) SONSTIGE ANGABEN: /note= "Neo cDNA"

(ix) MERKMAL:

(A) NAME / SCHLÜSSEL: misc_RNA
(B) LAGE: 4395-4981
(D) SONSTIGE ANGABEN: /note= "3'-LTR"

(ix) MERKMAL:

(A) NAME / SCHLÜSSEL: misc_RNA
(B) LAGE: 4982-7235
(D) SONSTIGE ANGABEN: /note= "Plasmid-Rueckgrat"

(ix) MERKMAL:

(A) NAME / SCHLÜSSEL: misc_RNA
(B) LAGE: 1..7235
(D) SONSTIGE ANGABEN: / product= "MPSV 91 TIN" /note= "Retroviraler Suizidgenvektor"

(xi) SEQUENZBESCHREIBUNG: SEQ ID NO: 2:

```
ctgcctcgcg cgtttcggtg atgacggtga aaacctctga cacatgcagc tcccggagac     60
ggtcacagct tgtctgtaag cggatgccgg gagcagacaa gcccgtcagg gcgcgtcagc    120
gggtgttggc gggtgtcggg gcgcagccat gacccagtca cgtagcgata gttactatgc    180
ggcatcagag cagattgtac tgagagtgca ccatatgcgg tgtgaaatac cgcacagatg    240
cgtaaggaga aaataccgca tcaggcgcca ttcgccattc aggctgcgca actgttggga    300
agggcgatcg gtgcgggcct cttcgctatt acgccagctg gcgaaagggg gatgtgctgc    360
aaggcgatta agttgggtaa cgccagggtt ttcccagtca cgacgttgta aaacgacggc    420
cagtgaatta gtactctagc ttaagtaacg ccattttgca aggcatggaa aatacataac    480
tgagaataga gaagttcaga tcaaggttag gaacagagag acagcagaat atgggccaaa    540
caggatatct gtggtaagca gttcctgccc cggctcaggg ccaagaacag ttggaacagc    600
agaatatggg ccaaacagga tatctgtggt aagcagttcc tgccccggct cagggccaag    660
aacagatggt ccccagatgc ggtcccgccc tcagcagttt ctagagaacc atcagatgtt    720
tccagggtgc cccaaggacc tgaaatgacc ctgtgcctta tttgaactaa ccaatcagtt    780
cgcttctcgc ttctgttcgc gcgcttctgc tccccgagct caataaaaga gcccacaacc    840
cctcactcgg ggcgccagtc ctccgattga ctgagtcgcc cgggtacccg tattcccaat    900
aaagcctctt gctgtttgca tccgaatcgt ggactcgctg atccttggga gggtctcctc    960
agattgattg actgcccacc tcgggggtct ttcatttgga ggttccaccg agatttggag   1020
acccctgccc agggaccacc gacccccccg ccgggaggta agctggccag cggtcgtttc   1080
gtgtctgtct ctgtctttgt gcgtgtttgt gccggcatct agtgtttgcg cctgcgtctg   1140
tactagttgg ctaactagat ctgtatctgg cggtcccgcg gaagaactga cgagttcgta   1200
ttcccggccg cagcccctgg gagacgtccc agcggcctcg gggcccgtt ttgtggccca   1260
ttctgtatca gttaacctac ccgagtcgga ctttttggag ctccgccact gtccgagggg   1320
tacgtggctt tgttgggga cgagagacag agacacttcc cgcccccgtc tgaatttttg   1380
ctttcggttt tacgccgaaa ccgcgccgcg cgtcttgtct gctgcagcat cgttctgtgt   1440
tgtctctgtc tgactgtgtt tctgtatttg tctgaaaatt agctcgacaa agttaagtaa   1500
tagtccctct ctccaagctc acttacaggc ggccgccatg gcttcgtacc cctgccatca   1560
acacgcgtct gcgttcgacc aggctgcgcg ttctcgcggc catagcaacc gacgtacggc   1620
gttgcgccct cgccggcagc aagaagccac ggaagtccgc ccggagcaga aaatgcccac   1680
gctactgcgg gtttatatag acggtcccca cgggatgggg aaaaccacca ccacgcaact   1740
gctggtggcc ctgggttcgc gcgacgatat cgtctacgta cccgagccga tgacttactg   1800
gcgggtgctg gggcttccg agacaatcgc gaacatctac accacacaac accgcctcga   1860
ccagggtgag atatcggccg gggacgcggc ggtggtaatg acaagcgccc agataacaat   1920
gggcatgcct tatgccgtga ccgacgccgt tctggctcct catatcgggg gggaggctgg   1980
gagctcacat gccccgcccc cggccctcac cctcatcttc gaccgccatc ccatcgccgc   2040
cctcctgtgc tacccggccg cgcggtacct tatgggcagc atgacccccc aggccgtgct   2100
ggcgttcgtg gccctcatcc cgccgacctt gcccggcacc aacatcgtgc ttgggggccct  2160
tccggaggac agacacatcg accgcctggc caaacgccag cgccccggcg agcggctgga   2220
cctggctatg ctggctgcga ttcgccgcgt ttacgggcta cttgccaata cggtgcggta   2280
tctgcagtgc ggcgggtcgt ggcgggagga ctggggacag ctttcgggga cggccgtgcc   2340
gccccagggt gccgagcccc agagcaacgc gggcccacga ccccatatcg ggacacgtt    2400
atttaccctg tttcgggccc ccgagttgct ggcccccaac ggcgacctgt ataacgtgtt   2460
tgcctgggcc ttggacgtct tggccaaacg cctccgttcc atgcacgtct ttatcctgga   2520
ttacgaccaa tcgcccgccg gctgccggga cgccctgctg caacttacct ccgggatggt   2580
ccagacccac gtcaccaccc ccggctccat accgacgata tgcgacctgg cgcgcacgtt   2640
tgcccgggag atgggggagg ctaactgata agcttaaaac agctctgggg ttgtacccac   2700
cccagaggcc cacgtggcgg ctagtactcc ggtattgcgg tacccttgta cgcctgtttt   2760
atactccctt cccgtaactt agacgcacaa aaccaagttc aatagaaggg ggtacaaacc   2820
agtaccacca cgaacaagca cttctgtttc cccggtgatg tcgtatagac tgcttgcgtg   2880
gttgaaagcg acggatccgt tatccgctta tgtacttcga gaagcccagt accacctcgg   2940
aatcttcaat gcgttgcgct cagcactcaa ccccagagtg tagcttaggc tgatgagtct   3000
ggacatccct caccggtgac ggtggtccag gctgcgttgg cggcctacct atggctaacg   3060
ccatgggacg ctagttgtga acaaggtgtg aagagcctat tgagctacat aagaatcctc   3120
cggcccctga atgcggctaa tcccaacctc ggagcaggtg gtcacaaacc agtgattggc   3180
```

```
ctgtcgtaac gcgcaagtcc gtggcggaac cgactacttt gggtgtccgt gtttcctttt 3240
attttattgt ggctgcttat ggtgacaatc acagattgtt atcataaagc gaattggatt 3300
gcggccgctc tagaactagt ggatctgaat taattcctgc agccaatatg ggatcggcca 3360
ttgaacaaga tggattgcac gcaggttctc cggccgcttg ggtggagagg ctattcggct 3420
atgactgggc acaacagaca atcggctgct ctgatgccgc cgtgttccgg ctgtcagcgc 3480
aggggcgccc ggttcttttt gtcaagaccg acctgtccgg tgccctgaat gaactgcagg 3540
acgaggcagc gcggctatcg tggctggcca cgacgggcgt tccttgcgca gctgtgctcg 3600
acgttgtcac tgaagcggga agggactggc tgctattggg cgaagtgccg gggcaggatc 3660
tcctgtcatc tcaccttgct cctgccgaga aagtatccat catggctgat gcaatgcggc 3720
ggctgcatac gcttgatccg gctacctgcc cattcgacca ccaagcgaaa catcgcatcg 3780
agcgagcacg tactcggatg gaagccggtc ttgtcgatca ggatgatctg gacgaagagc 3840
atcaggggct cgcgccagcc gaactgttcg ccaggctcaa ggcgcgcatg cccgacggcg 3900
aggatctcgt cgtgacccat ggcgatgcct gcttgccgaa tatcatggtg gaaaatggcc 3960
gcttttctgg attcatcgac tgtggccggc tgggtgtggc ggaccgctat caggacatag 4020
cgttggctac ccgtgatatt gctgaagagc ttggcggcga atgggctgac cgcttcctcg 4080
tgctttacgg tatcgccgct cccgattcgc agcgcatcgc cttctatcgc cttcttgacg 4140
agttcttctg agcgggactc tggggttcga aatgaccgac caagcgacgc ccaacctgcc 4200
atcacgagat ttcgattcca ccgccgcctt ctatgaaagg ttgggcttcg gaatcgtttt 4260
ccgggacgcc ggctggatga tcctccagcg cggggatctc atgctggagt tcttcgccca 4320
cccccggatc caagcttaac acgagccata gatagaataa aagattttat ttagtctcca 4380
gaaaaagggg ggaatgaaag accccacctg taggtttggc aagctagctt aagtaacgcc 4440
attttgcaag gcatggaaaa tacataactg agaatagaga agttcagatc aaggttagga 4500
acagagagac agcagaatat gggccaaaca ggatatctgt ggtaagcagt tcctgccccg 4560
gctcagggcc aagaacagtt ggaacagcag aatatgggcc aaacaggata tctgtggtaa 4620
gcagttcctg ccccggctca gggccaagaa cagatggtcc ccagatgcgg tcccgccctc 4680
agcagtttct agagaaccat cagatgtttc cagggtgccc caaggacctg aaatgaccct 4740
gtgccttatt tgaactaacc aatcagttcg cttctcgctt ctgttcgcgc gcttctgctc 4800
cccgagctca ataaaagagc ccacaacccc tcactcggcg cgccagtcct ccgatagact 4860
gcgtcgcccg ggtacccgtg ttctcaataa accctcttgc agttgcatcc gactcgtggt 4920
ctcgctgttc cttgggaggg tctcctctga gtgattgact gcccacctcg gggtctttc 4980
attctcgagc agcttggcgt aatcatggtc atagctgttt cctgtgtgaa attgttatcc 5040
gctcacaatt ccacacaaca tacgagccgg aagcataaag tgtaaagcct ggggtgccta 5100
atgagtgagc taactcacat taattgcgtt gcgctcactg cccgctttcc agtcgggaaa 5160
cctgtcgtgc cagctgcatt aatgaatcgg ccaacgcgcg gggagaggcg gtttgcgtat 5220
tgggcgctct tccgcttcct cgctcactga ctcgctgcgc tcggtcgttc ggctgcggcg 5280
agcggtatca gctcactcaa aggcggtaat acggttatcc acagaatcag gggataacgc 5340
aggaaagaac atgtgagcaa aaggccagca aaaggccagg aaccgtaaaa aggccgcgtt 5400
gctggcgttt ttccataggc tccgcccccc tgacgagcat cacaaaaatc gacgctcaag 5460
tcagaggtgg cgaaacccga caggactata aagataccag gcgtttcccc ctggaagctc 5520
cctcgtgcgc tctcctgttc cgaccctgcc gcttaccgga tacctgtccg cctttctccc 5580
ttcgggaagc gtggcgcttt ctcaatgctc acgctgtagg tatctcagtt cggtgtaggt 5640
cgttcgctcc aagctgggct gtgtgcacga accccccgtt cagcccgacc gctgcgcctt 5700
atccggtaac tatcgtcttg agtccaaccc ggtaagacac gacttatcgc cactggcagc 5760
agccactggt aacaggatta gcagagcgag gtatgtaggc ggtgctacag agttcttgaa 5820
gtggtggcct aactacggct acactagaag gacagtattt ggtatctgcg ctctgctgaa 5880
gccagttacc ttcggaaaaa gagttggtag ctcttgatcc ggcaaacaaa ccaccgctgg 5940
tagcggtggt ttttttgttt gcaagcagca gattacgcgc agaaaaaaag gatctcaaga 6000
agatcctttg atcttttcta cggggtctga cgctcagtgg aacgaaaact cacgttaagg 6060
gattttggtc atgagattat caaaaaggat cttcacctag atccttttaa attaaaaatg 6120
aagttttaaa tcaatctaaa gtatatatga gtaaacttgg tctgacagtt accaatgctt 6180
aatcagtgag gcacctatct cagcgatctg tctatttcgt tcatccatag ttgcctgact 6240
ccccgtcgtg tagataacta cgatacggga gggcttacca tctggcccca gtgctgcaat 6300
gataccgcga gacccacgct caccggctcc agatttatca gcaataaacc agccagccgg 6360
aagggccgag cgcagaagtg gtcctgcaac tttatccgcc tccatccagt ctattaattg 6420
ttgccgggaa gctagagtaa gtagttcgcc agttaatagt ttgcgcaacg ttgttgccat 6480
tgctgctggc atcgtggtgt cacgctcgtc gtttggtatg gcttcattca gctccggttc 6540
ccaacgatca aggcgagtta catgatcccc catgttgtgc aaaaaagcgg ttagctcctt 6600
cggtcctccg atcgttgtca gaagtaagtt ggccgcagtg ttatcactca tggttatggc 6660
agcactgcat aattctctta ctgtcatgcc atccgtaaga tgcttttctg tgactggtga 6720
gtactcaacc aagtcattct gagaatagtg tatgcggcga ccgagttgct cttgcccggc 6780
gtcaatacgg gataataccg cgccacatag cagaacttta aaagtgctca tcattggaaa 6840
acgttcttcg gggcgaaaac tctcaaggat cttaccgctg ttgagatcca gttcgatgta 6900
acccactcgt gcacccaact gatcttcagc atcttttact ttcaccagcg tttctgggtg 6960
```

```
agcaaaaaca ggaaggcaaa atgccgcaaa aaagggaata agggcgacac ggaaatgttg      7020
aatactcata ctcttccttt ttcaatatta ttgaagcatt tatcagggtt attgtctcat      7080
gagcggatac atatttgaat gtatttagaa aaataaacaa ataggggttc cgcgcacatt      7140
tccccgaaaa gtgccacctg acgtctaaga aaccattatt atcatgacat taacctataa      7200
aaataggcgt atcacgaggc cctttcgtct tcaag                                 7235
```

(2) Angaben zu SEQ ID NO: 3:

(i) SEQUENZKENNZEICHEN:

(A) LÄNGE: 7185 Basenpaare
(B) ART: Nukleotid
(C) STRANGFORM: Doppelstrang
(D) TOPOLOGIE: ringförmig

(ii) ART DES MOLEKÜLS: Sonstige Nucleinsäure

(A) BESCHREIBUNG: /desc = "Provirale Plasmid-DNA"

(ix) MERKMAL:

(A) NAME / SCHLÜSSEL: misc_RNA
(B) LAGE: 1-423
(D) SONSTIGE ANGABEN: /note= "Plasmid-Rueckgrat"

(ix) MERKMAL:

(A) NAME / SCHLÜSSEL: misc_RNA
(B) LAGE: 424-994
(D) SONSTIGE ANGABEN: /note= "5'-LTR"

(ix) MERKMAL:

(A) NAME / SCHLÜSSEL: 5' UTR
(B) LAGE: 995-1487

(ix) MERKMAL:

(A) NAME / SCHLÜSSEL: mat_peptide
(B) LAGE: 1488-2615
(D) SONSTIGE ANGABEN: /note= "HSV-TK/splice motifs destroyed cDNA"

(ix) MERKMAL:

(A) NAME / SCHLÜSSEL: misc_RNA
(B) LAGE: 2626-3250
(D) SONSTIGE ANGABEN: /note= "IRES"

(ix) MERKMAL:

(A) NAME / SCHLÜSSEL: mat_peptide
(B) LAGE: 3298-4098
(D) SONSTIGE ANGABEN: /note= "Neo cDNA"

(ix) MERKMAL:

(A) NAME / SCHLÜSSEL: misc_RNA
(B) LAGE: 4345-4931
(D) SONSTIGE ANGABEN: /note= "3 ‘-LTR"

(ix) MERKMAL:

(A) NAME / SCHLÜSSEL: misc_RNA
(B) LAGE: 4932-7185
(D) SONSTIGE ANGABEN: /note= "Plasmid-Rueckgrat"

(ix) MERKMAL:

(A) NAME / SCHLÜSSEL: misc_RNA
(B) LAGE: 1..7185
(D) SONSTIGE ANGABEN: / product= "MPSV11T*IN" /note= "Retroviraler Suizidgenvektor"

(xi) SEQUENZBESCHREIBUNG: SEQ ID NO: 3:

```
ctgcctcgcg cgtttcggtg atgacggtga aaacctctga cacatgcagc tcccggagac     60
ggtcacagct tgtctgtaag cggatgccgg gagcagacaa gcccgtcagg gcgcgtcagc    120
gggtgttggc gggtgtcggg gcgcagccat gacccagtca cgtagcgata gttactatgc    180
ggcatcagag cagattgtac tgagagtgca ccatatgcgg tgtgaaatac cgcacagatg    240
cgtaaggaga aaataccgca tcaggcgcca ttcgccattc aggctgcgca actgttggga    300
agggcgatcg gtgcgggcct cttcgctatt acgccagctg gcgaaagggg gatgtgctgc    360
aaggcgatta agttgggtaa cgccagggtt ttcccagtca cgacgttgta aaacgacggc    420
cagtgaatta gtactctagc ttaagtaacg ccattttgca aggcatggaa aatacataac    480
tgagaataga gaagttcaga tcaaggttag gaacagagag acagcagaat atgggccaaa    540
caggatatct gtggtaagca gttcctgccc cggctcaggg ccaagaacag ttggaacagc    600
```

```
agaatatggg ccaaacagga tatctgtggt aagcagttcc tgccccggct cagggccaag    660
aacagatggt ccccagatgc ggtcccgccc tcagcagttt ctagagaacc atcagatgtt    720
tccagggtgc cccaaggacc tgaaatgacc ctgtgcctta tttgaactaa ccaatcagtt    780
cgcttctcgc ttctgttcgc gcgcttctgc tccccgagct caataaaaga gcccacaacc    840
cctcactcgg cgcgccagtc ctccgataga ctgcgtcgcc cgggtacccg tattcccaat    900
aaagcctctt gctgtttgca tccgaatcgt ggactcgctg atccttggga gggtctcctc    960
agattgattg actgcccacc tcgggggtct ttcatttgga ggttccaccg agatttggag   1020
acccctgccc agggaccacc gacccccccg ccgggaggta agctggccag cggtcgtttc   1080
gtgtctgtct ctgtctttgt gcgtgtttgt gccggcatct aatgtttgcg cctgcgtctg   1140
tactagttgg ctaactagat ctgtatctgg cggtcccgcg gaagaactga cgagttcgta   1200
ttcccggccg cagcccctgg gagacgtccc agcggcctcg gggcccgtt ttgtggccca    1260
ttctgtatca gttaacctac ccgagtcgga ctttttggag ctccgccact gtccgagggg   1320
tacgtggctt tgttgggga cgagagacag agacacttcc cgcccccgtc tgaatttttg   1380
ctttcggttt tacgccgaaa ccgcgccgcg cgtcttgtct gctgcagcat cgttctgtgt   1440
tgtctctgtc tgacgtggtt ctgtattgtc tgaaaatagc ggccgccatg gcttcgtacc   1500
cctgccatca acacgcgtct gcgttcgacc aggctgcgcg ttctcgcggc catagcaacc   1560
gacgtacggc gttgcgccct cgccggcagc aagaagccac ggaagtccgc ccggagcaga   1620
aaatgcccac gctactgcgg gtttatatag acggtcccca cgggatgggg aaaaccacca   1680
ccacgcaact gctggtggcc ctgggttcgc gcgacgatat cgtctacgta cccgagccga   1740
tgacttactg gcgggtgctg gggcttccg agacaatcgc gaacatctac accacacaac    1800
accgcctcga ccaaggggag atatcggccg ggacgcggc ggtggtaatg acaagcgccc    1860
agataacaat gggcatgcct tatgccgtga ccgacgccgt tctggctcct catatcgggg   1920
gggaggctgg gagctcacat gccccgcccc cggccctcac cctcatcttc gaccgccatc   1980
ccatcgccgc cctcctgtgc tacccggccg cgcggtacct tatgggcagc atgacccccc   2040
aagccgtgct ggcgttcgtg gccctcatcc cgccgacctt gcccggcacc aacatcgtgc   2100
ttggggccct tccggaggac agacacatcg accgcctggc caaacgccag cgccccggcg   2160
agcggctgga cctggctatg ctggctgcga ttcgccgcgt ttacgggcta cttgccaata   2220
cggtgcggta tctgcagtgc ggcgggtcgt ggcgggagga ctggggacag ctttcgggga   2280
cggccgtgcc gccccagggt gccgagcccc agagcaacgc gggcccacga ccccatatcg   2340
gggacacgtt atttaccctg tttcgggccc ccgagttgct ggcccccaac ggcgacctgt   2400
ataacgtgtt tgcctgggcc ttggacgtct tggccaaacg cctccgttcc atgcacgtct   2460
ttatcctgga ttacgaccaa tcgcccgccg gctgccggga cgccctgctg caacttacct   2520
ccgggatggt ccagacccac gtcaccaccc ccggctccat accgacgata tgcgacctgg   2580
cgcgcacgtt tgcccgggag atgggggagg ctaactgata agcttaaaac agctctgggg   2640
ttgtacccac cccagaggcc cacgtggcgg ctagtactcc ggtattgcgg taccctgta    2700
cgcctgtttt atactccctt cccgtaactt agacgcacaa aaccaagttc aatagaaggg   2760
ggtacaaacc agtaccacca cgaacaagca cttctgtttc cccggtgatg tcgtatagac   2820
tgcttgcgtg gttgaaagcg acggatccgt tatccgctta tgtacttcga gaagcccagt   2880
accacctcgg aatcttcaat gcgttgcgct cagcactcaa ccccagagtg tagcttaggc   2940
tgatgagtct ggacatccct caccggtgac ggtggtccag gctgcgttgg cggcctacct   3000
atggctaacg ccatgggacg ctagttgtga acaaggtgtg aagagcctat tgagctacat   3060
aagaatcctc cggcccctga atgcggctaa tcccaacctc ggagcaggtg gtcacaaacc   3120
agtgattggc ctgtcgtaac gcgcaagtcc gtggcggaac cgactacttt gggtgtccgt   3180
gtttcctttt attttattgt ggctgcttat ggtgacaatc acagattgtt atcataaagc   3240
gaattggatt gcggccgctc tagaactagt ggatctgaat taattcctgc agccaatatg   3300
ggatcggcca ttgaacaaga tggattgcac gcaggttctc cggccgcttg ggtggagagg   3360
ctattcggct atgactgggc acaacagaca atcggctgct ctgatgccgc cgtgttccgg   3420
ctgtcagcgc aggggcgccc ggttcttttt gtcaagaccg acctgtccgg tgccctgaat   3480
gaactgcagg acgaggcagc gcggctatcg tggctggcca cgacgggcgt tccttgcgca   3540
gctgtgctcg acgttgtcac tgaagcggga agggactggc tgctattggg cgaagtgccg   3600
gggcaggatc tcctgtcatc tcaccttgct cctgccgaga aagtatccat catggctgat   3660
gcaatgcggc ggctgcatac gcttgatccg gctacctgcc cattcgacca ccaagcgaaa   3720
catcgcatcg agcgagcacg tactcggatg gaagccggtc ttgtcgatca ggatgatctg   3780
gacgaagagc atcaggggct cgcgccagcc gaactgttcg ccaggctcaa ggcgcgcatg   3840
cccgacggcg aggatctcgt cgtgacccat ggcgatgcct gcttgccgaa tatcatggtg   3900
gaaaatggcc gcttttctgg attcatcgac tgtggccggc tgggtgtggc ggaccgctat   3960
caggacatag cgttggctac ccgtgatatt gctgaagagc ttggcggcga atgggctgac   4020
cgcttcctcg tgctttacgg tatcgccgct cccgattcgc agcgcatcgc cttctatcgc   4080
cttcttgacg agttcttctg agcgggactc tggggttcga aatgaccgac caagcgacgc   4140
ccaacctgcc atcacgagat ttcgattcca ccgccgcctt ctatgaaagg ttgggcttcg   4200
gaatcgtttt ccgggacgcc ggctggatga tcctccagcg cggggatctc atgctggagt   4260
tcttcgccca cccccggatc caagcttaac acgagccata gatagaataa aagattttat   4320
ttagtctcca gaaaaagggg ggaatgaaag accccacctg taggtttggc aagctagctt   4380
```

```
aagtaacgcc attttgcaag gcatggaaaa tacataactg agaatagaga agttcagatc   4440
aaggttagga acagagagac agcagaatat gggccaaaca ggatatctgt ggtaagcagt   4500
tcctgccccg gctcagggcc aagaacagtt ggaacagcag aatatgggcc aaacaggata   4560
tctgtggtaa gcagttcctg ccccggctca gggccaagaa cagatggtcc ccagatgcgg   4620
tcccgccctc agcagtttct agagaaccat cagatgtttc cagggtgccc caaggacctg   4680
aaatgaccct gtgccttatt tgaactaacc aatcagttcg cttctcgctt ctgttcgcgc   4740
gcttctgctc cccgagctca ataaaagagc ccacaacccc tcactcggcg cgccagtcct   4800
ccgatagact gcgtcgcccg ggtacccgtg ttctcaataa accctcttgc agttgcatcc   4860
gactcgtggt ctcgctgttc cttgggaggg tctcctctga gtgattgact gcccacctcg   4920
ggggtctttc attctcgagc agcttggcgt aatcatggtc atagctgttt cctgtgtgaa   4980
attgttatcc gctcacaatt ccacacaaca tacgagccgg aagcataaag tgtaaagcct   5040
ggggtgccta atgagtgagc taactcacat taattgcgtt gcgctcactg cccgctttcc   5100
agtcgggaaa cctgtcgtgc cagctgcatt aatgaatcgg ccaacgcgcg gggagaggcg   5160
gtttgcgtat tgggcgctct tccgcttcct cgctcactga ctcgctgcgc tcggtcgttc   5220
ggctgcggcg agcggtatca gctcactcaa aggcggtaat acggttatcc acagaatcag   5280
gggataacgc aggaaagaac atgtgagcaa aaggccagca aaaggccagg aaccgtaaaa   5340
aggccgcgtt gctggcgttt ttccataggc tccgcccccc tgacgagcat cacaaaaatc   5400
gacgctcaag tcagaggtgg cgaaacccga caggactata aagataccag gcgtttcccc   5460
ctggaagctc cctcgtgcgc tctcctgttc cgaccctgcc gcttaccgga tacctgtccg   5520
cctttctccc ttcgggaagc gtggcgcttt ctcaatgctc acgctgtagg tatctcagtt   5580
cggtgtaggt cgttcgctcc aagctgggct gtgtgcacga accccccgtt cagcccgacc   5640
gctgcgcctt atccggtaac tatcgtcttg agtccaaccc ggtaagacac gacttatcgc   5700
cactggcagc agccactggt aacaggatta gcagagcgag gtatgtaggc ggtgctacag   5760
agttcttgaa gtggtggcct aactacggct acactagaag gacagtattt ggtatctgcg   5820
ctctgctgaa gccagttacc ttcggaaaaa gagttggtag ctcttgatcc ggcaaacaaa   5880
ccaccgctgg tagcggtggt ttttttgttt gcaagcagca gattacgcgc agaaaaaaag   5940
gatctcaaga agatcctttg atcttttcta cggggtctga cgctcagtgg aacgaaaact   6000
cacgttaagg gattttggtc atgagattat caaaaaggat cttcacctag atccttttaa   6060
attaaaaatg aagttttaaa tcaatctaaa gtatatatga gtaaacttgg tctgacagtt   6120
accaatgctt aatcagtgag gcacctatct cagcgatctg tctatttcgt tcatccatag   6180
ttgcctgact ccccgtcgtg tagataacta cgatacggga gggcttacca tctggcccca   6240
gtgctgcaat gataccgcga gacccacgct caccggctcc agatttatca gcaataaacc   6300
agccagccgg aagggccgag cgcagaagtg gtcctgcaac tttatccgcc tccatccagt   6360
ctattaattg ttgccgggaa gctagagtaa gtagttcgcc agttaatagt ttgcgcaacg   6420
ttgttgccat tgctgctggc atcgtggtgt cacgctcgtc gtttggtatg gcttcattca   6480
gctccggttc ccaacgatca aggcgagtta catgatcccc catgttgtgc aaaaaagcgg   6540
ttagctcctt cggtcctccg atcgttgtca gaagtaagtt ggccgcagtg ttatcactca   6600
tggttatggc agcactgcat aattctctta ctgtcatgcc atccgtaaga tgcttttctg   6660
tgactggtga gtactcaacc aagtcattct gagaatagtg tatgcggcga ccgagttgct   6720
cttgcccggc gtcaatacgg gataataccg cgccacatag cagaacttta aaagtgctca   6780
tcattggaaa acgttcttcg gggcgaaaac tctcaaggat cttaccgctg ttgagatcca   6840
gttcgatgta acccactcgt gcacccaact gatcttcagc atcttttact ttcaccagcg   6900
tttctgggtg agcaaaaaca ggaaggcaaa atgccgcaaa aaagggaata agggcgacac   6960
ggaaatgttg aatactcata ctcttccttt ttcaatatta ttgaagcatt tatcagggtt   7020
attgtctcat gagcggatac atatttgaat gtatttagaa aaataaacaa ataggggttc   7080
cgcgcacatt tccccgaaaa gtgccacctg acgtctaaga aaccattatt atcatgacat   7140
taacctataa aaataggcgt atcacgaggc cctttcgtct tcaag                   7185
```

(2) Angaben zu SEQ ID NO: 4:

(i) SEQUENZKENNZEICHEN:

(A) LÄNGE: 7925 Basenpaare
(B) ART: Nukleotid
(C) STRANGFORM: Doppelstrang
(D) TOPOLOGIE: ringförmig

(ii) ART DES MOLEKÜLS: Sonstige Nucleinsäure

(A) BESCHREIBUNG: /desc = "Provirale Plasmid-DNA"

(ix) MERKMAL:

(A) NAME / SCHLÜSSEL: misc_RNA
(B) LAGE: 1-161
(D) SONSTIGE ANGABEN: /note= "Plasmid-Rueckgrat"

(ix) MERKMAL:

(A) NAME / SCHLÜSSEL: misc_RNA
(B) LAGE: 162-674
(D) SONSTIGE ANGABEN: /note= "5'-LTR'

(ix) MERKMAL:

(A) NAME / SCHLÜSSEL: 5'UTR
(B) LAGE: 675-1572

(ix) MERKMAL:

(A) NAME / SCHLÜSSEL: mat_peptide
(B) LAGE: 1573-2700
(D) SONSTIGE ANGABEN: /note= "HSV-TK cDNA"

(ix) MERKMAL:

(A) NAME / SCHLÜSSEL: misc_RNA
(B) LAGE: 2711-3335
(D) SONSTIGE ANGABEN: /note= "IRES"

(ix) MERKMAL:

(A) NAME / SCHLÜSSEL: mat_peptide
(B) LAGE: 3383-4183
(D) SONSTIGE ANGABEN: /note= "Neo cDNA"

(ix) MERKMAL:

(A) NAME / SCHLÜSSEL: misc_RNA
(B) LAGE: 4478-5067
(D) SONSTIGE ANGABEN: /note= "3'-LTR"

(ix) MERKMAL:

(A) NAME / SCHLÜSSEL: misc_RNA
(B) LAGE: 5068-7925
(D) SONSTIGE ANGABEN: /note= "Plasmid-Rueckgrat"

(ix) MERKMAL:

(A) NAME / SCHLÜSSEL: misc_RNA
(B) LAGE: 1..7925
(D) SONSTIGE ANGABEN: / product= "MO3TIN" /note= "Retroviraler Suizidgenvektor"

(xi) SEQUENZBESCHREIBUNG: SEQ ID NO: 4:

```
tcgagggggg gcccggtcac gattagtcca atttgttaaa gacaggatat caggtggtcc     60
aggctctagt tttgactcaa caatatcacc agctgaagcc tatagagtac gagccataat    120
agaataaaag attttattta gtctccagaa aaagggggga atgaaagacc ccacctgtag    180
gtttggcaag ctagcttaag taacgccatt ttgcaaggca tggaaaatac ataactgaga    240
atagagaagt tcagatcaag gttaggaaca gagagacagc agaatatggg ccaaacagga    300
tatctgtggt aagcagttcc tgccccggct cagggccaag aacagatggt ccccagatgc    360
ggtcccgccc tcagcagttt ctagagaacc atcagatgtt tccagggtgc cccaaggacc    420
tgaaatgacc ctgtgcctta tttgaactaa ccaatcagtt cgcttctcgc ttctgttcgc    480
gcgcttctgc tccccgagct caataaaaga gcccacaacc cctcactcgg ggcgccagtc    540
ctccgattga ctgagtcgcc cgggtacccg tattcccaat aaagcctctt gctgtttgca    600
tccgaatcgt ggtctcgctg ttccttggga gggtctcctc tgagtgattg actacccacg    660
acgggggtct ttcatttggg ggctcgtccg ggatttggag acccctgccc agggaccacc    720
gacccaccac cgggaggtaa gctggccagc aacttatctg tgtctgtccg attgtctagt    780
gtctatgttt gatgttatgc gcctgcgtct gtactagtta gctaactagc tctgtatctg    840
gcggacccgt ggtggaactg acgagttctg aacacccggc cgcaaccctg ggagacgtcc    900
cagggacttt gggggccgtt tttgtggccc gacctgagga agggagtcga tgtggaatcc    960
gaccccgtca ggatatgtgg ttctggtagg agacgagaac ctaaaacagt tcccgcctcc   1020
gtctgaattt ttgctttcgg tttggaaccg aagccgcgcg tcttgtctgc tgcagcgctg   1080
cagcatcgtt ctgtgttgtc tctgtctgac tgtgtttctg tatttgtctg aaaattaggg   1140
ccagactgtt accactccct taagtttgac cttaggtcac tggaaagatg tcgagcggat   1200
cgctcacaac cagtcggtag atgtcaagaa gagacgttgg gttaccttct gctctgcaga   1260
atggccaacc tttaacgtcg gatggccgcg agacggcacc tttaaccgag acctcatcac   1320
ccaggttaag atcaaggtct tttcacctgg cccgcatgga cacccagacc aggtccccta   1380
catcgtgacc tgggaagcct tggcttttga cccccctccc tgggtcaagc cctttgtaca   1440
ccctaagcct ccgcctcctc ttcctccatc cgccccgtct ctccccccttg aacctcctcg   1500
ttcgaccccg cctcgatcct ccctttatcc agccctcact ccttctctag gcggaccgcg   1560
gtggcggccg ccatggcttc gtacccctgc catcaacacg cgtctgcgtt cgaccaggct   1620
gcgcgttctc gcggccatag caaccgacgt acggcgttgc gccctcgccg gcagcaagaa   1680
gccacggaag tccgcccgga gcagaaaatg cccacgctac tgcgggttta tatagacggt   1740
ccccacggga tggggaaaac caccaccacg caactgctgg tggccctggg ttcgcgcgac   1800
```

```
gatatcgtct acgtacccga gccgatgact tactggcggg tgctgggggc ttccgagaca   1860
atcgcgaaca tctacaccac acaacaccgc ctcgaccagg gtgagatatc ggccggggac   1920
gcggcggtgg taatgacaag cgcccagata acaatgggca tgccttatgc cgtgaccgac   1980
gccgttctgg ctcctcatat cgggggggag gctgggagct cacatgcccc gcccccggcc   2040
ctcaccctca tcttcgaccg ccatcccatc gccgccctcc tgtgctaccc ggccgcgcgg   2100
taccttatgg gcagcatgac cccccaggcc gtgctggcgt tcgtggccct catcccgccg   2160
accttgcccg gcaccaacat cgtgcttggg gcccttccgg aggacagaca catcgaccgc   2220
ctggccaaac gccagcgccc cggcgagcgg ctggacctgg ctatgctggc tgcgattcgc   2280
cgcgtttacg ggctacttgc caatacggtg cggtatctgc agtgcggcgg gtcgtggcgg   2340
gaggactggg gacagctttc ggggacggcc gtgccgcccc agggtgccga gccccagagc   2400
aacgcgggcc cacgacccca tatcggggac acgttattta ccctgtttcg ggcccccgag   2460
ttgctggccc ccaacggcga cctgtataac gtgtttgcct gggccttgga cgtcttggcc   2520
aaacgcctcc gttccatgca cgtctttatc ctggattacg accaatcgcc cgccggctgc   2580
cgggacgccc tgctgcaact tacctccggg atggtccaga cccacgtcac cacccccggc   2640
tccataccga cgatatgcga cctggcgcgc acgtttgccc gggagatggg ggaggctaac   2700
tgataagctt aaaacagctc tggggttgta cccaccccag aggcccacgt ggcggctagt   2760
actccggtat tgcggtaccc ttgtacgcct gttttatact ccccttcccgt aacttagacg   2820
cacaaaacca agttcaatag aaggggggtac aaaccagtac caccacgaac aagcacttct   2880
gtttccccgg tgatgtcgta tagactgctt gcgtggttga aagcgacgga tccgttatcc   2940
gcttatgtac ttcgagaagc ccagtaccac ctcggaatct tcaatgcgtt gcgctcagca   3000
ctcaacccca gagtgtagct taggctgatg agtctggaca tccctcaccg gtgacggtgg   3060
tccaggctgc gttggcggcc tacctatggc taacgccatg ggacgctagt tgtgaacaag   3120
gtgtgaagag cctattgagc tacataagaa tcctccggcc cctgaatgcg gctaatccca   3180
acctcggagc aggtggtcac aaaccagtga ttggcctgtc gtaacgcgca agtccgtggc   3240
ggaaccgact actttgggtg tccgtgtttc cttttatttt attgtggctg cttatggtga   3300
caatcacaga ttgttatcat aaagcgaatt ggattgcggc cgctctagaa ctagtggatc   3360
tgaattaatt cctgcagcca atatgggatc ggccattgaa caagatggat tgcacgcagg   3420
ttctccggcc gcttgggtgg agaggctatt cggctatgac tgggcacaac agacaatcgg   3480
ctgctctgat gccgccgtgt tccggctgtc agcgcagggg cgcccggttc tttttgtcaa   3540
gaccgacctg tccggtgccc tgaatgaact gcaggacgag gcagcgcggc tatcgtggct   3600
ggccacgacg ggcgttcctt gcgcagctgt gctcgacgtt gtcactgaag cgggaaggga   3660
ctggctgcta ttgggcgaag tgccggggca ggatctcctg tcatctcacc ttgctcctgc   3720
cgagaaagta tccatcatgg ctgatgcaat gcggcggctg catacgcttg atccggctac   3780
ctgcccattc gaccaccaag cgaaacatcg catcgagcga gcacgtactc ggatggaagc   3840
cggtcttgtc gatcaggatg atctggacga agagcatcag gggctcgcgc cagccgaact   3900
gttcgccagg ctcaaggcgc gcatgcccga cggcgaggat ctcgtcgtga cccatggcga   3960
tgcctgcttg ccgaatatca tggtggaaaa tggccgcttt tctggattca tcgactgtgg   4020
ccggctgggt gtggcggacc gctatcagga catagcgttg gctacccgtg atattgctga   4080
agagcttggc ggcgaatggg ctgaccgctt cctcgtgctt tacggtatcg ccgctcccga   4140
ttcgcagcgc atcgccttct atcgccttct tgacgagttc ttctgagcgg gactctgggg   4200
ttcgaaatga ccgaccaagc gacgcccaac ctgccatcac gagatttcga ttccaccgcc   4260
gccttctatg aaaggttggg cttcggaatc gttttccggg acgccggctg gatgatcctc   4320
cagcgcgggg atctcatgct ggagttcttc gcccaccccc ggatccaagc ttatcgatag   4380
gcctaggcct atcgataggc ctaggcctat cgataggcct aacacgagcc atagataaaa   4440
taaaagattt tatttagtct ccagaaaaag gggggaatga aagaccccac ctgtaggttt   4500
ggcaagctag cttaagtaac gccattttgc aaggcatgga aaaatacata actgagaata   4560
gagaagttca gatcaaggtc aggaacagat ggaacagctg aatatgggcc aaacaggata   4620
tctgtggtaa gcagttcctg ccccggctca gggccaagaa cagatggaac agctgaatat   4680
gggccaaaca ggatatctgt ggtaagcagt tcctgccccg gctcagggcc aagaacagat   4740
ggtccccaga tgcggtccag ccctcagcag tttctagaga accatcagat gtttccaggg   4800
tgccccaagg acctgaaatg accctgtgcc ttatttgaac taaccaatca gttcgcttct   4860
cgcttctgtt cgcgcgcttc tgctccccga gctcaataaa agagcccaca acccctcact   4920
cggggcgcca gtcctccgat tgactgagtc gcccgggtac ccgtgttctc aataaaccct   4980
cttgcagttg catccgactc gtggtctcgc tgttccttgg gagggtctcc tctgagtgat   5040
tgactacccg tcagcggggg tctttcagta ggatctcgac cgatgccctt gagagccttc   5100
aacccagtca gctccttccg gtgggcgcgg ggcatgacta tcgtcgccgc acttatgact   5160
gtcttcttta tcatgcaact cgtaggacag gtgccggcag cgctctgggt cattttcggc   5220
gaggaccgct ttcgctggag cgcgacgatg atcggcctgt cgcttgcggt attcggaatc   5280
ttgcacgccc tcgctcaagc cttcgtcact ggtcccgcca ccaaacgttt cggcgagaag   5340
caggccatta tcgccggcat ggcggccgac gcgctgggct acgtcttgct ggcgttcgcg   5400
acgcgaggct ggatggcctt ccccattatg attcttctcg cttccggcgg catcgggatg   5460
cccgcgttgc aggccatgct gtccaggcag gtagatgacg accatcaggg acagcttcaa   5520
ggatcgctcg cggctcttac cagcctaact tcgatcactg gaccgctgat cgtcacggcg   5580
```

```
atttatgccg cctcggcgag cacatggaac gggttggcat ggattgtagg cgcctgatgc   5640
ggtattttct ccttacgcat ctgtgcggta tttcacaccg catatggtgc actctcagta   5700
caatctgctc tgatgccgca tagttaagcc agccccgaca cccgccaaca cccgctgacg   5760
cgccctgacg ggcttgtctg ctcccggcat ccgcttacag acaagctgtg accgtctccg   5820
ggagctgcat gtgtcagagg ttttcaccgt catcaccgaa acgcgcgaga cgaaagggcc   5880
tcgtgatacg cctattttta taggttaatg tcatgataat aatggtttct tagacgtcag   5940
gtggcacttt tcggggaaat gtgcgcggaa cccctatttg tttatttttc taaatacatt   6000
caaatatgta tccgctcatg agacaataac cctgataaat gcttcaataa tattgaaaaa   6060
ggaagagtat gagtattcaa catttccgtg tcgcccttat tccctttttt gcggcatttt   6120
gccttcctgt ttttgctcac ccagaaacgc tggtgaaagt aaaagatgct gaagatcagt   6180
tgggtgcacg agtgggttac atcgaactgg atctcaacag cggtaagatc cttgagagtt   6240
ttcgccccga agaacgtttt ccaatgatga gcacttttaa agttctgcta tgtggcgcgg   6300
tattatcccg tattgacgcc gggcaagagc aactcggtcg ccgcatacac tattctcaga   6360
atgacttggt tgagtactca ccagtcacag aaaagcatct tacggatggc atgacagtaa   6420
gagaattatg cagtgctgcc ataaccatga gtgataacac tgcggccaac ttacttctga   6480
caacgatcgg aggaccgaag gagctaaccg cttttttgca caacatgggg gatcatgtaa   6540
ctcgccttga tcgttgggaa ccggagctga atgaagccat accaaacgac gagcgtgaca   6600
ccacgatgcc tgtagcaatg gcaacaacgt tgcgcaaact attaactggc gaactactta   6660
ctctagcttc ccggcaacaa ttaatagact ggatggaggc ggataaagtt gcaggaccac   6720
ttctgcgctc ggcccttccg gctggctggt ttattgctga taaatctgga gccggtgagc   6780
gtgggtctcg cggtatcatt gcagcactgg ggccagatgg taagccctcc cgtatcgtag   6840
ttatctacac gacggggagt caggcaacta tggatgaacg aaatagacag atcgctgaga   6900
taggtgcctc actgattaag cattggtaac tgtcagacca agtttactca tatatacttt   6960
agattgattt aaaacttcat ttttaattta aaaggatcta ggtgaagatc ctttttgata   7020
atctcatgac caaaatccct taacgtgagt tttcgttcca ctgagcgtca gaccccgtag   7080
aaaagatcaa aggatcttct tgagatcctt tttttctgcg cgtaatctgc tgcttgcaaa   7140
caaaaaaacc accgctacca gcggtggttt gtttgccgga tcaagagcta ccaactcttt   7200
ttccgaaggt aactggcttc agcagagcgc agataccaaa tactgtcctt ctagtgtagc   7260
cgtagttagg ccaccacttc aagaactctg tagcaccgcc tacatacctc gctctgctaa   7320
tcctgttacc agtggctgct gccagtggcg ataagtcgtg tcttaccggg ttggactcaa   7380
gacgatagtt accggataag gcgcagcggt cgggctgaac ggggggttcg tgcacacagc   7440
ccagcttgga gcgaacgacc tacaccgaac tgagatacct acagcgtgag cattgagaaa   7500
gcgccacgct tcccgaaggg agaaaggcgg acaggtatcc ggtaagcggc agggtcggaa   7560
caggagagcg cacgagggag cttccagggg gaaacgcctg gtatctttat agtcctgtcg   7620
ggtttcgcca cctctgactt gagcgtcgat ttttgtgatg ctcgtcaggg gggcggagcc   7680
tatggaaaaa cgccagcaac gcggcctttt tacggttcct ggccttttgc tggccttttg   7740
ctcacatgtt ctttcctgcg ttatcccctg attctgtgga taaccgtatt accgcctttg   7800
agtgagctga taccgctcgc cgcagccgaa cgaccgagcg cagcgagtca gtgagcgagg   7860
aagcggaaga gcgcccaata cgcaaaccgc ctctccccgc gcgttggccg attcattaat   7920
gcagg                                                               7925
```

(2) Angaben zu SEQ ID NO: 5:

(i) SEQUENZKENNZEICHEN:

(A) LÄNGE: 636 Basenpaare
(B) ART: Nukleotid
(C) STRANGFORM: Doppelstrang
(D) TOPOLOGIE: linear

(ii) ART DES MOLEKÜLS: Sonstige Nucleinsäure
(ix) MERKMAL:

(A) NAME / SCHLÜSSEL: misc_feature
(B) LAGE: 1..636
(D) SONSTIGE ANGABEN: / product= "leader11"

(xi) SEQUENZBESCHREIBUNG: SEQ ID NO: 5:

```
cgcgccagtc ctccgataga ctgcgtcgcc cgggtacccg tattcccaat aaagcctctt    60
gctgtttgca tccgaatcgt ggactcgctg atccttggga gggtctcctc agattgattg   120
actgcccacc tcgggggtct ttcatttgga ggttccaccg agatttggag acccctgccc   180
agggaccacc gacccccccg ccgggaggta agctggccag cggtcgtttc gtgtctgtct   240
ctgtctttgt gcgtgtttgt gccggcatct aatgtttgcg cctgcgtctg tactagttgg   300
ctaactagat ctgtatctgg cggtcccgcg gaagaactga cgagttcgta ttcccggccg   360
cagcccctgg gagacgtccc agcggcctcg gggcccgtt ttgtggccca ttctgtatca    420
gttaacctac ccgagtcgga ctttttggag ctccgccact gtccgagggg tacgtggctt   480
tgttggggga cgagagacag agacacttcc cgccccccgtc tgaattttg ctttcggttt   540
tacgccgaaa ccgcgccgcg cgtcttgtct gctgcagcat cgttctgtgt tgtctctgtc   600
tgacgtggtt ctgtattgtc tgaaaatagc ggccgcc                            637
```

(2) Angaben zu SEQ ID NO: 6:

(i) SEQUENZKENNZEICHEN:

(A) LÄNGE:687 Basenpaare
(B) ART: Nukleotid
(C) STRANGFORM: Doppelstrang
(D) TOPOLOGIE: linear

(ii) ART DES MOLEKÜLS: Sonstige Nucleinsäure
(ix) MERKMAL:

(A) NAME / SCHLÜSSEL: misc-feature
(B) LAGE: 1..686
(D) SONSTIGE ANGABEN: / product= "leader91"

(xi) SEQUENZBESCHREIBUNG: SEQ ID NO: 6:

```
ggcgccagtc ctccgattga ctgagtcgcc cgggtacccg tattcccaat aaagcctctt    60
gctgtttgca tccgaatcgt ggactcgctg atccttggga gggtctcctc agattgattg   120
actgcccacc tcgggggtct ttcatttgga ggttccaccg agatttggag acccctgccc   180
agggaccacc gacccccccg ccgggaggta agctggccag cggtcgtttc gtgtctgtct   240
ctgtctttgt gcgtgtttgt gccggcatct agtgtttgcg cctgcgtctg tactagttgg   300
ctaactagat ctgtatctgg cggtcccgcg gaagaactga cgagttcgta ttcccggccg   360
cagcccctgg gagacgtccc agcggcctcg gggcccgtt ttgtggccca ttctgtatca    420
gttaacctac ccgagtcgga ctttttggag ctccgccact gtccgagggg tacgtggctt   480
tgttggggga cgagagacag agacacttcc cgccccccgtc tgaattttg ctttcggttt   540
tacgccgaaa ccgcgccgcg cgtcttgtct gctgcagcat cgttctgtgt tgtctctgtc   600
```

```
tgactgtgtt tctgtatttg tctgaaaatt agctcgacaa agttaagtaa tagtccctct   660
ctccaagctc acttacaggc ggccgcc                                       687
```

(2) Angaben zu SEQ ID NO: 7:

(i) SEQUENZKENNZEICHEN:

(A) LÄNGE: 1147 Basenpaare
(B) ART: Nukleotid
(C) STRANGFORM: Doppelstrang
(D) TOPOLOGIE: linear

(ii) ART DES MOLEKÜLS: Sonstige Nucleinsäure
(ix) MERKMAL:

(A) NAME / SCHLÜSSEL: misc_feature
(B) LAGE: 1..1147
(D) SONSTIGE ANGABEN: / product= "HSV-TK"

(xi) SEQUENZBESCHREIBUNG: SEQ ID NO: 7:

```
gcggccgcca tggcttcgta cccctgccat caacacgcgt ctgcgttcga ccaggctgcg      60
cgttctcgcg gccatagcaa ccgacgtacg gcgttgcgcc ctcgccggca gcaagaagcc     120
acggaagtcc gcccggagca gaaaatgccc acgctactgc gggtttatat agacggtccc     180
cacgggatgg ggaaaaccac caccacgcaa ctgctggtgg ccctgggttc gcgcgacgat     240
atcgtctacg tacccgagcc gatgacttac tggcgggtgc tgggggcttc cgagacaatc     300
gcgaacatct acaccacaca acaccgcctc gaccagggtg agatatcggc cggggacgcg     360
gcggtggtaa tgacaagcgc ccagataaca atgggcatgc cttatgccgt gaccgacgcc     420
gttctggctc ctcatatcgg gggggaggct gggagctcac atgccccgcc cccggccctc     480
accctcatct tcgaccgcca tcccatcgcc gccctcctgt gctacccggc cgcgcggtac     540
cttatgggca gcatgacccc ccaggccgtg ctggcgttcg tggccctcat cccgccgacc     600
ttgcccggca ccaacatcgt gcttggggcc cttccggagg acagacacat cgaccgcctg     660
gccaaacgcc agcgccccgg cgagcggctg gacctggcta tgctggctgc gattcgccgc     720
gtttacgggc tacttgccaa tacggtgcgg tatctgcagt gcggcgggtc gtggcgggag     780
gactggggac agctttcggg gacggccgtg ccgccccagg gtgccgagcc ccagagcaac     840
gcgggcccac gaccccatat cggggacacg ttatttaccc tgtttcgggc ccccgagttg     900
ctggccccca acggcgacct gtataacgtg tttgcctggg ccttggacgt cttggccaaa     960
cgcctccgtt ccatgcacgt ctttatcctg gattacgacc aatcgcccgc cggctgccgg    1020
gacgccctgc tgcaacttac ctccgggatg gtccagaccc acgtcaccac ccccggctcc    1080
ataccgacga tatgcgacct ggcgcgcacg tttgcccggg agatggggga ggctaactga    1140
taagctt                                                             1147
```

(2) Angaben zu SEQ ID NO: 8:

(i) SEQUENZKENNZEICHEN:

(A) LÄNGE: 1147 Basenpaare
(B) ART: Nukleotid
(C) STRANGFORM: Doppelstrang
(D) TOPOLOGIE: linear

(ii) ART DES MOLEKÜLS: Sonstige Nucleinsäure
(ix) MERKMAL:

(A) NAME / SCHLÜSSEL: misc_feature
(B) LAGE: 1..1147
(D) SONSTIGE ANGABEN: / product= "HSV-TK/splice motifs destroyed"

(xi) SEQUENZBESCHREIBUNG: SEQ ID NO: 8:

```
gcggccgcca tggcttcgta cccctgccat caacacgcgt ctgcgttcga ccaggctgcg      60
cgttctcgcg gccatagcaa ccgacgtacg gcgttgcgcc ctcgccggca gcaagaagcc     120
acggaagtcc gcccggagca gaaaatgccc acgctactgc gggtttatat agacggtccc     180
cacgggatgg ggaaaaccac caccacgcaa ctgctggtgg ccctgggttc gcgcgacgat     240
atcgtctacg tacccgagcc gatgacttac tggcgggtgc tgggggcttc cgagacaatc     300
gcgaacatct acaccacaca acaccgcctc gaccaagggg agatatcggc cggggacgcg     360
gcggtggtaa tgacaagcgc ccagataaca atgggcatgc cttatgccgt gaccgacgcc     420
gttctggctc ctcatatcgg gggggaggct gggagctcac atgccccgcc cccggccctc     480
accctcatct tcgaccgcca tcccatcgcc gccctcctgt gctacccggc cgcgcggtac     540
cttatgggca gcatgacccc ccaagccgtg ctggcgttcg tggccctcat cccgccgacc     600
ttgcccggca ccaacatcgt gcttggggcc cttccggagg acagacacat cgaccgcctg     660
gccaaacgcc agcgccccgg cgagcggctg gacctggcta tgctggctgc gattcgccgc     720
gtttacgggc tacttgccaa tacggtgcgg tatctgcagt gcggcgggtc gtggcgggag     780
gactggggac agctttcggg gacggccgtg ccgccccagg gtgccgagcc ccagagcaac     840
gcgggcccac gaccccatat cggggacacg ttatttaccc tgtttcgggc ccccgagttg     900
ctggccccca acggcgacct gtataacgtg tttgcctggg ccttggacgt cttggccaaa     960
cgcctccgtt ccatgcacgt ctttatcctg gattacgacc aatcgcccgc cggctgccgg    1020
gacgccctgc tgcaacttac ctccgggatg gtccagaccc acgtcaccac ccccggctcc    1080
ataccgacga tatgcgacct ggcgcgcacg tttgcccggg agatgggga ggctaactga     1140
taagctt                                                              1147
```

(2) Angaben zu SEQ ID NO: 9:

(i) SEQUENZKENNZEICHEN:

(A) LÄNGE: 639 Basenpaare
(B) ART: Nukleotid
(C) STRANGFORM: Doppelstrang
(D) TOPOLOGIE: linear

(ii) ART DES MOLEKÜLS: Sonstige Nucleinsäure
(ix) MERKMAL:

(A) NAME / SCHLÜSSEL: misc_feature
(B) LAGE: 1..639
(D) SONSTIGE ANGABEN: / product= "IRES"

(xi) SEQUENZBESCHREIBUNG: SEQ ID NO: 9:

```
aagcttaaaa cagctctggg gttgtaccca ccccagaggc ccacgtggcg gctagtactc      60
cggtattgcg gtacccttgt acgcctgttt tatactccct tcccgtaact tagacgcaca     120
aaaccaagtt caatagaagg gggtacaaac cagtaccacc acgaacaagc acttctgttt     180
ccccggtgat gtcgtataga ctgcttgcgt ggttgaaagc gacggatccg ttatccgctt     240
atgtacttcg agaagcccag taccacctcg gaatcttcaa tgcgttgcgc tcagcactca     300
accccagagt gtagcttagg ctgatgagtc tggacatccc tcaccggtga cggtggtcca     360
ggctgcgttg gcggcctacc tatggctaac gccatgggac gctagttgtg aacaaggtgt     420
gaagagccta ttgagctaca taagaatcct ccggcccctg aatgcggcta atcccaacct     480
cggagcaggt ggtcacaaac cagtgattgg cctgtcgtaa cgcgcaagtc cgtggcggaa     540
ccgactactt tgggtgtccg tgtttccttt tattttattg tggctgctta tggtgacaat     600
cacagattgt tatcataaag cgaattggat tgcggccgc                           639
```

(2) Angaben zu SEQ ID NO: 10:

(i) SEQUENZKENNZEICHEN:

(A) LÄNGE: 1037 Basenpaare
(B) ART: Nukleotid

(C) STRANGFORM: Doppelstrang
(D) TOPOLOGIE: linear

(ii) ART DES MOLEKÜLS: Sonstige Nucleinsäure
(ix) MERKMAL:

(A) NAME / SCHLÜSSEL: misc_feature
(B) LAGE: 1..1037
(D) SONSTIGE ANGABEN: / product= "Neo"

(xi) SEQUENZBESCHREIBUNG: SEQ ID NO: 10:

```
gcggccgctc tagaactagt ggatctgaat taattcctgc agccaatatg ggatcggcca      60
ttgaacaaga tggattgcac gcaggttctc cggccgcttg ggtggagagg ctattcggct     120
atgactgggc acaacagaca atcggctgct ctgatgccgc cgtgttccgg ctgtcagcgc     180
aggggcgccc ggttcttttt gtcaagaccg acctgtccgg tgccctgaat gaactgcagg     240
acgaggcagc gcggctatcg tggctggcca cgacgggcgt tccttgcgca gctgtgctcg     300
acgttgtcac tgaagcggga agggactggc tgctattggg cgaagtgccg gggcaggatc     360
tcctgtcatc tcaccttgct cctgccgaga aagtatccat catggctgat gcaatgcggc     420
ggctgcatac gcttgatccg gctacctgcc cattcgacca ccaagcgaaa catcgcatcg     480
agcgagcacg tactcggatg gaagccggtc ttgtcgatca ggatgatctg gacgaagagc     540
atcaggggct cgcgccagcc gaactgttcg ccaggctcaa ggcgcgcatg cccgacggcg     600

aggatctcgt cgtgacccat ggcgatgcct gcttgccgaa tatcatggtg gaaaatggcc     660
gcttttctgg attcatcgac tgtggccggc tgggtgtggc ggaccgctat caggacatag     720
cgttggctac ccgtgatatt gctgaagagc ttggcggcga atgggctgac cgcttcctcg     780
tgctttacgg tatcgccgct cccgattcgc agcgcatcgc cttctatcgc cttcttgacg     840
agttcttctg agcgggactc tggggttcga aatgaccgac caagcgacgc ccaacctgcc     900
atcacgagat ttcgattcca ccgccgcctt ctatgaaagg ttgggcttcg gaatcgtttt     960
ccgggacgcc ggctggatga tcctccagcg cggggatctc atgctggagt tcttcgccca    1020
cccccggatc caagctt                                                   1037
```

(2) Angaben zu SEQ ID NO: 11:

(i) SEQUENZKENNZEICHEN:

(A) LÄNGE: 32 Basenpaare
(B) ART: Nukleotid
(C) STRANGFORM: Einzelstrang
(D) TOPOLOGIE: linear

(ii) ART DES MOLEKÜLS: Sonstige Nucleinsäure
(ix) MERKMAL:

(A) NAME / SCHLÜSSEL: misc_feature
(B) LAGE: 1..32
(D) SONSTIGE ANGABEN: / product= "TKdSD5"

(xi) SEQUENZBESCHREIBUNG: SEQ ID NO: 11:

caacaccgcc tcgaccaagg ggagatatcg gc 32

(2) Angaben zu SEQ ID NO : 12:

(i) SEQUENZKENNZEICHEN:

(A) LÄNGE: 32 Basenpaare

(B) ART: Nukleotid
(C) STRANGFORM: Einzelstrang
(D) TOPOLOGIE: linear

(ii) ART DES MOLEKÜLS: Sonstige Nucleinsäure
(ix) MERKMAL:

(A) NAME / SCHLÜSSEL: misc_feature
(B) LAGE: 1..32
(D) SONSTIGE ANGABEN: / product= "TKdSD3"

(xi) SEQUENZBESCHREIBUNG: SEQ ID NO: 12:

gccgattatct ccccttgtc gaggcggtgt tg 32

(2) Angaben zu SEQ ID NO: 13:

(i) SEQUENZKENNZEICHEN:

(A) LÄNGE: 29 Basenpaare
(B) ART: Nukleotid
(C) STRANGFORM: Einzelstrang
(D) TOPOLOGIE: linear

(ii) ART DES MOLEKÜLS: Sonstige Nucleinsäure
(ix) MERKMAL:

(A) NAME / SCHLÜSSEL: misc_feature
(B) LAGE: 1..29
(D) SONSTIGE ANGABEN: / product= "TKdSA5"

(xi) SEQUENZBESCHREIBUNG: SEQ ID NO: 13:

gcatgacccc ccaagccgtg ctggcgttc 29

(2) Angaben zu SEQ ID NO: 14:

(i) SEQUENZKENNZEICHEN:

(A) LÄNGE: 29 Basenpaare
(B) ART: Nukleotid
(C) STRANGFORM: Einzelstrang
(D) TOPOLOGIE: linear

(ii) ART DES MOLEKÜLS: Sonstige Nucleinsäure
(ix) MERKMAL:

(A) NAME / SCHLÜSSEL: misc_feature
(B) LAGE: 1..29
(D) SONSTIGE ANGABEN: / product= "TKdSA3"

(xi) SEQUENZBESCHREIBUNG: SEQ ID NO: 14:

gaacgccagc acggcttggg gggtcatgc 29

(2) Angaben zu SEQ ID NO: 15:

(i) SEQUENZKENNZEICHEN:

(A) LÄNGE: 7235 Basenpaare
(B) ART: Nukleotid
(C) STRANGFORM: Doppelstrang
(D) TOPOLOGIE: ringförmig

(ii) ART DES MOLEKÜLS: Sonstige Nucleinsäure

(A) BESCHREIBUNG: /desc = "Provirale Plasmid-DNA"

(ix) MERKMAL:

(A) NAME / SCHLÜSSEL: misc_RNA
(B) LAGE: 1-423
(D) SONSTIGE ANGABEN: /note= "Plasmid-Rueckgrat"

(ix) MERKMAL:

(A) NAME / SCHLÜSSEL: misc_RNA
(B) LAGE: 424-994
(D) SONSTIGE ANGABEN: /note= "5'-LTR'

(ix) MERKMAL:

(A) NAME / SCHLÜSSEL: 5' UTR
(B) LAGE: 995-1537

(ix) MERKMAL:

(A) NAME / SCHLÜSSEL: mat_peptide
(B) LAGE: 1538-2665
(D) SONSTIGE ANGABEN: /note= "HSV-TK cDNA"

(ix) MERKMAL:

(A) NAME / SCHLÜSSEL: misc_RNA
(B) LAGE: 2676-3300
(D) SONSTIGE ANGABEN: /note= "IRES"

(ix) MERKMAL:

(A) NAME / SCHLÜSSEL: mat_peptide
(B) LAGE: 3348-4148
(D) SONSTIGE ANGABEN: /note= "Neo cDNA"

(ix) MERKMAL:

(A) NAME / SCHLÜSSEL: misc_RNA
(B) LAGE: 4395-4981
(D) SONSTIGE ANGABEN: /note= "3'-LTR'

(ix) MERKMAL:

(A) NAME / SCHLÜSSEL: misc_RNA
(B) LAGE: 4982-7235
(D) SONSTIGE ANGABEN: /note= "Plasmid-Rueckgrat"

(ix) MERKMAL:

(A) NAME / SCHLÜSSEL: misc_RNA
(B) LAGE: 1..7235
(D) SONSTIGE ANGABEN: / product= "MPSV 71 TIN" /note= "Retroviraler Suizidgenvektor"

(xi) SEQUENZBESCHREIBUNG: SEQ ID NO: 15:

```
ctgcctcgcg cgtttcggtg atgacggtga aaacctctga cacatgcagc tcccggagac     60
ggtcacagct tgtctgtaag cggatgccgg gagcagacaa gcccgtcagg gcgcgtcagc    120
gggtgttggc gggtgtcggg gcgcagccat gacccagtca cgtagcgata gttactatgc    180
ggcatcagag cagattgtac tgagagtgca ccatatgcgg tgtgaaatac cgcacagatg    240
cgtaaggaga aaataccgca tcaggcgcca ttcgccattc aggctgcgca actgttggga    300
agggcgatcg gtgcgggcct cttcgctatt acgccagctg gcgaaagggg gatgtgctgc    360
aaggcgatta agttgggtaa cgccagggtt ttcccagtca cgacgttgta aaacgacggc    420
cagtgaatta gtactctagc ttaagtaacg ccattttgca aggcatggaa aatacataac    480
tgagaataga gaagttcaga tcaaggttag gaacagagag acagcagaat atgggccaaa    540
caggatatct gtggtaagca gttcctgccc cggctcaggg ccaagaacag ttggaacagc    600
agaatatggg ccaaacagga tatctgtggt aagcagttcc tgccccggct cagggccaag    660
aacagatggt ccccagatgc ggtcccgccc tcagcagttt ctagagaacc atcagatgtt    720
tccagggtgc cccaaggacc tgaaatgacc ctgtgcctta tttgaactaa ccaatcagtt    780
cgcttctcgc ttctgttcgc gcgcttctgc tccccgagct caataaaaga gcccacaacc    840
cctcactcgg ggcgccagtc ctccgattga ctgagtcgcc cgggtacccg tattcccaat    900
aaagcctctt gctgtttgca tccgaatcgt ggactcgctg atccttggga gggtctcctc    960
agattgattg actgcccacc tcgggggtct ttcatttgga ggttccaccg agatttggag   1020
acccctgccc agggaccacc gacccccccg ccgggaggta agctggccag cggtcgtttc   1080
gtgtctgtct ctgtctttgt gcgtgtttgt gccggcatct aatgtttgcg cctgcgtctg   1140
tactagttgg ctaactagat ctgtatctgg cggtcccgcg gaagaactga cgagttcgta   1200
ttcccggccg cagcccctgg gagacgtccc agcggcctcg gggcccgtt ttgtggccca    1260
ttctgtatca gttaacctac ccgagtcgga ctttttggag ctccgccact gtccgagggg   1320
tacgtggctt tgttgggga cgagagacag agacacttcc cgcccccgtc tgaatttttg    1380
ctttcggttt tacgccgaaa ccgcgccgcg cgtcttgtct gctgcagcat cgttctgtgt   1440
tgtctctgtc tgactgtgtt tctgtatttg tctgaaaatt agctcgacaa agttaagtaa   1500
tagtccctct ctccaagctc acttacaggc ggccgccatg gcttcgtacc cctgccatca   1560
acacgcgtct gcgttcgacc aggctgcgcg ttctcgcggc catagcaacc gacgtacggc   1620
gttgcgccct cgccggcagc aagaagccac ggaagtccgc ccggagcaga aaatgcccac   1680
gctactgcgg gtttatatag acggtcccca cgggatgggg aaaaccacca ccacgcaact   1740
gctggtggcc ctgggttcgc gcgacgatat cgtctacgta cccgagccga tgacttactg   1800
gcgggtgctg gggcttccg agacaatcgc gaacatctac accacacaac accgcctcga    1860
ccagggtgag atatcggccg gggacgcggc ggtggtaatg acaagcgccc agataacaat   1920
gggcatgcct tatgccgtga ccgacgccgt tctggctcct catatcgggg gggaggctgg   1980
gagctcacat gccccgcccc cggccctcac cctcatcttc gaccgccatc ccatcgccgc   2040
cctcctgtgc tacccggccg cgcggtacct tatgggcagc atgacccccc aggccgtgct   2100
ggcgttcgtg gccctcatcc cgccgacctt gcccggcacc aacatcgtgc ttggggccct   2160
tccggaggac agacacatcg accgcctggc caaacgccag cgccccggcg agcggctgga   2220
cctggctatg ctggctgcga ttcgccgcgt ttacgggcta cttgccaata cggtgcggta   2280
tctgcagtgc ggcgggtcgt ggcgggagga ctggggacag ctttcgggga cggccgtgcc   2340
gccccagggt gccgagcccc agagcaacgc gggcccacga ccccatatcg gggacacgtt   2400
atttaccctg tttcgggccc ccgagttgct ggcccccaac ggcgacctgt ataacgtgtt   2460
tgcctgggcc ttggacgtct tggccaaacg cctccgttcc atgcacgtct ttatcctgga   2520
ttacgaccaa tcgcccgccg gctgccggga cgccctgctg caacttacct ccgggatggt   2580
ccagacccac gtcaccaccc ccggctccat accgacgata tgcgacctgg cgcgcacgtt   2640
tgcccgggag atgggggagg ctaactgata agcttaaaac agctctgggg ttgtacccac   2700
cccagaggcc cacgtggcgg ctagtactcc ggtattgcgg tacccttgta cgcctgtttt   2760
atactccctt cccgtaactt agacgcacaa aaccaagttc aatagaaggg ggtacaaacc   2820
agtaccacca cgaacaagca cttctgtttc cccggtgatg tcgtatagac tgcttgcgtg   2880
gttgaaagcg acggatccgt tatccgctta tgtacttcga gaagcccagt accacctcgg   2940
aatcttcaat gcgttgcgct cagcactcaa ccccagagtg tagcttaggc tgatgagtct   3000
ggacatccct caccggtgac ggtggtccag gctgcgttgg cggcctacct atggctaacg   3060
ccatgggacg ctagttgtga acaaggtgtg aagagcctat tgagctacat aagaatcctc   3120
cggcccctga atgcggctaa tcccaacctc ggagcaggtg gtcacaaacc agtgattggc   3180
ctgtcgtaac gcgcaagtcc gtggcggaac cgactacttt gggtgtccgt gtttcctttt   3240
attttattgt ggctgcttat ggtgacaatc acagattgtt atcataaagc gaattggatt   3300
gcggccgctc tagaactagt ggatctgaat taattcctgc agccaatatg ggatcggcca   3360
ttgaacaaga tggattgcac gcaggttctc cggccgcttg ggtggagagg ctattcggct   3420
atgactgggc acaacagaca atcggctgct ctgatgccgc cgtgttccgg ctgtcagcgc   3480
aggggcgccc ggttcttttt gtcaagaccg acctgtccgg tgccctgaat gaactgcagg   3540
```

```
acgaggcagc gcggctatcg tggctggcca cgacgggcgt tccttgcgca gctgtgctcg     3600
acgttgtcac tgaagcggga agggactggc tgctattggg cgaagtgccg gggcaggatc     3660
tcctgtcatc tcaccttgct cctgccgaga aagtatccat catggctgat gcaatgcggc     3720
ggctgcatac gcttgatccg gctacctgcc cattcgacca ccaagcgaaa catcgcatcg     3780
agcgagcacg tactcggatg gaagccggtc ttgtcgatca ggatgatctg gacgaagagc     3840
atcaggggct cgcgccagcc gaactgttcg ccaggctcaa ggcgcgcatg cccgacggcg     3900
aggatctcgt cgtgacccat ggcgatgcct gcttgccgaa tatcatggtg gaaaatggcc     3960
gcttttctgg attcatcgac tgtggccggc tgggtgtggc ggaccgctat caggacatag     4020
cgttggctac ccgtgatatt gctgaagagc ttggcggcga atgggctgac cgcttcctcg     4080
tgctttacgg tatcgccgct cccgattcgc agcgcatcgc cttctatcgc cttcttgacg     4140
agttcttctg agcgggactc tggggttcga aatgaccgac caagcgacgc ccaacctgcc     4200
atcacgagat ttcgattcca ccgccgcctt ctatgaaagg ttgggcttcg gaatcgtttt     4260
ccgggacgcc ggctggatga tcctccagcg cggggatctc atgctggagt tcttcgccca     4320
cccccggatc caagcttaac acgagccata gatagaataa aagattttat ttagtctcca     4380
gaaaaagggg ggaatgaaag accccacctg taggtttggc aagctagctt aagtaacgcc     4440
attttgcaag gcatggaaaa tacataactg agaatagaga agttcagatc aaggttagga     4500
acagagagac agcagaatat gggccaaaca ggatatctgt ggtaagcagt tcctgccccg     4560
gctcagggcc aagaacagtt ggaacagcag aatatgggcc aaacaggata tctgtggtaa     4620
gcagttcctg ccccggctca gggccaagaa cagatggtcc ccagatgcgg tcccgccctc     4680
agcagtttct agagaaccat cagatgtttc cagggtgccc caaggacctg aaatgaccct     4740
gtgccttatt tgaactaacc aatcagttcg cttctcgctt ctgttcgcgc gcttctgctc     4800
cccgagctca ataaaagagc ccacaacccc tcactcggcg cgccagtcct ccgatagact     4860
gcgtcgcccg ggtacccgtg ttctcaataa accctcttgc agttgcatcc gactcgtggt     4920
ctcgctgttc cttgggaggg tctcctctga gtgattgact gcccacctcg ggggtctttc     4980
attctcgagc agcttggcgt aatcatggtc atagctgttt cctgtgtgaa attgttatcc     5040
gctcacaatt ccacacaaca tacgagccgg aagcataaag tgtaaagcct ggggtgccta     5100
atgagtgagc taactcacat taattgcgtt gcgctcactg cccgctttcc agtcgggaaa     5160
cctgtcgtgc cagctgcatt aatgaatcgg ccaacgcgcg gggagaggcg gtttgcgtat     5220
tgggcgctct tccgcttcct cgctcactga ctcgctgcgc tcggtcgttc ggctgcggcg     5280
agcggtatca gctcactcaa aggcggtaat acggttatcc acagaatcag gggataacgc     5340
aggaaagaac atgtgagcaa aaggccagca aaaggccagg aaccgtaaaa aggccgcgtt     5400
gctggcgttt ttccataggc tccgcccccc tgacgagcat cacaaaaatc gacgctcaag     5460
tcagaggtgg cgaaacccga caggactata aagataccag gcgtttcccc ctggaagctc     5520
cctcgtgcgc tctcctgttc cgaccctgcc gcttaccgga tacctgtccg cctttctccc     5580
ttcgggaagc gtggcgcttt ctcaatgctc acgctgtagg tatctcagtt cggtgtaggt     5640
cgttcgctcc aagctgggct gtgtgcacga accccccgtt cagcccgacc gctgcgcctt     5700
atccggtaac tatcgtcttg agtccaaccc ggtaagacac gacttatcgc cactggcagc     5760
agccactggt aacaggatta gcagagcgag gtatgtaggc ggtgctacag agttcttgaa     5820
gtggtggcct aactacggct acactagaag gacagtattt ggtatctgcg ctctgctgaa     5880
gccagttacc ttcggaaaaa gagttggtag ctcttgatcc ggcaaacaaa ccaccgctgg     5940
tagcggtggt ttttttgttt gcaagcagca gattacgcgc agaaaaaaag gatctcaaga     6000
agatcctttg atcttttcta cggggtctga cgctcagtgg aacgaaaact cacgttaagg     6060
gattttggtc atgagattat caaaaaggat cttcacctag atccttttaa attaaaaatg     6120
aagttttaaa tcaatctaaa gtatatatga gtaaacttgg tctgacagtt accaatgctt     6180
aatcagtgag gcacctatct cagcgatctg tctatttcgt tcatccatag ttgcctgact     6240
ccccgtcgtg tagataacta cgatacggga gggcttacca tctggcccca gtgctgcaat     6300
gataccgcga gacccacgct caccggctcc agatttatca gcaataaacc agccagccgg     6360
aagggccgag cgcagaagtg gtcctgcaac tttatccgcc tccatccagt ctattaattg     6420
ttgccgggaa gctagagtaa gtagttcgcc agttaatagt ttgcgcaacg ttgttgccat     6480
tgctgctggc atcgtggtgt cacgctcgtc gtttggtatg gcttcattca gctccggttc     6540
ccaacgatca aggcgagtta catgatcccc catgttgtgc aaaaaagcgg ttagctcctt     6600
cggtcctccg atcgttgtca gaagtaagtt ggccgcagtg ttatcactca tggttatggc     6660
agcactgcat aattctctta ctgtcatgcc atccgtaaga tgcttttctg tgactggtga     6720
gtactcaacc aagtcattct gagaatagtg tatgcggcga ccgagttgct cttgcccggc     6780
gtcaatacgg gataataccg cgccacatag cagaacttta aaagtgctca tcattggaaa     6840
acgttcttcg gggcgaaaac tctcaaggat cttaccgctg ttgagatcca gttcgatgta     6900
acccactcgt gcacccaact gatcttcagc atcttttact ttcaccagcg tttctgggtg     6960
agcaaaaaca ggaaggcaaa atgccgcaaa aaagggaata agggcgacac ggaaatgttg     7020
aatactcata ctcttccttt ttcaatatta ttgaagcatt tatcagggtt attgtctcat     7080
gagcggatac atatttgaat gtatttagaa aaataaacaa ataggggttc cgcgcacatt     7140
tccccgaaaa gtgccacctg acgtctaaga aaccattatt atcatgacat taacctataa     7200
aaataggcgt atcacgaggc cctttcgtct tcaag                               7235
```

(2) Angaben zu SEQ ID NO: 16:

(i) SEQUENZKENNZEICHEN:

(A) LÄNGE: 7235 Basenpaare
(B) ART: Nukleotid
(C) STRANGFORM: Doppelstrang
(D) TOPOLOGIE: ringförmig

(ii) ART DES MOLEKÜLS: Sonstige Nucleinsäure

(A) BESCHREIBUNG: /desc = "Provirale Plasmid-DNA"

(ix) MERKMAL:

(A) NAME / SCHLÜSSEL: misc_RNA
(B) LAGE: 1-423
(D) SONSTIGE ANGABEN: /note= "Pläsmid-Rueckgrat"

(ix) MERKMAL:

(A) NAME / SCHLÜSSEL: misc_RNA
(B) LAGE: 424-994
(D) SONSTIGE ANGABEN: /note= "5'-LTR"

(ix) MERKMAL:

(A) NAME / SCHLÜSSEL: 5' UTR
(B) LAGE: 995-1537

(ix) MERKMAL:

(A) NAME / SCHLÜSSEL: mat_peptide
(B) LAGE: 1538-2665
(D) SONSTIGE ANGABEN: /note= "HSV-TK/splice motifs destroyed cDNA"

(ix) MERKMAL:

(A) NAME / SCHLÜSSEL: misc_RNA
(B) LAGE: 2676-3300
(D) SONSTIGE ANGABEN: /note= "IRES"

(ix) MERKMAL:

(A) NAME / SCHLÜSSEL: mat_peptide
(B) LAGE: 3348-4148
(D) SONSTIGE ANGABEN: /note= "Neo cDNA"

(ix) MERKMAL:

(A) NAME / SCHLÜSSEL: misc_RNA
(B) LAGE: 4395-4981
(D) SONSTIGE ANGABEN: /note= "3'-LTR"

(ix) MERKMAL:

(A) NAME / SCHLÜSSEL: misc_RNA
(B) LAGE: 4982-7235
(D) SONSTIGE ANGABEN: /note= "Plasmid-Rueckgrat"

(ix) MERKMAL:

(A) NAME / SCHLÜSSEL: misc_RNA
(B) LAGE: 1..7235
(D) SONSTIGE ANGABEN: / product= "MPSV 91 T*IN" /note= "Retroviraler Suizidgenvektor"

(xi) SEQUENZBESCHREIBUNG: SEQ ID NO: 16:

```
ctgcctcgcg cgtttcggtg atgacggtga aaacctctga cacatgcagc tcccggagac      60
ggtcacagct tgtctgtaag cggatgccgg gagcagacaa gcccgtcagg gcgcgtcagc     120
gggtgttggc gggtgtcggg gcgcagccat gacccagtca cgtagcgata gttactatgc     180
ggcatcagag cagattgtac tgagagtgca ccatatgcgg tgtgaaatac cgcacagatg     240
cgtaaggaga aaataccgca tcaggcgcca ttcgccattc aggctgcgca actgttggga     300
agggcgatcg gtgcgggcct cttcgctatt acgccagctg gcgaaagggg gatgtgctgc     360
aaggcgatta agttgggtaa cgccagggtt ttcccagtca cgacgttgta aaacgacggc     420
cagtgaatta gtactctagc ttaagtaacg ccattttgca aggcatggaa aatacataac     480
tgagaataga gaagttcaga tcaaggttag gaacagagag acagcagaat atgggccaaa     540
caggatatct gtggtaagca gttcctgccc cggctcaggg ccaagaacag ttggaacagc     600
agaatatggg ccaaacagga tatctgtggt aagcagttcc tgccccggct cagggccaag     660
```

```
aacagatggt ccccagatgc ggtcccgccc tcagcagttt ctagagaacc atcagatgtt     720
tccagggtgc cccaaggacc tgaaatgacc ctgtgcctta tttgaactaa ccaatcagtt     780
cgcttctcgc ttctgttcgc gcgcttctgc tccccgagct caataaaaga gcccacaacc     840
cctcactcgg ggcgccagtc ctccgattga ctgagtcgcc cgggtacccg tattcccaat     900
aaagcctctt gctgtttgca tccgaatcgt ggactcgctg atccttggga gggtctcctc     960
agattgattg actgcccacc tcgggggtct ttcatttgga ggttccaccg agatttggag    1020
acccctgccc agggaccacc gacccccccg ccgggaggta agctggccag cggtcgtttc    1080
gtgtctgtct ctgtctttgt gcgtgtttgt gccggcatct agtgtttgcg cctgcgtctg    1140
tactagttgg ctaactagat ctgtatctgg cggtcccgcg gaagaactga cgagttcgta    1200
ttcccggccg cagcccctgg gagacgtccc agcggcctcg gggcccgtt ttgtggccca     1260
ttctgtatca gttaacctac ccgagtcgga ctttttggag ctccgccact gtccgagggg    1320
tacgtggctt tgttgggga cgagagacag agacacttcc cgcccccgtc tgaatttttg     1380
ctttcggttt tacgccgaaa ccgcgccgcg cgtcttgtct gctgcagcat cgttctgtgt    1440
tgtctctgtc tgactgtgtt tctgtatttg tctgaaaatt agctcgacaa agttaagtaa    1500
tagtccctct ctccaagctc acttacaggc ggccgccatg gcttcgtacc cctgccatca    1560
acacgcgtct gcgttcgacc aggctgcgcg ttctcgcggc catagcaacc gacgtacggc    1620
gttgcgccct cgccggcagc aagaagccac ggaagtccgc ccggagcaga aaatgcccac    1680
gctactgcgg gtttatatag acggtcccca cgggatgggg aaaaccacca ccacgcaact    1740
gctggtggcc ctgggttcgc gcgacgatat cgtctacgta cccgagccga tgacttactg    1800
gcgggtgctg ggggcttccg agacaatcgc gaacatctac accacacaac accgcctcga    1860
ccaaggggag atatcggccg gggacgcggc ggtggtaatg acaagcgccc agataacaat    1920
gggcatgcct tatgccgtga ccgacgccgt tctggctcct catatcgggg gggaggctgg    1980
gagctcacat gccccgcccc cggccctcac cctcatcttc gaccgccatc ccatcgccgc    2040
cctcctgtgc tacccggccg cgcggtacct tatgggcagc atgacccccc aagccgtgct    2100
ggcgttcgtg gccctcatcc cgccgacctt gcccggcacc aacatcgtgc ttggggccct    2160
tccggaggac agacacatcg accgcctggc caaacgccag cgccccggcg agcggctgga    2220
cctggctatg ctggctgcga ttcgccgcgt ttacgggcta cttgccaata cggtgcggta    2280
tctgcagtgc ggcgggtcgt ggcgggagga ctggggacag ctttcgggga cggccgtgcc    2340
gccccagggt gccgagcccc agagcaacgc gggcccacga ccccatatcg ggacacgtt     2400
atttaccctg tttcgggccc ccgagttgct ggcccccaac ggcgacctgt ataacgtgtt    2460
tgcctgggcc ttggacgtct tggccaaacg cctccgttcc atgcacgtct ttatcctgga    2520
ttacgaccaa tcgcccgccg gctgccggga cgccctgctg caacttacct ccgggatggt    2580
ccagacccac gtcaccaccc ccggctccat accgacgata tgcgacctgg cgcgcacgtt    2640
tgcccgggag atgggggagg ctaactgata agcttaaaac agctctgggg ttgtacccac    2700
cccagaggcc cacgtggcgg ctagtactcc ggtattgcgg tacccttgta cgcctgtttt    2760
atactccctt cccgtaactt agacgcacaa aaccaagttc aatagaaggg ggtacaaacc    2820
agtaccacca cgaacaagca cttctgtttc cccggtgatg tcgtatagac tgcttgcgtg    2880
gttgaaagcg acggatccgt tatccgctta tgtacttcga gaagcccagt accacctcgg    2940
aatcttcaat gcgttgcgct cagcactcaa ccccagagtg tagcttaggc tgatgagtct    3000
ggacatccct caccggtgac ggtggtccag gctgcgttgg cggcctacct atggctaacg    3060
ccatgggacg ctagttgtga acaaggtgtg aagagcctat tgagctacat aagaatcctc    3120
cggcccctga atgcggctaa tcccaacctc ggagcaggtg gtcacaaacc agtgattggc    3180
ctgtcgtaac gcgcaagtcc gtggcggaac cgactacttt gggtgtccgt gtttcctttt    3240
attttattgt ggctgcttat ggtgacaatc acagattgtt atcataaagc gaattggatt    3300
gcggccgctc tagaactagt ggatctgaat taattcctgc agccaatatg ggatcggcca    3360
ttgaacaaga tggattgcac gcaggttctc cggccgcttg ggtggagagg ctattcggct    3420
atgactgggc acaacagaca atcggctgct ctgatgccgc cgtgttccgg ctgtcagcgc    3480
aggggcgccc ggttcttttt gtcaagaccg acctgtccgg tgccctgaat gaactgcagg    3540
acgaggcagc gcggctatcg tggctggcca cgacgggcgt tccttgcgca gctgtgctcg    3600
acgttgtcac tgaagcggga agggactggc tgctattggg cgaagtgccg gggcaggatc    3660
tcctgtcatc tcaccttgct cctgccgaga aagtatccat catggctgat gcaatgcggc    3720
ggctgcatac gcttgatccg gctacctgcc cattcgacca ccaagcgaaa catcgcatcg    3780
agcgagcacg tactcggatg gaagccggtc ttgtcgatca ggatgatctg gacgaagagc    3840
atcaggggct cgcgccagcc gaactgttcg ccaggctcaa ggcgcgcatg cccgacggcg    3900
aggatctcgt cgtgacccat ggcgatgcct gcttgccgaa tatcatggtg gaaaatggcc    3960
gcttttctgg attcatcgac tgtggccggc tgggtgtggc ggaccgctat caggacatag    4020
cgttggctac ccgtgatatt gctgaagagc ttggcggcga atgggctgac cgcttcctcg    4080
tgctttacgg tatcgccgct cccgattcgc agcgcatcgc cttctatcgc cttcttgacg    4140
agttcttctg agcgggactc tggggttcga aatgaccgac caagcgacgc ccaacctgcc    4200
atcacgagat ttcgattcca ccgccgcctt ctatgaaagg ttgggcttcg gaatcgtttt    4260
ccgggacgcc ggctggatga tcctccagcg cggggatctc atgctggagt tcttcgccca    4320
cccccggatc caagcttaac acgagccata gatagaataa aagattttat ttagtctcca    4380
gaaaaagggg ggaatgaaag accccacctg taggtttggc aagctagctt aagtaacgcc    4440
```

```
attttgcaag gcatggaaaa tacataactg agaatagaga agttcagatc aaggttagga   4500
acagagagac agcagaatat gggccaaaca ggatatctgt ggtaagcagt tcctgccccg   4560
gctcagggcc aagaacagtt ggaacagcag aatatgggcc aaacaggata tctgtggtaa   4620
gcagttcctg ccccggctca gggccaagaa cagatggtcc ccagatgcgg tcccgccctc   4680
agcagtttct agagaaccat cagatgtttc cagggtgccc caaggacctg aaatgaccct   4740
gtgccttatt tgaactaacc aatcagttcg cttctcgctt ctgttcgcgc gcttctgctc   4800
cccgagctca ataaaagagc ccacaacccc tcactcggcg cgccagtcct ccgatagact   4860
gcgtcgcccg ggtacccgtg ttctcaataa accctcttgc agttgcatcc gactcgtggt   4920
ctcgctgttc cttgggaggg tctcctctga gtgattgact gcccacctcg ggggtctttc   4980
attctcgagc agcttggcgt aatcatggtc atagctgttt cctgtgtgaa attgttatcc   5040
gctcacaatt ccacacaaca tacgagccgg aagcataaag tgtaaagcct ggggtgccta   5100
atgagtgagc taactcacat taattgcgtt gcgctcactg cccgctttcc agtcgggaaa   5160
cctgtcgtgc cagctgcatt aatgaatcgg ccaacgcgcg gggagaggcg gtttgcgtat   5220
tgggcgctct tccgcttcct cgctcactga ctcgctgcgc tcggtcgttc ggctgcggcg   5280
agcggtatca gctcactcaa aggcggtaat acggttatcc acagaatcag gggataacgc   5340
aggaaagaac atgtgagcaa aaggccagca aaaggccagg aaccgtaaaa aggccgcgtt   5400
gctggcgttt ttccataggc tccgcccccc tgacgagcat cacaaaaatc gacgctcaag   5460
tcagaggtgg cgaaacccga caggactata aagataccag gcgtttcccc ctggaagctc   5520
cctcgtgcgc tctcctgttc cgaccctgcc gcttaccgga tacctgtccg cctttctccc   5580
ttcgggaagc gtggcgcttt ctcaatgctc acgctgtagg tatctcagtt cggtgtaggt   5640
cgttcgctcc aagctgggct gtgtgcacga accccccgtt cagcccgacc gctgcgcctt   5700
atccggtaac tatcgtcttg agtccaaccc ggtaagacac gacttatcgc cactggcagc   5760
agccactggt aacaggatta gcagagcgag gtatgtaggc ggtgctacag agttcttgaa   5820
gtggtggcct aactacggct acactagaag gacagtattt ggtatctgcg ctctgctgaa   5880
gccagttacc ttcggaaaaa gagttggtag ctcttgatcc ggcaaacaaa ccaccgctgg   5940
tagcggtggt ttttttgttt gcaagcagca gattacgcgc agaaaaaaag gatctcaaga   6000
agatcctttg atcttttcta cggggtctga cgctcagtgg aacgaaaact cacgttaagg   6060
gattttggtc atgagattat caaaaaggat cttcacctag atccttttaa attaaaaatg   6120
aagttttaaa tcaatctaaa gtatatatga gtaaacttgg tctgacagtt accaatgctt   6180
aatcagtgag gcacctatct cagcgatctg tctatttcgt tcatccatag ttgcctgact   6240
ccccgtcgtg tagataacta cgatacggga gggcttacca tctggcccca gtgctgcaat   6300
gataccgcga gacccacgct caccggctcc agatttatca gcaataaacc agccagccgg   6360
aagggccgag cgcagaagtg gtcctgcaac tttatccgcc tccatccagt ctattaattg   6420
ttgccgggaa gctagagtaa gtagttcgcc agttaatagt ttgcgcaacg ttgttgccat   6480
tgctgctggc atcgtggtgt cacgctcgtc gtttggtatg gcttcattca gctccggttc   6540
ccaacgatca aggcgagtta catgatcccc catgttgtgc aaaaaagcgg ttagctcctt   6600
cggtcctccg atcgttgtca gaagtaagtt ggccgcagtg ttatcactca tggttatggc   6660
agcactgcat aattctctta ctgtcatgcc atccgtaaga tgcttttctg tgactggtga   6720
gtactcaacc aagtcattct gagaatagtg tatgcggcga ccgagttgct cttgcccggc   6780
gtcaatacgg gataataccg cgccacatag cagaacttta aaagtgctca tcattggaaa   6840
acgttcttcg gggcgaaaac tctcaaggat cttaccgctg ttgagatcca gttcgatgta   6900
acccactcgt gcacccaact gatcttcagc atcttttact ttcaccagcg tttctgggtg   6960
agcaaaaaca ggaaggcaaa atgccgcaaa aaagggaata agggcgacac ggaaatgttg   7020
aatactcata ctcttccttt ttcaatatta ttgaagcatt tatcagggtt attgtctcat   7080
gagcggatac atatttgaat gtatttagaa aaataaacaa ataggggttc cgcgcacatt   7140
tccccgaaaa gtgccacctg acgtctaaga aaccattatt atcatgacat taacctataa   7200
aaataggcgt atcacgaggc cctttcgtct tcaag                              7235
```

(2) Angaben zu SEQ ID NO: 17:

(i) SEQUENZKENNZEICHEN:

(A) LÄNGE: 7235 Basenpaare
(B) ART: Nukleotid
(C) STRANGFORM: Doppelstrang
(D) TOPOLOGIE: ringförmig

(ii) ART DES MOLEKÜLS: Sonstige Nucleinsäure

(A) BESCHREIBUNG: /desc = "Provirale Plasmid-DNA"

(ix) MERKMAL:

(A) NAME / SCHLÜSSEL: misc_RNA
(B) LAGE: 1-423
(D) SONSTIGE ANGABEN: /note= "Plasmid-Rueckgrat"

(ix) MERKMAL:

(A) NAME / SCHLÜSSEL: misc_RNA
(B) LAGE: 424-994
(D) SONSTIGE ANGABEN: /note= "5'-LTR"

(ix) MERKMAL:

(A) NAME / SCHLÜSSEL: 5' UTR
(B) LAGE: 995-1537

(ix) MERKMAL:

(A) NAME / SCHLÜSSEL: mat_peptide
(B) LAGE: 1538-2665
(D) SONSTIGE ANGABEN: /note= "HSV-TK/splice motifs destroyed cDNA"

(ix) MERKMAL:

(A) NAME / SCHLÜSSEL: misc_RNA
(B) LAGE: 2676-3300
(D) SONSTIGE ANGABEN: /note= "IRES"

(ix) MERKMAL:

(A) NAME / SCHLÜSSEL: mat_peptide
(B) LAGE: 3348-4148
(D) SONSTIGE ANGABEN: /note= "Neo cDNA"

(ix) MERKMAL:

(A) NAME / SCHLÜSSEL: misc_RNA
(B) LAGE: 4395-4981
(D) SONSTIGE ANGABEN: /note= "3'-LTR"

(ix) MERKMAL:

(A) NAME / SCHLÜSSEL: misc_RNA
(B) LAGE: 4982-7235
(D) SONSTIGE ANGABEN: /note= "Plasmid-Rueckgrat"

(ix) MERKMAL:

(A) NAME / SCHLÜSSEL: misc_RNA
(B) LAGE: 1..7235
(D) SONSTIGE ANGABEN: / product= "MPSV 71 T*IN" /note= "Retroviraler Suizidgenvektor"

(xi) SEQUENZBESCHREIBUNG: SEQ ID NO: 17:

```
ctgcctcgcg cgtttcggtg atgacggtga aaacctctga cacatgcagc tcccggagac      60
ggtcacagct tgtctgtaag cggatgccgg gagcagacaa gcccgtcagg gcgcgtcagc     120
gggtgttggc gggtgtcggg gcgcagccat gacccagtca cgtagcgata gttactatgc     180
ggcatcagag cagattgtac tgagagtgca ccatatgcgg tgtgaaatac cgcacagatg     240
cgtaaggaga aaataccgca tcaggcgcca ttcgccattc aggctgcgca actgttggga     300
agggcgatcg gtgcgggcct cttcgctatt acgccagctg gcgaaagggg gatgtgctgc     360
aaggcgatta agttgggtaa cgccagggtt ttcccagtca cgacgttgta aaacgacggc     420
cagtgaatta gtactctagc ttaagtaacg ccattttgca aggcatggaa aatacataac     480
tgagaataga gaagttcaga tcaaggttag gaacagagag acagcagaat atgggccaaa     540
caggatatct gtggtaagca gttcctgccc cggctcaggg ccaagaacag ttggaacagc     600
agaatatggg ccaaacagga tatctgtggt aagcagttcc tgccccggct cagggccaag     660
aacagatggt ccccagatgc ggtcccgccc tcagcagttt ctagagaacc atcagatgtt     720
tccagggtgc cccaaggacc tgaaatgacc ctgtgcctta tttgaactaa ccaatcagtt     780
cgcttctcgc ttctgttcgc gcgcttctgc tccccgagct caataaaaga gcccacaacc     840
cctcactcgg ggcgccagtc ctccgattga ctgagtcgcc cgggtacccg tattcccaat     900
aaagcctctt gctgtttgca tccgaatcgt ggactcgctg atccttggga gggtctcctc     960
agattgattg actgcccacc tcgggggtct ttcatttgga ggttccaccg agatttggag    1020
acccctgccc agggaccacc gacccccccg ccgggaggta agctggccag cggtcgtttc    1080
gtgtctgtct ctgtctttgt gcgtgtttgt gccggcatct aatgtttgcg cctgcgtctg    1140
tactagttgg ctaactagat ctgtatctgg cggtcccgcg gaagaactga cgagttcgta    1200
ttcccggccg cagcccctgg gagacgtccc agcggcctcg gggcccgtt ttgtggccca    1260
ttctgtatca gttaacctac ccgagtcgga ctttttggag ctccgccact gtccgagggg    1320
tacgtggctt tgttggggga cgagagacag agacacttcc cgcccccgtc tgaatttttg    1380
ctttcggttt tacgccgaaa ccgcgccgcg cgtcttgtct gctgcagcat cgttctgtgt    1440
tgtctctgtc tgactgtgtt tctgtatttg tctgaaaatt agctcgacaa agttaagtaa    1500
tagtccctct ctccaagctc acttacaggc ggccgccatg gcttcgtacc cctgccatca    1560
acacgcgtct gcgttcgacc aggctgcgcg ttctcgcggc catagcaacc gacgtacggc    1620
gttgcgccct cgccggcagc aagaagccac ggaagtccgc ccggagcaga aaatgcccac    1680
```

```
gctactgcgg gtttatatag acggtcccca cgggatgggg aaaaccacca ccacgcaact     1740
gctggtggcc ctgggttcgc gcgacgatat cgtctacgta cccgagccga tgacttactg     1800
gcgggtgctg gggcttccg agacaatcgc gaacatctac accacacaac accgcctcga      1860
ccaaggggag atatcggccg gggacgcggc ggtggtaatg acaagcgccc agataacaat     1920
gggcatgcct tatgccgtga ccgacgccgt tctggctcct catatcgggg gggaggctgg     1980
gagctcacat gccccgcccc cggccctcac cctcatcttc gaccgccatc ccatcgccgc     2040
cctcctgtgc tacccggccg cgcggtacct tatgggcagc atgacccccc aagccgtgct     2100
ggcgttcgtg gccctcatcc cgccgacctt gcccggcacc aacatcgtgc ttggggccct     2160
tccggaggac agacacatcg accgcctggc caaacgccag cgccccggcg agcggctgga     2220
cctggctatg ctggctgcga ttcgccgcgt ttacgggcta cttgccaata cggtgcggta     2280
tctgcagtgc ggcgggtcgt ggcgggagga ctggggacag ctttcgggga cggccgtgcc     2340
gccccagggt gccgagcccc agagcaacgc gggcccacga ccccatatcg gggacacgtt     2400
atttaccctg tttcgggccc ccgagttgct ggcccccaac ggcgacctgt ataacgtgtt     2460
tgcctgggcc ttggacgtct tggccaaacg cctccgttcc atgcacgtct ttatcctgga     2520
ttacgaccaa tcgcccgccg gctgccggga cgccctgctg caacttacct ccgggatggt     2580
ccagacccac gtcaccaccc ccggctccat accgacgata tgcgacctgg cgcgcacgtt     2640
tgcccgggag atgggggagg ctaactgata agcttaaaac agctctgggg ttgtacccac     2700
cccagaggcc cacgtggcgg ctagtactcc ggtattgcgg taccttgta cgcctgtttt      2760
atactccctt cccgtaactt agacgcacaa aaccaagttc aatagaaggg ggtacaaacc     2820
agtaccacca cgaacaagca cttctgtttc cccggtgatg tcgtatagac tgcttgcgtg     2880
gttgaaagcg acggatccgt tatccgctta tgtacttcga gaagcccagt accacctcgg     2940
aatcttcaat gcgttgcgct cagcactcaa ccccagagtg tagcttaggc tgatgagtct     3000
ggacatccct caccggtgac ggtggtccag gctgcgttgg cggcctacct atggctaacg     3060
ccatgggacg ctagttgtga acaaggtgtg aagagcctat tgagctacat aagaatcctc     3120
cggcccctga atgcggctaa tcccaacctc ggagcaggtg gtcacaaacc agtgattggc     3180
ctgtcgtaac gcgcaagtcc gtggcggaac cgactacttt gggtgtccgt gtttcctttt     3240
attttattgt ggctgcttat ggtgacaatc acagattgtt atcataaagc gaattggatt     3300
gcggccgctc tagaactagt ggatctgaat taattcctgc agccaatatg ggatcggcca     3360
ttgaacaaga tggattgcac gcaggttctc cggccgcttg ggtggagagg ctattcggct     3420
atgactgggc acaacagaca atcggctgct ctgatgccgc cgtgttccgg ctgtcagcgc     3480
aggggcgccc ggttcttttt gtcaagaccg acctgtccgg tgccctgaat gaactgcagg     3540
acgaggcagc gcggctatcg tggctggcca cgacgggcgt tccttgcgca gctgtgctcg     3600
acgttgtcac tgaagcggga agggactggc tgctattggg cgaagtgccg gggcaggatc     3660
tcctgtcatc tcaccttgct cctgccgaga aagtatccat catggctgat gcaatgcggc     3720
ggctgcatac gcttgatccg gctacctgcc cattcgacca ccaagcgaaa catcgcatcg     3780
agcgagcacg tactcggatg gaagccggtc ttgtcgatca ggatgatctg gacgaagagc     3840
atcaggggct cgcgccagcc gaactgttcg ccaggctcaa ggcgcgcatg cccgacggcg     3900
aggatctcgt cgtgacccat ggcgatgcct gcttgccgaa tatcatggtg gaaaatggcc     3960
gcttttctgg attcatcgac tgtggccggc tgggtgtggc ggaccgctat caggacatag     4020
cgttggctac ccgtgatatt gctgaagagc ttggcggcga atgggctgac cgcttcctcg     4080
tgctttacgg tatcgccgct cccgattcgc agcgcatcgc cttctatcgc cttcttgacg     4140
agttcttctg agcgggactc tggggttcga aatgaccgac caagcgacgc ccaacctgcc     4200
atcacgagat ttcgattcca ccgccgcctt ctatgaaagg ttgggcttcg gaatcgtttt     4260
ccgggacgcc ggctggatga tcctccagcg cggggatctc atgctggagt tcttcgccca     4320
cccccggatc caagcttaac acgagccata gatagaataa aagattttat ttagtctcca     4380
gaaaaagggg ggaatgaaag accccacctg taggtttggc aagctagctt aagtaacgcc     4440
attttgcaag gcatggaaaa tacataactg agaatagaga agttcagatc aaggttagga     4500
acagagagac agcagaatat gggccaaaca ggatatctgt ggtaagcagt tcctgccccg     4560
gctcagggcc aagaacagtt ggaacagcag aatatgggcc aaacaggata tctgtggtaa     4620
gcagttcctg ccccggctca gggccaagaa cagatggtcc ccagatgcgg tcccgccctc     4680
agcagtttct agagaaccat cagatgtttc cagggtgccc caaggacctg aaatgaccct     4740
gtgccttatt tgaactaacc aatcagttcg cttctcgctt ctgttcgcgc gcttctgctc     4800
cccgagctca ataaaagagc ccacaacccc tcactcggcg cgccagtcct ccgatagact     4860
gcgtcgcccg ggtacccgtg ttctcaataa accctcttgc agttgcatcc gactcgtggt     4920
ctcgctgttc cttgggaggg tctcctctga gtgattgact gcccacctcg ggggtctttc     4980
attctcgagc agcttggcgt aatcatggtc atagctgttt cctgtgtgaa attgttatcc     5040
gctcacaatt ccacacaaca tacgagccgg aagcataaag tgtaaagcct ggggtgccta     5100
atgagtgagc taactcacat taattgcgtt gcgctcactg cccgctttcc agtcgggaaa     5160
cctgtcgtgc cagctgcatt aatgaatcgg ccaacgcgcg gggagaggcg gtttgcgtat     5220
tgggcgctct tccgcttcct cgctcactga ctcgctgcgc tcggtcgttc ggctgcggcg     5280
agcggtatca gctcactcaa aggcggtaat acggttatcc acagaatcag gggataacgc     5340
aggaaagaac atgtgagcaa aaggccagca aaaggccagg aaccgtaaaa aggccgcgtt     5400
gctggcgttt ttccataggc tccgcccccc tgacgagcat cacaaaaatc gacgctcaag     5460
```

```
tcagaggtgg cgaaacccga caggactata aagataccag gcgtttcccc ctggaagctc      5520
cctcgtgcgc tctcctgttc cgaccctgcc gcttaccgga tacctgtccg cctttctccc      5580
ttcgggaagc gtggcgcttt ctcaatgctc acgctgtagg tatctcagtt cggtgtaggt      5640
cgttcgctcc aagctgggct gtgtgcacga accccccgtt cagcccgacc gctgcgcctt      5700
atccggtaac tatcgtcttg agtccaaccc ggtaagacac gacttatcgc cactggcagc      5760
agccactggt aacaggatta gcagagcgag gtatgtaggc ggtgctacag agttcttgaa      5820
gtggtggcct aactacggct acactagaag gacagtattt ggtatctgcg ctctgctgaa      5880
gccagttacc ttcggaaaaa gagttggtag ctcttgatcc ggcaaacaaa ccaccgctgg      5940
tagcggtggt ttttttgttt gcaagcagca gattacgcgc agaaaaaaag gatctcaaga      6000
agatcctttg atcttttcta cggggtctga cgctcagtgg aacgaaaact cacgttaagg      6060
gattttggtc atgagattat caaaaaggat cttcacctag atccttttaa attaaaaatg      6120
aagttttaaa tcaatctaaa gtatatatga gtaaacttgg tctgacagtt accaatgctt      6180
aatcagtgag gcacctatct cagcgatctg tctatttcgt tcatccatag ttgcctgact      6240
ccccgtcgtg tagataacta cgatacggga gggcttacca tctggcccca gtgctgcaat      6300
gataccgcga gacccacgct caccggctcc agatttatca gcaataaacc agccagccgg      6360
aagggccgag cgcagaagtg gtcctgcaac tttatccgcc tccatccagt ctattaattg      6420
ttgccgggaa gctagagtaa gtagttcgcc agttaatagt ttgcgcaacg ttgttgccat      6480
tgctgctggc atcgtggtgt cacgctcgtc gtttggtatg gcttcattca gctccggttc      6540
ccaacgatca aggcgagtta catgatcccc catgttgtgc aaaaaagcgg ttagctcctt      6600
cggtcctccg atcgttgtca gaagtaagtt ggccgcagtg ttatcactca tggttatggc      6660
agcactgcat aattctctta ctgtcatgcc atccgtaaga tgcttttctg tgactggtga      6720
gtactcaacc aagtcattct gagaatagtg tatgcggcga ccgagttgct cttgcccggc      6780
gtcaatacgg gataataccg cgccacatag cagaacttta aaagtgctca tcattggaaa      6840
acgttcttcg gggcgaaaac tctcaaggat cttaccgctg ttgagatcca gttcgatgta      6900
acccactcgt gcacccaact gatcttcagc atcttttact ttcaccagcg tttctgggtg      6960
agcaaaaaca ggaaggcaaa atgccgcaaa aaagggaata agggcgacac ggaaatgttg      7020
aatactcata ctcttccttt ttcaatatta ttgaagcatt tatcagggtt attgtctcat      7080
gagcggatac atatttgaat gtatttagaa aaataaacaa ataggggttc cgcgcacatt      7140
tccccgaaaa gtgccacctg acgtctaaga aaccattatt atcatgacat taacctataa      7200
aaataggcgt atcacgaggc cctttcgtct tcaag                                 7235
```

(2) Angaben zu SEQ ID NO: 18:

(i) SEQUENZKENNZEICHEN:

(A) LÄNGE: 686 Basenpaare
(B) ART: Nukleotid
(C) STRANGFORM: Doppelstrang
(D) TOPOLOGIE: linear

(ii) ART DES MOLEKÜLS: Sonstige Nucleinsäure
(ix) MERKMAL:

(A) NAME / SCHLÜSSEL: misc_feature
(B) LAGE: 1..686
(D) SONSTIGE ANGABEN: / product= "leader71"

(xi) SEQUENZBESCHREIBUNG: SEQ ID NO: 18:

```
ggcgccagtc ctccgattga ctgagtcgcc cgggtacccg tattcccaat aaagcctctt      60
```

```
gctgtttgca tccgaatcgt ggactcgctg atccttggga gggtctcctc agattgattg   120
actgcccacc tcgggggtct ttcatttgga ggttccaccg agatttggag acccctgccc   180
agggaccacc gacccccccg ccgggaggta agctggccag cggtcgtttc gtgtctgtct   240
ctgtctttgt gcgtgtttgt gccggcatct aatgtttgcg cctgcgtctg tactagttgg   300
ctaactagat ctgtatctgg cggtcccgcg gaagaactga cgagttcgta ttcccggccg   360
cagcccctgg gagacgtccc agcggcctcg gggcccgtt ttgtggccca ttctgtatca    420
gttaacctac ccgagtcgga ctttttggag ctccgccact gtccgagggg tacgtggctt   480
tgttggggga cgagagacag agacacttcc cgcccccgtc tgaatttttg ctttcggttt   540
tacgccgaaa ccgcgccgcg cgtcttgtct gctgcagcat cgttctgtgt tgtctctgtc   600
tgactgtgtt tctgtatttg tctgaaaatt agctcgacaa agttaagtaa tagtccctct   660
ctccaagctc acttacaggc ggccgcc                                        687
```